# EUROPÄISCHE PATENTSCHRIFT

(11) **EP 2 791 119 B1**
(45) Veröffentlichungstag und Bekanntmachung des Hinweises auf die Patenterteilung: **01.02.2017**
(21) Anmeldenummer: 12798737.8
(22) Anmeldetag: 10.12.2012
(51) Int. Cl.: C07D 249/14, C07D 257/06, C07D 271/08, C07D 401/12, C07D 403/12, C07D 405/12, C07D 413/04, C07D 413/06, C07D 413/12, C07D 271/113, A01N 43/653, A01N 43/713, A01N 43/80, A01N 43/832, A01N 43/824

(54) **N-(1,2,5-OXADIAZOL-3-YL)-, N-(1,3,4-OXADIAZOL-2-YL)-, N-(TETRAZOL-5-YL)- UND N-(TRIAZOL-5-YL)-ARYLCARBONSÄUREAMIDE UND IHRE VERWENDUNG ALS HERBIZIDE**
N-(1,2,5-OXADIAZOL-3-YL)-, N-(1,3,4-OXADIAZOL-2-YL), N-(TETRAZOL-5-YL)- UND N-(TRIAZOL-5-YL)-ARYL CARBOXYLIC ACID AMIDES AND USE OF SAME AS HERBICIDES
ACIDE CARBOXYLIQUE DE N-(1,2,5-OXADIAZOL-3-YL)-, N-(1,3,4-OXADIAZOL-2-YL), N-(TÉTRAZOL-5-YL)- ET N-(TRIAZOL-5-YL)ARYLE AMINÉ ET LEUR UTILISATION COMME HERBICIDE

(30) Priorität: 13.12.2011 EP 11193166
(43) Veröffentlichungstag der Anmeldung: 22.10.2014
(73) Patentinhaber: Bayer Intellectual Property GmbH, 40789 Monheim (DE)
(72) Erfinder: KÖHN, Armin, 55270 Klein-Winternheim (DE); LEHR, Stefan, 69006 Lyon (FR); BRAUN, Ralf, 76857 Ramberg (DE); DÖRNER-RIEPING, Simon, 61267 Neu-Anspach (DE); AHRENS, Hartmut, 63329 Egelsbach (DE); DIETRICH, Hansjörg, 65835 Liederbach am Taunus (DE); HÄUSER-HAHN, Isolde, 51375 Leverkusen (DE); ROSINGER, Christopher, Hugh, 65719 Hofheim (DE); GATZWEILER, Elmar, 61231 Bad Nauheim (DE)
(74) Vertreter: BIP Patents
(86) Internationale Anmeldenummer: PCT/EP2012/074975
(87) Internationale Veröffentlichungsnummer: WO 2013/087577

(56) Entgegenhaltungen:
- EP-A1- 0 049 071
- WO-A1-00/31066
- WO-A1-2011/035874
- JP-A- 1 009 978
- BOWDEN KEITH ET AL: "Structure-activity relations. Part 10. Metal ion complexation studies of a series of substituted benzamidotetrazoles", JOURNAL OF CHEMICAL RESEARCH, SCIENCE REVIEWS LTD, GB, 1. Januar 1991 (1991-01-01), Seite 304, XP009158569, ISSN: 0308-2342 in der Anmeldung erwähnt
- G. WERBER ET AL: "Nucleophilic behaviour of some 1,3,4-oxadiazoles in benzoylation, nitrosation, acetylation, and methylation reactions", JOURNAL OF HETEROCYCLIC CHEMISTRY, Bd. 9, Nr. 1, 1. Februar 1972 (1972-02-01) , Seiten 107-109, XP055024795, ISSN: 0022-152X, DOI: 10.1002/jhet.5570090117 in der Anmeldung erwähnt

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus WO 2003/010143 A1 und WO 2003/010153 A1 sind N-(Tetrazol-5-yl)- und N-(Triazol-5-yl)benzamide und ihre pharmakologische Wirkung bekannt. EP 0 049 071 A1 offenbart N-Arylbenzamide mit pharmakologischer Wirkung. In J. Chem. Res. (S) 1991, 11, 304 werden die Verbindungen 2-Methoxy-N-methyl-N-(1H-tetrazol-5-yl)benzamid, 2-Hydroxy-N-methyl-N-(1H-tetrazol-5-yl)benzamid, 2-Methoxy-N-methyl-N-(1-methyl-1 H-tetrazol-5-yl)benzamid und 2-Methoxy-N-(2-methyl-2H-tetrazol-5-yl)benzamid mit pharmakologischer Wirkung beschrieben.

WO 00/31066 A1 beschreibt N-Pyrazolyl-phenoxynicotinsäure(thio)amide mit herbizider Wirkung. JP01009978 offenbart N-Isoxazolylbenzamide mit herbizider Wirkung. Aus WO 2011/035874 A1 sind N-(1,2,5-Oxadiazol-3-yl)benzamide als Herbizide bekannt. Aus der prioritätsälteren, nicht vorveröffentlichen europäischen Patentanmeldung Nr. EP101748937 sind bestimmte N-(Tetrazol-5-yl)- und N-(Triazol-5-yl)benzamide und -nicotinamide als Herbizide bekannt. Die herbizide Wirksamkeit und/oder die Kulturpflanzenverträglichkeit der in diesen Schriften genannten Verbindungen ist jedoch nicht immer ausreichend. In J. Het. Chem. 1972,9, 107-109 wird die Verbindung N-Methyl-N-(5-phenyl-1,3,4-oxadiazol-2-yl)benzamid genannt. Eine herbizide Wirkung dieser Verbindung ist nicht offenbart. Unter der CAS-Nummer 931735-86-3 ist die Verbindung 3-Brom-4-methoxy-N-methyl-N-(5-phenyl-1,3,4-oxadiazol-2-yl)benzamid bekannt. Eine herbizide Wirkung dieser Verbindung ist nicht offenbart.

Aufgabe der vorliegenden Erfindung war die Bereitstellung von herbizid wirksamen Verbindungen mit gegenüber den aus dem Stand der Technik bekannten Verbindungen verbesserten Eigenschaften.

Es wurde nun gefunden, dass Benzoylamide, die am Stickstoffatom durch bestimmte Reste subsituiert sind, als Herbizide besonders gut geeignet sind.

Ein Gegenstand der vorliegenden Erfindung sind somit N-(1,2,5-Oxadiazol-3-yl)-, N-(1,3,4-Oxadiazol-2-yl)-, N-(Tetrazol-5-yl)- und N-(Triazol-5-yl)-arylcarbonsäureamide der Formel (I) oder deren Salze 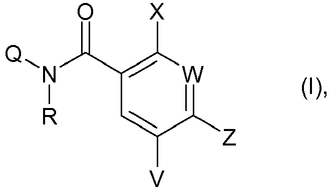 worin die Symbole und Indizes folgende Bedeutungen haben:
R bedeutet (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₃-C₆)-Halogenalkinyl, (C₃-C₆)-Cycloalkyl, (C₃-C₆)-Halogencycloalkyl, (C₃-C₆)-Cycloalkyl-(C₁-C₆)-alkyl, (C₃-C₆)-Halogencycloalkyl-(C₁-C₆)-alkyl, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-COOR¹, (C₁-C₆)-Alkyl-C(O)R¹, (C₁-C₆)-Alkyl-CN, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², OR¹, COOR¹, CON(R¹)₂, N(R¹)₂, NR¹COOR¹ NR¹CON(R¹)₂ oder
   jeweils durch s Reste aus der Gruppe Methyl, Ethyl, Methoxy, Nitro, Trifluormethyl und Halogen substituiertes Benzyl,
W bedeutet N oder CY,
X und Z bedeuten unabhängig voneinander jeweils Wasserstoff, Nitro, Halogen, Cyano, Formyl, Rhodano, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₃-C₆)-Halogenalkinyl, (C₃-C₆)-Cycloalkyl, (C₃-C₆)-Halogencycloalkyl, (C₃-C₆)-Cycloalkyl-(C₁-C₆)-alkyl, (C₃-C₆)-Halogencycloalkyl-(C₁-C₆)-alkyl, COR¹, OR¹, OCOR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-COOR¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², NR₁R₂, P(O)(OR⁵)₂, oder
   jeweils durch s Reste aus der Gruppe Methyl, Ethyl, Methoxy, Nitro, Trifluormethyl und Halogen substituiertes Heteroaryl, Heterocyclyl oder Phenyl,
Y bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹,OCOOR¹, NR¹COOR¹, C(O)N(R¹)₂, NR¹C(O)N(R¹)₂, OC(O)N(R¹)₂, C(O)N(R¹)OR¹, NR¹SO₂R², NR¹COR¹, OR¹, OSO₂R², S(O)ₙR², SO₂OR¹, SO₂N(R¹)₂ (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-CN, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², N(R¹)₂, P(O)(OR⁵)₂, CH₂P(O)(OR⁵)₂, CH=NOR¹, (C₁-C₆)-Alkyl-CH=NOR¹, (C₁-C₆)-Alkyl-O-N=C(R¹)₂, (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die 6 letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
   oder
Y und Z bilden gemeinsam mit den beiden Atomen, an die sie gebunden sind, einen 5-, 6- oder 7-gliedrigen, ungesättigten, teilgesättigten oder gesättigten Ring, der neben Kohlenstoffatomen jeweils s Stickstoffatome, n Sauerstoffatome, n Schwefelatome und n Elemente S(O), S(O)₂, C=N-R¹⁷, C(OR¹⁷)₂, C[-O-(CH₂)₂-O-] oder C(O) als Ringglieder umfasst,
   dessen Kohlenstoffatome durch s Reste aus der Gruppe bestehend aus Halogen, Cyano, (C₁-C₆)-Alkyl, (C₂-C₁₀)-Alkenyl, (C₂-C₁₀)-Alkinyl, (C₁-C₆)-Haloalkyl, (C₁-C₆)-Alkoxy, Phenoxy, Halogen-(C₁-C₆)-alkoxy, (C₃-C₈)-Cycloalkyl, (C₂-C₈)-Alkoxyalkyl und Phenyl substituiert sind,
   dessen Stickstoffatome durch n Reste aus der Gruppe bestehend aus (C₁-C₆)-Alkyl und Phenyl substituiert sind,
   und worin die vorstehend genannten Phenylreste durch s Reste aus der Gruppe bestehend aus Cyano, Nitro, Halogen, (C₁-C₆)-Alkyl, (C₁-C₆)-Haloalkyl und (C₁-C₆)-Alkoxy substituiert sind,
V bedeutet Wasserstoff, Nitro, Halogen, Cyano, (C₁-C₄)-Alkyl, (C₁-C₄)-Halogenalkyl, OR¹, S(O)ₙR²,
R¹ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocycl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl wobei die 21 letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
R² bedeutet (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl wobei die 21 letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
R³ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R⁴ bedeutet (C₁-C₆)-Alkyl, (C₂-C₆)-Alkenyl oder (C₂-C₆)-Alkinyl, (C₃-C₆)-Cycloalkyl oder (C₃-C₆)-Cycloalkyl-(C₁-C₆)-alkyl,
R⁵ bedeutet (C₁-C₄)-Alkyl,
n bedeutet 0, 1 oder 2,
s bedeutet 0, 1, 2 oder 3,
Q bedeutet einen Rest Q1, Q2, Q3 oder Q4 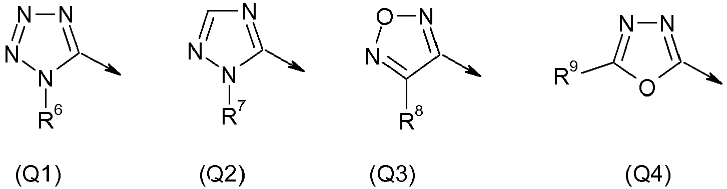
R⁶ bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl,
   wobei diese 6 vorstehend genannten Reste jeweils durch s Reste aus der Gruppe bestehend aus Nitro, Cyano, SiR¹²₃, PO(OR¹²)₃, S(O)ₙ-(C₁-C₆)-Alkyl, (C₁-C₆)-Alkoxy, Halogen-(C₁-C₆)-alkoxy, N(R¹⁰)₂, COR¹⁰, COOR¹⁰, OCOR¹⁰, OCO₂R¹⁰, NR¹⁰COR¹⁰, NR¹⁰SO₂R¹¹, (C₃-C₆)-Cycloalkyl, Heteroaryl, Heterocyclyl, Phenyl, D-Heteroaryl, D-Heterocyclyl, D-Phenyl oder D-Benzyl substituiert sind, und wobei die 7 letztgenannten Reste durch s Reste aus der Gruppe Methyl, Ethyl, Methoxy, Trifluormethyl und Halogen substituiert sind, und wobei Heterocyclyl n Oxogruppen trägt,
   oder
   R⁶ bedeutet jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiertes (C₃-C₇)-Cycloalkyl, Heteroaryl, Heterocyclyl oder Phenyl,
R⁷ bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl,
   wobei diese 6 vorstehend genannten Reste jeweils durch s Reste aus der Gruppe bestehend aus Nitro, Cyano, SiR¹²₃, PO(OR¹²)₃, S(O)ₙ-(C₁-C₆)-Alkyl, (C₁-C₆)-Alkoxy, Halogen-(C₁-C₆)-alkoxy, N(R¹⁰)₂, COR¹⁰, COOR¹⁰, OCOR¹⁰, OCO₂R¹⁰, NR¹⁰COR¹⁰, NR¹⁰SO₂R¹¹, (C₃-C₆)-Cycloalkyl, Heteroaryl, Heterocyclyl, Phenyl, D-Heteroaryl, D-Heterocyclyl, D-Phenyl und D-Benzyl substituiert sind, und wobei die 7 letztgenannten Reste durch s Reste aus der Gruppe Methyl, Ethyl, Methoxy, Trifluormethyl und Halogen substituiert sind, und wobei Heterocyclyl n Oxogruppen trägt, oder
   R⁷ bedeutet jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiertes (C₃-C₇)-Cycloalkyl, Heteroaryl, Heterocyclyl oder Phenyl,
R⁸ bedeutet Wasserstoff, (C₁-C₆)-Alkyl, (C₃-C₇)-Cycloalkyl, Halogen-(C₁-C₆)-alkyl, (C₁-C₆)-Alkoxy, Halogen-(C₁-C₆)-alkoxy, (C₂-C₆)-Alkenyl, (C₂-C₆)-Alkenyloxy, Halogen-(C₂-C₆)-alkenyl, (C₂-C₆)-Alkinyl, (C₂-C₆)-Alkinyloxy, Halogen-(C₂-C₆)-alkinyl, Cyano, Nitro, Methylsulfenyl, Methylsulfinyl, Methylsulfonyl, (C₁-C₆)-Alkylcarbonylamino, (C₁-C₆)-Alkoxycarbonylamino, Benzoylamino, Methoxycarbonyl, Ethoxycarbonyl, Methoxycarbonylmethyl, Ethoxycarbonylmethyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl, oder jeweils durch s Reste aus der Gruppe bestehend aus Methyl, Ethyl, Methoxy, Trifluormethyl und Halogen substituiertes Heteroaryl, Heterocyclyl oder Phenyl;
R⁹ bedeutet Wasserstoff, (C₁-C₆)-Alkyl, R¹³O-(C₁-C₆)-Alkyl, CH₂R¹⁴, (C₃-C₇)-Cycloalkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, OR¹³, NHR¹³, Methoxycarbonyl, Ethoxycarbonyl, Methoxycarbonylmethyl, Ethoxycarbonylmethyl, Methylcarbonyl, Trifluormethylcarbonyl, Dimethylamino, Acetylamino, Methylsulfenyl, Methylsulfinyl, Methylsulfonyl oder jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiertes Heteroaryl, Heterocyclyl, Benzyl oder Phenyl,
R¹⁰ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl oder Phenyl,
R¹¹ bedeutet (C₁-C₆)-Alkyl, (C₂-C₆)-Alkenyl, (C₂-C₆)-Alkinyl oder Phenyl,
R¹² bedeutet (C₁-C₆)-Alkyl,
R¹³ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocycl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR¹⁵-Heteroaryl oder (C₁-C₆)-Alkyl-NR¹⁵-Heterocyclyl, wobei die 21 letztgenannten Reste durch s Reste aus der Gruppe bestehend aus Cyano, Halogen, Nitro, Rhodano, OR¹⁵, S(O)ₙR¹⁶, N(R¹⁵)₂, NR¹⁵OR¹⁵, COR¹⁵, OCOR¹⁵, SCOR¹⁶, NR¹⁵COR¹⁵, NR¹⁵SO₂R¹⁶, CO₂R¹⁵, COSR¹⁶, CON(R¹⁵)₂ und (C₁-C₄)-Alkoxy-(C₂-C₆)-alkoxycarbonyl substituiert sind, und wobei Heterocyclyl n Oxogruppen trägt,
R¹⁴ bedeutet Acetoxy, Acetamido, N-Methylacetamido, Benzoyloxy, Benzamido, N-Methylbenzamido, Methoxycarbonyl, Ethoxycarbonyl, Benzoyl, Methylcarbonyl, Piperidinylcarbonyl, Morpholinylcarbonyl, Trifluormethylcarbonyl, Aminocarbonyl, Methylaminocarbonyl, Dimethylaminocarbonyl, (C₃-C₆)-Alkoxy, (C₃-C₆)-Cycloalkyl oder jeweils durch s Reste aus der Gruppe bestehend aus Methyl, Ethyl, Methoxy, Trifluormethyl und Halogen substituiertes Heteroaryl, Heterocyclyl oder Phenyl;
R¹⁵ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R¹⁶ bedeutet (C₁-C₆)-Alkyl, (C₂-C₆)-Alkenyl oder (C₂-C₆)-Alkinyl,
R¹⁷ bedeutet (C₁-C₆)-Alkyl, Halogen-(C₁-C₆)-alkyl, (C₁-C₆)-Alkoxy und Halogen-(C₁-C₆)-alkoxy,
s bedeutet 0, 1, 2 oder 3,
n bedeutet 0, 1 oder 2,
D bedeutet O, S, oder NR¹¹,
wobei die Verbindungen 2-Methoxy-N-methyl-N-(1 H-tetrazol-5-yl)benzamid, 2-Hydroxy-N-methyl-N-(1 H-tetrazol-5-yl)benzamid, 2-Methoxy-N-methyl-N-(1-methyl-1 H-tetrazol-5-yl)benzamid, N-Methyl-N-(5-phenyl-1,3,4-oxadiazol-2-yl)benzamid und 3-Brom-4-methoxy-N-methyl-N-(5-phenyl-1,3,4-oxadiazol-2-yl)benzamidausgenommen sind.

Von besonderem Interesse sind N-(1,3,4-Oxadiazol-2-yl)-, N-(Tetrazol-5-yl)- und N-(Triazol-5-yl)-arylcarbonsäureamide der Formel (I) oder deren Salze, worin Q bedeutet einen Rest Q1, Q2 oder Q4 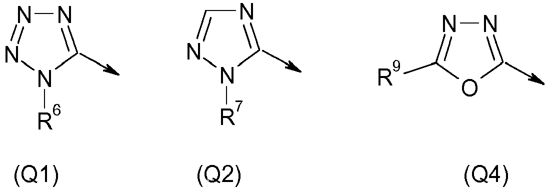 und alle anderen Reste und Indices jeweils die oben genannten Bedeutungen haben.

Von ganz besonderem Interesse N-(1,3,4-Oxadiazol-2-yl)- und N-(Tetrazol-5-yl)-arylcarbonsäureamide der Formel (I) oder deren Salze, worin
Q bedeutet einen Rest Q1 oder Q4 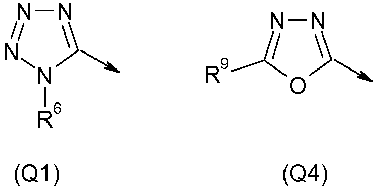 und alle anderen Reste und Indices jeweils die oben genannten Bedeutungen haben.

Bevorzugt sind N-(Tetrazol-5-yl)-arylcarbonsäureamide der Formel (I) oder deren Salze, worin
Q bedeutet den Rest Q1 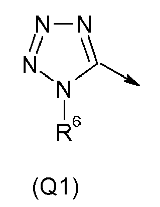 und alle anderen Reste und Indices jeweils die oben genannten Bedeutungen haben.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Analog bedeutet Alkenyl z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl. Alkinyl bedeutet z.B. Propargyl, But-2-in-1-yl, But-3-in-1-yl, 1-Methyl-but-3-in-1-yl. Die Mehrfachbindung kann sich jeweils in beliebiger Position des ungesättigten Rests befinden. Cycloalkyl bedeutet ein carbocyclisches, gesättigtes Ringsystem mit drei bis sechs C-Atomen, z.B. Cyclopropyl, Cyclobutyl, Cyclopentyl oder Cyclohexyl. Analog bedeutet Cycloalkenyl eine monocyclische Alkenylgruppe mit drei bis sechs Kohlenstoffringgliedern, z.B. Cyclopropenyl, Cyclobutenyl, Cyclopentenyl und Cyclohexenyl, wobei sich die Doppelbindung an beliebiger Position befinden kann.

Halogen steht für Fluor, Chlor, Brom oder Iod.

Heterocyclyl bedeutet einen gesättigten, teilgesättigten oder vollständig ungesättigten cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heterocyclyl für Piperidinyl, Pyrrolidinyl, Tetrahydrofuranyl, Dihydrofuranyl und Oxetanyl,

Heteroaryl bedeutet einen aromatischen cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heteroaryl für Benzimidazol-2-yl, Furanyl, Imidazolyl, Isoxazolyl, Isothiazolyl, Oxazolyl, Pyrazinyl, Pyrimidinyl, Pyridazinyl, Pyridinyl, Benzisoxazolyl, Thiazolyl, Pyrrolyl, Pyrazolyl, Thiophenyl, 1,2,3-Oxadiazolyl, 1,2,4-Oxadiazolyl, 1,2,5-Oxadiazolyl, 1,3,4-Oxadiazolyl, 1,2,4-Triazolyl, 1,2,3-Triazolyl, 1,2,5-Triazolyl, 1,3,4-Triazolyl, 1,2,4-Triazolyl, 1,2,4-Thiadiazolyl, 1,3,4-Thiadiazolyl, 1,2,3-Thiadiazolyl, 1,2,5-Thiadiazolyl, 2H-1,2,3,4-Tetrazolyl, 1 H-1,2,3,4-Tetrazolyl, 1,2,3,4-Oxatriazolyl, 1,2,3,5-Oxatriazolyl, 1,2,3,4-Thiatriazolyl und 1,2,3,5-Thiatriazolyl.

Ist eine Gruppe mehrfach durch Reste substituiert, so ist darunter zu verstehen, daß diese Gruppe durch ein oder mehrere gleiche oder verschiedene der genannten Reste substituiert ist. Analoges gilt für den Aufbau von Ringsystemen durch verschiedene Atome und Elemente. Dabei sollen solche Verbindungen vom Anspruchsbegehren ausgenommen sein, von denen der Fachmann weiß, dass sie unter Normalbedingungen chemisch instabil sind.

Die Verbindungen der allgemeinen Formel (I) können je nach Art und Verknüpfung der Substituenten als Stereoisomere vorliegen. Sind beispielsweise ein oder mehrere asymmetrische Kohlenstoffatome vorhanden, so können Enantiomere und Diastereomere auftreten. Ebenso treten Stereoisomere auf, wenn n für 1 steht (Sulfoxide). Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfasst, jedoch nicht spezifisch definiert sind. Die erfindungsgemäßen Verbindungen können auf Grund der Oximether-Struktur auch als geometrische Isomere (E-/Z-Isomere) auftreten. Die Erfindung betrifft auch alle E-/Z-Isomere und deren Gemische, die von der allgemeinen Formel (I) umfasst, jedoch nicht spezifisch definiert sind.

Die Verbindungen der Formel (I) können Salze bilden. Salzbildung kann durch Einwirkung einer Base auf solche Verbindungen der Formel (I) erfolgen, die ein acides Wasserstoffatom tragen, z.B. im Falle dass R¹ eine COOH-Gruppe oder eine Sulfonamid-Gruppe -NHSO₂- enthält. Geeignete Basen sind beispielsweise organische Amine , wie Trialkylamine, Morpholin, Piperidin oder Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze, zum Beispiel mit Kationen der Formel [NRR'R"R"']⁺, worin R bis R"' jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Die Verbindungen der Formel (I) können durch Anlagerung einer geeigneten anorganischen oder organischen Säure, wie beispielsweise Mineralsäuren, wie beispielsweise HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Amino, Alkylamino, Dialkylamino, Piperidino, Morpholino oder Pyridino, Salze bilden. Diese Salze enthalten dann die konjugierte Base der Säure als Anion.

Erfindungsgemäße Verbindungen können beispielsweise nach der in Schema 1 angegebenen Methode durch Umsetzung eines N-(1,2,5-Oxadiazol-3-yl)-, N-(1,3,4-Oxadiazol-2-yl)-, N-(Tetrazol-5-yl)- oder N-(Triazol-5-yl)- arylcarbonsäureamide (II) mit einer Verbindung der allgemeinen Formel (III), wobei L für eine Abgangsgruppe wie z. B. ein Chlor, Brom, Jod, Mesyloxy, Tosyloxy, Trifluormethylsulfonyloxy etc. steht, hergestellt werden:

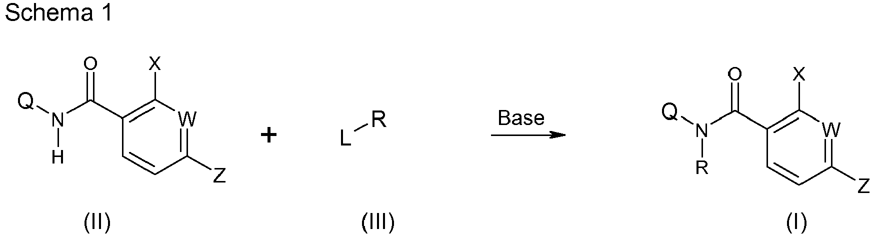

Die N-(1,2,5-Oxadiazol-3-yl-arylcarbonsäureamide der Formel (II) sind grundsätzlich bekannt und können beispielsweise gemäß den in WO 2011/ 035874 A1 beschriebenen Methoden hergestellt werden.

Die Verbindungen der Formel (III), bei denen L eine Abgangsgruppe wie z. B.: Chlor, Brom, Jod, Methylsulfonyloxy, Tosyloxy oder Trifluormethylsulfonyloxy bedeutet sind entweder käuflich oder können nach bekannten in der Literatur beschriebenen Methoden hergestellt werden.

Erfindungsgemäße Verbindungen können auch nach der in Schema 2 angegebenen Methode durch Umsetzung eines Amins der Formel (IV) mit einem Säurechlorid (V), wie zum Beispiel in J. Het: Chem. (1972), 9 (1), 107-109 beschrieben, hergestellt werden:

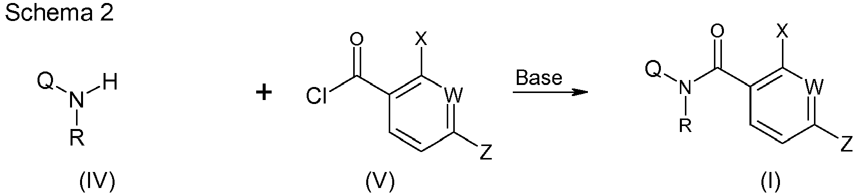

Erfindungsgemäße Verbindungen können auch nach der in Schema 3 angegebenen Methode durch Umsetzung eines Amins der Formel (IV) mit einer Säure der Formel (VI) hergestellt werden:

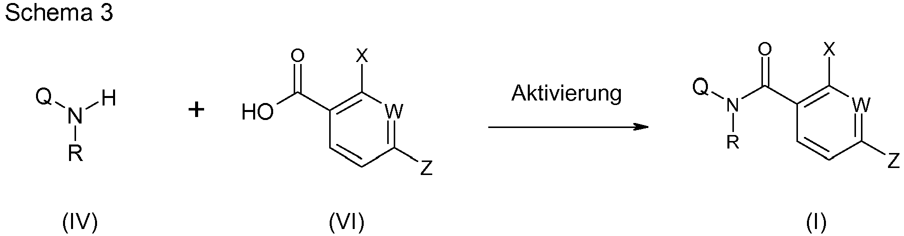

Für die Aktivierung können wasserentziehende Reagenzien, die üblicherweise für Amidierungsreaktionen, wie z. B. 1,1'-Carbonyldiimidazol (CDI), Dicyclohexylcarbodiimid (DCC), 2,4,6-Tripropyl-1,3,5,2,4,6-trioxatriphosphinane 2,4,6-trioxide (T3P) etc. eingesetzt werden.

Die Benzoesäurechloride der Formel (V) beziehungsweise die ihnen zugrunde liegenden Benzoesäuren der Formel (VI) sind grundsätzlich bekannt und können beispielsweise gemäß den in US 6,376,429 B1, EP 1 585 742 A1 und EP 1 202 978 A1 beschriebenen Methoden hergestellt werden.

Die Amine der Formel (IV) sind entweder käuflich oder in der Literatur bekannt oder können beispielsweise nach der in Schema 4 beschriebenen Methoden durch basenkatalysierte Alkylierung oder durch reduktive Alkylierung oder nach der in Schema 5 beschriebenen Methode durch nucleophile Substitution einer Abgangsgruppe L durch Amine R-NH₂ hergestellt werden.

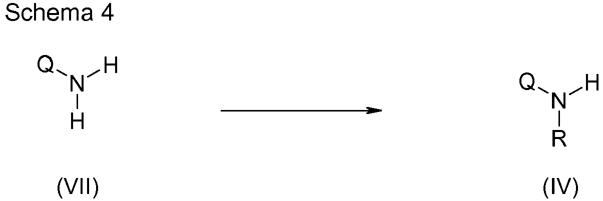

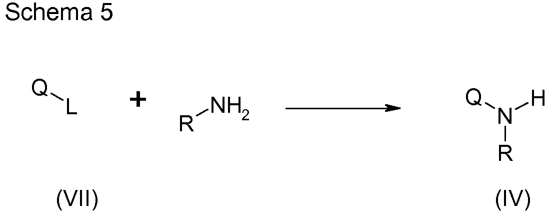

Die Amine der Formel (IV) können auch durch Cyclisierungsreaktionen wie zum Beispiel in J. Org. Chem. 73(10), 3738-3744 (2008) für Q = Q1 oder in Buletinul Institutului Politehnic din lasi (1974), 20(1-2), 95-99 oder in J. Org. Chem. 67(21), 7361-7364 (2002); für Q = Q4 beschrieben, hergestellt werden.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, Iowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasenunterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz- und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Bevorzugt ist die Anwendung der erfindungsgemäßen Verbindungen oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualität auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt, siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY, oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227, Wolter et al., Proc. Natl. Acad. Sci. USA 85 (1988), 846-850, Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.
Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Geeignete Safener sind beispielsweise Mefenpyr-diethyl, Cyprosulfamid, Isoxadifen-ethyl, Cloquintocet-mexyl und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 14th edition, The British Crop Protection Council and the Royal Soc. of Chemistry, 2003 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:
Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminopyralid, Amitrole, Ammoniumsulfamat, Ancymidol, Anilofos, Asulam, Atrazine, Azafenidin, Azimsulfuron, Aziprotryn, BAH-043, BAS-140H, BAS-693H, BAS-714H, BAS-762H, BAS-776H, BAS-800H, Beflubutamid, Benazolin, Benazolin-ethyl, bencarbazone, Benfluralin, Benfuresate, Bensulide, Bensulfuronmethyl, Bentazone, Benzfendizone, Benzobicyclon, Benzofenap, Benzofluor, Benzoylprop, Bifenox, Bilanafos, Bilanafos-natrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenac-natrium, Chlorfenprop, Chlorflurenol, Chlorflurenol-methyl, Chloridazon, Chlorimuron, Chlorimuron-ethyl, Chlormequatchlorid, Chlornitrofen, Chlorophthalim, Chlorthal-dimethyl, Chlorotoluron, Chlorsulfuron, Cinidon, Cinidon-ethyl, Cinmethylin, Cinosulfuron, Clethodim, Clodinafop Clodinafoppropargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyr-natrium, Dimefuron, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3fluorpropyl)-4,5-dihydro-5-oxo-1H-tetrazol-1-yl]-phenyl]-ethansulfonamid, Fenoprop, Fenoxaprop, Fenoxaprop-P, Fenoxaprop-ethyl, Fenoxaprop-P-ethyl, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofenethyl, Flupoxam, Flupropacil, Flupropanate, Flupyrsulfuron, Flupyrsulfuron-methylsodium, Flurenol, Flurenol-butyl, Fluridone, Flurochloridone, Fluroxypyr, Fluroxypyrmeptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, L-Glufosinate, L-Glufosinate-ammonium, Glufosinate-ammonium, Glyphosate, Glyphosate-isopropylammonium, H-9201, Halosafen, Halosulfuron, Halosulfuron-methyl, Haloxyfop, Haloxyfop-P, Haloxyfop-ethoxyethyl, Haloxyfop-P-ethoxyethyl, Haloxyfop-methyl, Haloxyfop-P-methyl, Hexazinone, HNPC-9908, HOK-201, HW-02, Imazamethabenz, Imazamethabenz-methyl, Imazamox, Imazapic, Imazapyr, Imazaquin, Imazethapyr, Imazosulfuron, Inabenfide, Indanofan, Indolessigsäure (IAA), 4-Indol-3-ylbuttersäure (IBA), lodosulfuron, lodosulfuron-methyl-natrium, loxynil, Isocarbamid, Isopropalin, Isoproturon, Isouron, Isoxaben, Isoxachlortole, Isoxaflutole, Isoxapyrifop, IDH-100, KUH-043, KUH-071, Karbutilate, Ketospiradox, Lactofen, Lenacil, Linuron, Maleinsäurehydrazid, MCPA, MCPB, MCPBmethyl, -ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Methazole, Methoxyphenone, Methyldymron, 1-Methylcyclopropen, Methylisothiocyanat, Metobenzuron, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monuron, MT 128, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-dichlorobenzoyl)-1-methyl-5-benzyloxypyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron-ethyl, Pyrazoxyfen, Pyribambenz, Pyribambenz-isopropyl, Pyribenzoxim, Pyributicarb, Pyridafol, Pyridate, Pyriftalid, Pyriminobac, Pyriminobac-methyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Secbumeton, Sethoxydim, Siduron, Simazine, Simetryn, SN-106279, Sulcotrione, Sulfallate (CDEC), Sulfentrazone, Sulfometuron, Sulfometuron-methyl, Sulfosate (Glyphosate-trimesium), Sulfosulfuron, SYN-523, SYP-249, SYP-298, SYP-300, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, TH-547, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapac-ethyl, Tritosulfuron, Tsitodef, Uniconazole, Uniconazole-P, Vernolate, ZJ-0166, ZJ-0270, ZJ-0543, ZJ-0862 sowie die folgenden Verbindungen

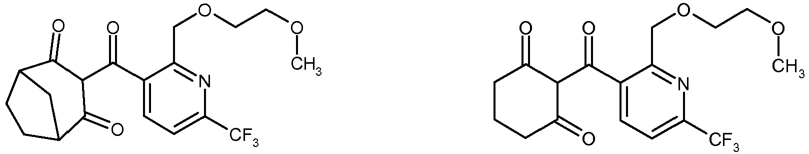

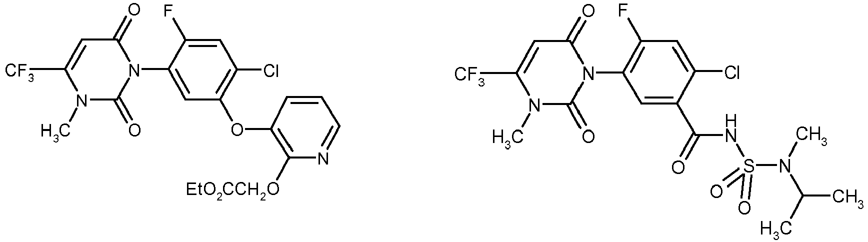

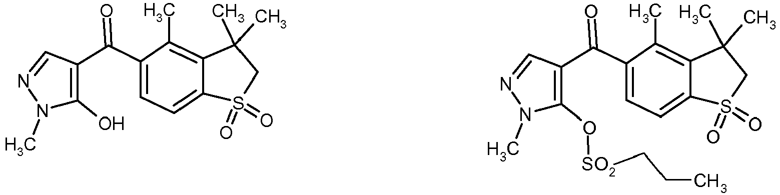

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

### Synthese von N-Ethyl-2-methyl-3-(methylsulfonyl)-N-(1-methyl-1 H-tetrazol-5-yl)-4-(trifluormethyl)benzamid (Tabellenbeispiel Nr. A-2)

100 mg (0,275 mmol) 2-Methyl-3-(methylsulfonyl)-N-(1-methyl-1 H-tetrazol-5-yl)-4-(trifluormethyl)benzamid und 43 mg (0,275 mmol) Jodethan werden in 5 ml N,N-Dimethylformamid gelöst und mit 0,038 mg (0,275 mmol) Kaliumcarbonat versetzt. Das Reaktionsgemisch wird 16 h bei 80 °C gerührt und in 5 ml Wasser aufgenommen und zweimal mit je 10 ml Dichlormethan extrahiert. Die organische Phase wird über Na₂SO₄ getrocknet und eingedampft. Der Rückstand wird über präparative HPLC (Acetonitril, Wasser) gereinigt. Ausbeute 30 mg (26%).
¹H-NMR (400 MHz; CDCl₃): 7.96 ppm (d, 0.5 H); 7.7 ppm (d, 0.5H), 7.67 (d, 0.5H), 7.38 (d; 0.5H); 4.10-3.61 (m, 5H); 3.28 (s, 1.5H); 3.18 (s, 1.5H); 2.83 (s, 3H); 1.32 (t; 1.5H), 1.11 (s; 1.5H).

### Synthese von 2-Chlor-3-[5-(cyanmethyl)-4,5-dihydro-1,2-oxazol-3-yl]-4-(ethylsulfonyl)-N-(1-methyl-1 H-tetrazol-5-yl)benzamid (Tabellenbeispiel Nr. A12)

178 mg (0,5 mmol) 2-Chlor-3-[5-(cyanmethyl)-4,5-dihydro-1,2-oxazol-3-yl]-4-(ethylsulfonyl)- benzoesäure und 90 mg (0,65 mmol) N-Allyl-1-methyl-1 H-tetrazol-5-amin werden in 3 ml Pyridin 1 h bei Raumtemperatur (RT) gerührt und dann mit einer Lösung von 86 mg (0,675 mmol) Oxalylchlorid in 2 ml Dichlorethan versetzt. Anschließend wird 4 h bei 75 °C gerührt. Nach dem Abkühlen auf RT werden 0,5 ml Wasser zugegeben. Dann wird 30 min bei RT gerührt. Die Reaktionslösung wird filtriert und eingeengt. Der Rückstand wird über präparative HPLC (Acetonitril / Wasser) gereinigt. Ausbeute: 19 mg (8%).
¹H-NMR (400 MHz; DMSO-d₆): 8.23; 8.15; 7.90 und 7.82; (4d, 2 H); 5.88 (m, 1H), 5.42 - 4.98 (m, 3H), 4.55 (bs, 1 H), 4.29 (d, 1 H), 4.10 and 4.04 (2s, 3H), 3.67 - 2.98 (m, 7H), 1.18 and 1.04 (2t, 3H).

### Synthese von Methyl-[2-(methylsulfonyl)-4-(trifluormethyl)benzoyl](1-methyl-1 H-tetrazol-5-yl)carbamat (Tabellenbeispiel Nr. A14)

Zu einer Lösung von 175 mg (0,5 mmol) 2-(Methylsulfonyl)-N-(1-methyl-1H-tetrazol-5-yl)-4-(trifluormethyl)benzamid in 4 ml THF (abs.) werden unter Schutzgas bei 0°C 14 mg (0,55 mmol) Natriumhydrid (95 %ig) zugegeben. Nach 10 min. Rühren werden bei 0°C 46 µl (0,6 mmol) Chlorameisensäuremethylester zugegeben. Das Reaktionsgemisch wird 1 h bei RT gerührt. Anschließend wird das Reaktionsgemisch mit einer wäßrigen ges. Natriumhydrogencarbonatlösung auf pH 7 eingestellt und mit 10 ml Wasser versetzt. Dann wird mit Essigester extrahiert. Die organische Phase wird über Natriumsulfat getrocknet, filtriert und eingeengt. Ausbeute: 231 mg (57 %)
¹H-NMR (400 MHz; DMSO-d₆): 8.40 (s, 1H), 8.31 (d, 1H), 8.15 (d, 1H), 4.14 (s, 3H), 3.67 (s, 3H); 3.38 (s, 3H).

### Synthese von N-Allyl-2-methyl-N-(4-methyl-1,2,5-oxadiazol-3-yl)-3-(methylsulfonyl)-4-(trifluormethyl)benzamid (Tabellenbeispiel Nr. C-1)

150 mg (0,413 mmol) 2-Methyl-N-(4-methyl-1,2,5-oxadiazol-3-yl)-3-(methylsulfonyl)-4-(trifluormethyl)benzamid und 50 mg (0,413 mmol) Allylbromid werden in 5 ml N,N-Dimethylformamid gelöst und mit 0,057 mg (0,413 mmol) Kaliumcarbonat versetzt. Das Reaktionsgemisch wird 8 h bei 80 °C gerührt und in 5 ml Wasser aufgenommen und zweimal mit je 10 ml Dichlormethan extrahiert: Die organische Phase wird über Na₂SO₄ getrocknet und eingedampft. Der Rückstand wird über präparative HPLC (Acetonitril, Wasser) gereinigt. Ausbeute 160 mg (86%).
¹H-NMR (400 MHz; DMSO-d₆): 8.10-8.01 ppm (b, 1 H); 7.84 ppm (d, 0.5H), 7.75 (d, 0.5H), 7,38 (d, 0.5H); 5.98-5.75(m, 1 H); 5.38 (d, 0.5H); 5.26 (d, 0.5H); 5.14 (d, 0.5H); 5.02 (d, 0.5H); 4.19 (d, 2H); 3.46 (s, 1.5H); 3.30 (s; 1.5H), 2.89 (s; 1.5H); 2.70 (s, 1.5H); 2.43 (s, 3H).

### Synthese von N-Methyl-N-(5-methyl-1,3,4-oxadiazol-2-yl)-2-methyl-3-(methylsulfonyl)-4-(trifluormethyl)benzamid (Tabellenbeispiel Nr. D1)

180 mg (0,495 mmol) 2-Methyl-N-(5-methyl-1,3,4-oxadiazol-2-yl)-3-(methylsulfonyl)-4-(trifluoromethyl)benzamid werden in 6 ml DMF (abs.) gelöst und mit 68 mg (0,495 mmol) K₂CO₃ und 0,047 ml (0,495 mmol) Dimethylsulfat versetzt. Das Reaktionsgemisch wird 15 Stunden unter Rückfluß gekocht. Dann wird filtriert, das Filtrat wird eingeengt und säulenchromatographisch (Kieselgel; Heptan / Essigester) gereinigt.
¹H-NMR (400 MHz; CDCl₃): 7.98 (d, 1H), 7.49 (d, 1H), 3.57 (bs, 3H), 3.23 (s, 3H), 2.92 (s, 3H), 2.37 (s, 3H).

Die in den nachfolgenden Tabellen aufgeführten Beispiele wurden analog oben genannten Methoden hergestellt beziehungsweise sind analog oben genannten Methoden erhältlich. Die in den nachfolgenden Tabellen 1 bis 336 angebenenen Verbindungen, die analog der hier genannten Methoden erhältlich sind, sind besonders bevorzugt.

Die verwendeten Abkürzungen bedeuten:
Et = Ethyl Me = Methyl n-Pr = n-Propyl i-Pr = Isopropyl c-Pr = Cyclopropyl Ph = Phenyl Bn = Benzyl Prg = Propargyl

Tabelle 1: Erfindungsgemäße Verbindungen der Formel (I), worin Q für Q1, R⁶ für Methyl, X für Chlor, W für C-Y, Y für (2,2,2-Trifluorethoxy)methyl, V für Wasserstoff und Z für Methylsulfonyl steht:

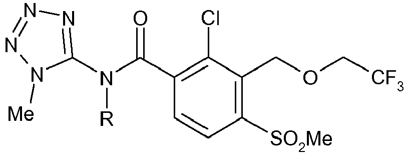

| Nr. | R | Physikalische Daten, (¹H-NMR, DMSO-d₆, 400 MHz) |
|---|---|---|
| 1-1 | Me | |
| 1-2 | Et | |
| 1-3 | i-Pr | |
| 1-4 | CH₂CHF₂ | |
| 1-5 | CH₂CF₃ | |
| 1-6 | CH₂OMe | |
| 1-7 | CH₂OEt | |
| 1-8 | CH₂OPh | |
| 1-9 | CH₂SMe | |
| 1-10 | CH₂SEt | |
| 1-11 | CH₂SPh | |
| 1-12 | CH₂SO₂Me | |
| 1-13 | CH₂CH₂CN | |
| 1-14 | CH₂CH₂OMe | |
| 1-15 | CH₂CH₂OEt | |
| 1-16 | CH₂CH₂OPh | |
| 1-17 | CH₂CH₂SMe | |
| 1-18 | CH₂CH₂S(O)Me | |
| 1-19 | CH₂CH₂SO₂Me | |
| 1-20 | CH₂CH₂SEt | |
| 1-21 | CH₂CH₂S(O)Et | |
| 1-22 | CH₂CH₂SO₂Et | |
| 1-23 | CH₂CH₂SPh | |
| 1-24 | CH₂CH₂S(O)Ph | |
| 1-25 | CH₂CH₂SO₂Ph | |
| 1-26 | CH₂C(O)Me | |
| 1-27 | CH₂C(O)Ph | |
| 1-28 | CH₂CO₂He | |
| 1-29 | CH₂CO₂Me | |
| 1-30 | CH₂CO₂Et | |
| 1-31 | CH₂CN | |
| 1-32 | CH₂CH₂CN | |
| 1-33 | Allyl | |
| 1-34 | Prg | |
| 1-35 | Benzyl | |
| 1-36 | CO₂Me | |
| 1-37 | CO₂Et | |
| 1-38 | CO₂-iPr | |
| 1-39 | CO₂CH₂-CHMe₂ | |
| 1-40 | CO₂CH₂-t-Bu | |

Tabelle 2: Erfindungsgemäße Verbindungen der Formel (I), worin Q für Q1, R⁶ für Ethyl, X für Chlor, W für C-Y, Y für (2,2,2-Trifluorethoxy)methyl, V für Wasserstoff und Z für Methylsulfonyl steht, und R die in Tabelle 1 angegebenen Bedeutungen hat:

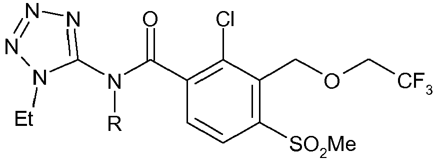

Tabelle 3: Erfindungsgemäße Verbindungen der Formel (I), worin Q für Q1, R⁶ für Methyl, X für Chlor, W für C-Y, Y für 4,5-Dihydro-1,2-oxazol-3-yl, V für Wasserstoff und Z für Methylsulfonyl steht, und R die in Tabelle 1 angegebenen Bedeutungen hat:

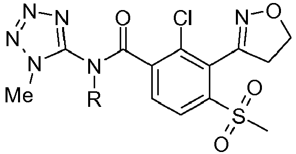

Tabelle 4: Erfindungsgemäße Verbindungen der Formel (I), worin Q für Q1, R⁶ für Methyl, X für Chlor, W für C-Y, Y für 4,5-Dihydro-1,2-oxazol-3-yl, V für Wasserstoff und Z für Ethylsulfonyl steht, und R die in Tabelle 1 angegebenen Bedeutungen hat:

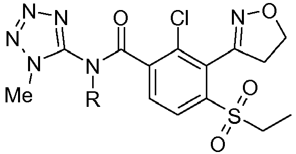

Tabelle 5: Erfindungsgemäße Verbindungen der Formel (I), worin Q für Q1, R⁶ für Methyl, X für Chlor, W für C-Y, Y für 5-Cyanmethyl-4,5-dihydro-1,2-oxazol-3-yl, V für Wasserstoff und Z für Ethylsulfonyl steht, und R die in Tabelle 1 angegebenen Bedeutungen hat:

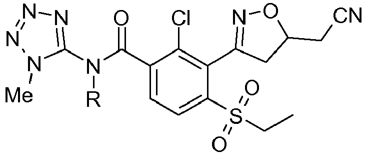

Tabelle 6: Erfindungsgemäße Verbindungen der Formel (I), worin Q für Q1, R⁶ für Methyl, X für Chlor, W für C-Y, Y für 5-(Methoxymethyl)-4,5-dihydro-1,2-oxazol-3-yl, V für Wasserstoff und Z für Ethylsulfonyl steht, und R die in Tabelle 1 angegebenen Bedeutungen hat:

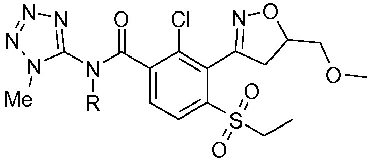

Tabelle 7: Erfindungsgemäße Verbindungen der Formel (I), worin Q für Q1, R⁶ für Methyl, X für Chlor, W für C-Y, Y für Methoxymethyl, V für Wasserstoff und Z für Methylsulfonyl steht, und R die in Tabelle 1 angegebenen Bedeutungen hat:

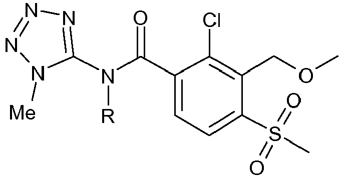

Tabelle 8: Erfindungsgemäße Verbindungen der Formel (I), worin Q für Q1, R⁶ für Ethyl, X für Chlor, W für C-Y, Y für Ethoxymethyl, V für Wasserstoff und Z für Methylsulfonyl steht, und R die in Tabelle 1 angegebenen Bedeutungen hat:

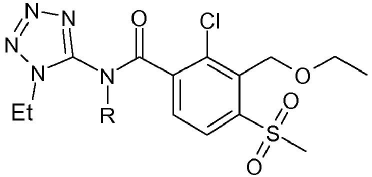

Tabelle 9: Erfindungsgemäße Verbindungen der Formel (I), worin Q für Q1, R⁶ für Methyl, X für Chlor, W für C-Y, Y für (2-Methoxy-ethoxy)-methyl, V für Wasserstoff und Z für Methylsulfonyl steht, und R die in Tabelle 1 angegebenen Bedeutungen hat:

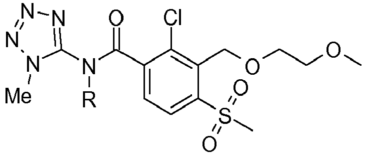

Tabelle 10: Erfindungsgemäße Verbindungen der Formel (I), worin Q für Q1, R⁶ für Methyl, X für Chlor, W für C-Y, Y für Methoxy, V für Wasserstoff und Z für Methylsulfonyl steht, und R die in Tabelle 1 angegebenen Bedeutungen hat:

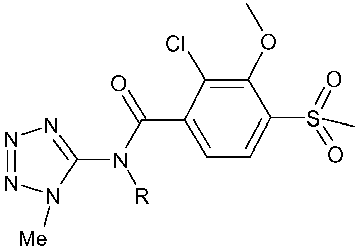

Tabelle 11: Erfindungsgemäße Verbindungen der Formel (I), worin Q für Q1, R⁶ für Methyl, X für Chlor, W für C-Y, Y für Methoxy, V für Wasserstoff und Z für Ethylsulfonyl steht, und R die in Tabelle 1 angegebenen Bedeutungen hat:

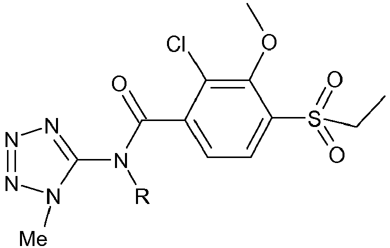

Tabelle 12: Erfindungsgemäße Verbindungen der Formel (I), worin Q für Q1, R⁶ für Methyl, X für Chlor, W für C-Y, Y für Ethoxy, V für Wasserstoff und Z für Methylsulfonyl steht, und R die in Tabelle 1 angegebenen Bedeutungen hat:

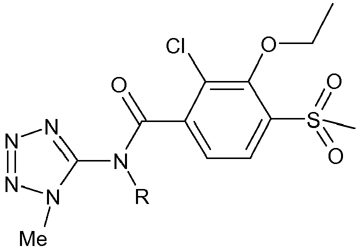

Tabelle 13: Erfindungsgemäße Verbindungen der Formel (I), worin Q für Q1, R⁶ für Methyl, X für Chlor, W für C-Y, Y für Ethoxy, V für Wasserstoff und Z für Ethylsulfonyl steht, und R die in Tabelle 1 angegebenen Bedeutungen hat:

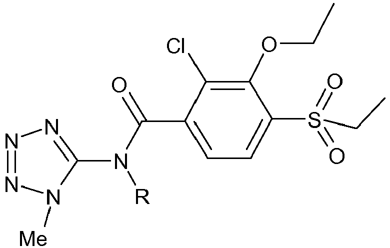

Tabelle 14: Erfindungsgemäße Verbindungen der Formel (I), worin Q für Q1, R⁶ für Ethyl, X für Chlor, W für C-Y, Y für Ethoxy, V für Wasserstoff und Z für Methylsulfonyl steht, und R die in Tabelle 1 angegebenen Bedeutungen hat:

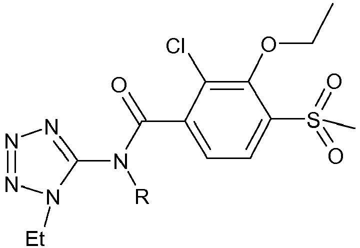

Tabelle 15: Erfindungsgemäße Verbindungen der Formel (I), worin Q für Q1, R⁶ für Methyl, X für Chlor, W für C-Y, Y für Allyloxy, V für Wasserstoff und Z für Methylsulfonyl steht, und R die in Tabelle 1 angegebenen Bedeutungen hat:

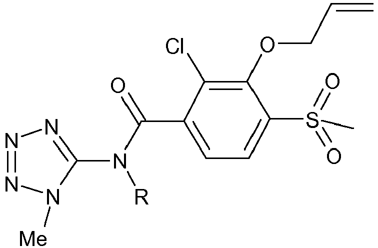

Tabelle 16: Erfindungsgemäße Verbindungen der Formel (I), worin Q für Q1, R⁶ für Methyl, X für Chlor, W für C-Y, Y für 2-Fluorethoxy, V für Wasserstoff und Z für Methylsulfonyl steht, und R die in Tabelle 1 angegebenen Bedeutungen hat:

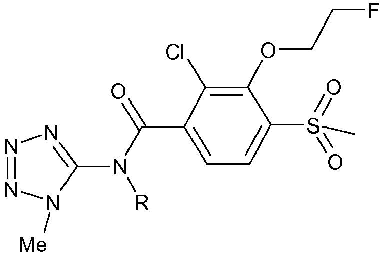

Tabelle 17: Erfindungsgemäße Verbindungen der Formel (I), worin Q für Q1, R⁶ für Methyl, X für Chlor, W für C-Y, Y für Propoxy, V für Wasserstoff und Z für Ethylsulfonyl steht, und R die in Tabelle 1 angegebenen Bedeutungen hat:

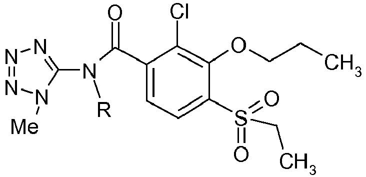

Tabelle 18: Erfindungsgemäße Verbindungen der Formel (I), worin Q für Q1, R⁶ für Methyl, X für Chlor, W für C-Y, Y für Propoxy, V für Wasserstoff und Z für Methylsulfonyl steht, und R die in Tabelle 1 angegebenen Bedeutungen hat:

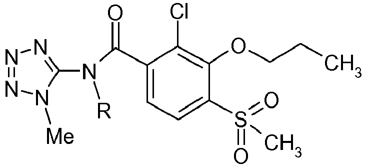

Tabelle 19: Erfindungsgemäße Verbindungen der Formel (I), worin Q für Q1, R⁶ für Methyl, X für Chlor, W für C-Y, Y für (3-Methylsulfanyl)-propoxy, V für Wasserstoff und Z für Chlor steht, und R die in Tabelle 1 angegebenen Bedeutungen hat:

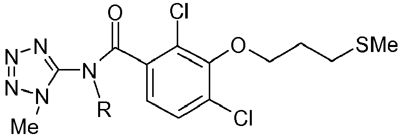

Tabelle 20: Erfindungsgemäße Verbindungen der Formel (I), worin Q für Q1, R⁶ für Methyl, X für Chlor W für C-Y, Y für (2-Methylsulfanyl)-ethoxy, V für Wasserstoff und Z für Chlor steht, und R die in Tabelle 1 angegebenen Bedeutungen hat:

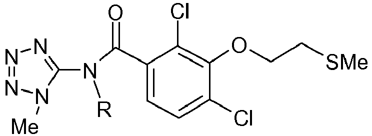

Tabelle 21: Erfindungsgemäße Verbindungen der Formel (I), worin Q für Q1, R⁶ für Methyl, X für Chlor, W für C-Y, Y für Cyclopropylmethoxy, V für Wasserstoff und Z für Methylsulfonyl steht, und R die in Tabelle 1 angegebenen Bedeutungen hat:

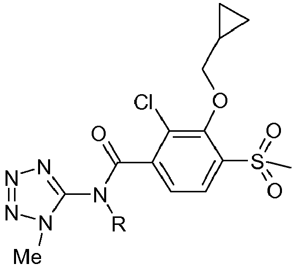

Tabelle 22: Erfindungsgemäße Verbindungen der Formel (I), worin Q für Q1, R⁶ für Methyl, X für Chlor, W für C-Y, Y für Cyclopropylmethoxy, V für Wasserstoff und Z für Ethylsulfonyl steht, und R die in Tabelle 1 angegebenen Bedeutungen hat:

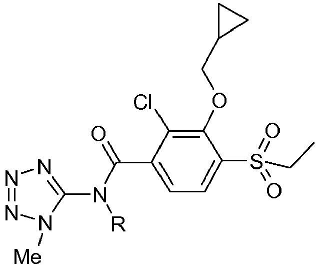

Tabelle 23: Erfindungsgemäße Verbindungen der Formel (I), worin Q für Q1, R⁶ für Methyl, X für Chlor, W für C-Y, Y für (2-Dimethylamino)-2-oxoethoxy, V für Wasserstoff und Z für Brom steht, und R die in Tabelle 1 angegebenen Bedeutungen hat:

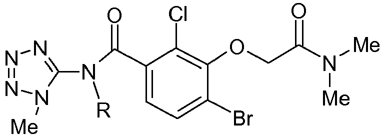

Tabelle 24: Erfindungsgemäße Verbindungen der Formel (I), worin Q für Q1, R⁶ für Methyl, X für Chlor, W für C-Y, Y für Methoxycarbonyl, V für Wasserstoff und Z für Methylsulfonyl steht, und R die in Tabelle 1 angegebenen Bedeutungen hat:

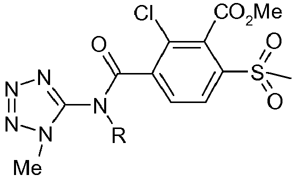

Tabelle 25: Erfindungsgemäße Verbindungen der Formel (I), worin Q für Q1, R⁶ für Methyl, X für Chlor, W für C-Y, Y für (Ethoxyimino)methyl, V für Wasserstoff und Z für Methylsulfonyl steht, und R die in Tabelle 1 angegebenen Bedeutungen hat:

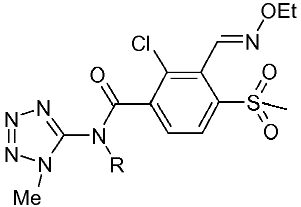

Tabelle 26: Erfindungsgemäße Verbindungen der Formel (I), worin Q für Q1, R⁶ für Methyl, X für Chlor, W für C-Y, Y für 1-(Ethylamino)-1-oxopropan-2-yl]amino, V für Wasserstoff und Z für Chlor steht, und R die in Tabelle 1 angegebenen Bedeutungen hat:

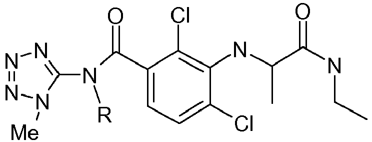

Tabelle 27: Erfindungsgemäße Verbindungen der Formel (I), worin Q für Q1, R⁶ für Methyl, X für Chlor, W für C-Y, Y für Methylsulfanyl, V für Wasserstoff und Z für Chlor steht, und R die in Tabelle 1 angegebenen Bedeutungen hat:

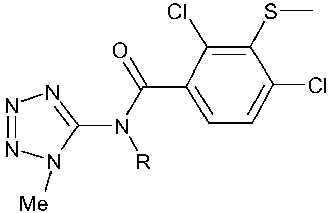

Tabelle 28: Erfindungsgemäße Verbindungen der Formel (I), worin Q für Q1, R⁶ für Methyl, X für Chlor, W für C-Y, Y für Methylsulfanyl, V für Wasserstoff und Z für Trifluormethyl steht, und R die in Tabelle 1 angegebenen Bedeutungen hat:

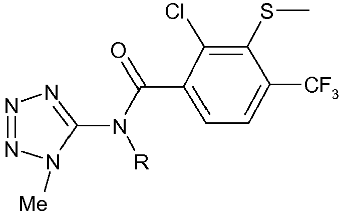

Tabelle 29: Erfindungsgemäße Verbindungen der Formel (I), worin Q für Q1, R⁶ für Ethyl, X für Chlor, W für C-Y, Y für Methylsulfanyl, V für Wasserstoff und Z für Chlor steht, und R die in Tabelle 1 angegebenen Bedeutungen hat:

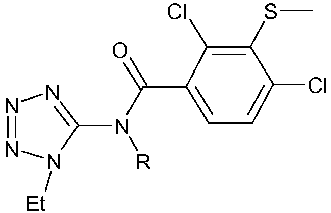

Tabelle 30: Erfindungsgemäße Verbindungen der Formel (I), worin Q für Q1, R⁶ für Methyl, X für Chlor, W für C-Y, Y für Methylsulfinyl, V für Wasserstoff und Z für Chlor steht, und R die in Tabelle 1 angegebenen Bedeutungen hat:

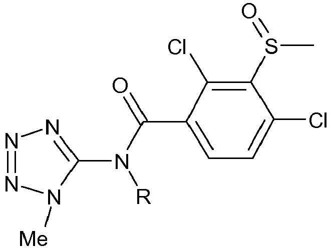

Tabelle 31: Erfindungsgemäße Verbindungen der Formel (I), worin Q für Q1, R⁶ für Ethyl, X für Chlor, W für C-Y, Y für Methylsulfinyl, V für Wasserstoff und Z für Chlor steht, und R die in Tabelle 1 angegebenen Bedeutungen hat:

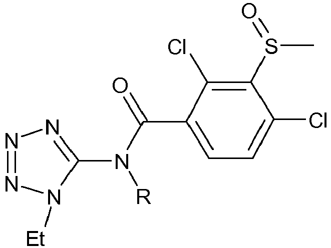

Tabelle 32: Erfindungsgemäße Verbindungen der Formel (I), worin Q für Q1, R⁶ für Methyl, X für Chlor, W für C-Y, Y für Methylsulfinyl, V für Wasserstoff und Z für Methyl steht, und R die in Tabelle 1 angegebenen Bedeutungen hat:

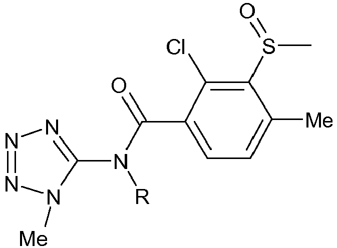

Tabelle 33: Erfindungsgemäße Verbindungen der Formel (I), worin Q für Q1, R⁶ für Methyl, X für Chlor, W für C-Y, Y für Methylsulfanyl, V für Wasserstoff und Z für Methyl steht, und R die in Tabelle 1 angegebenen Bedeutungen hat:

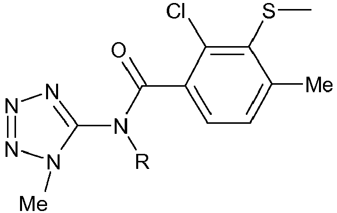

Tabelle 34: Erfindungsgemäße Verbindungen der Formel (I), worin Q für Q1, R⁶ für Methyl, X für Chlor, W für C-Y, Y für Methylsulfonyl, V für Wasserstoff und Z für Chlor steht, und R die in Tabelle 1 angegebenen Bedeutungen hat:

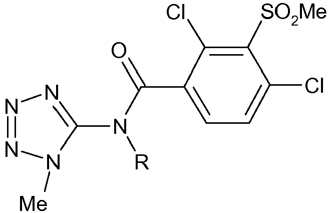

Tabelle 35: Erfindungsgemäße Verbindungen der Formel (I), worin Q für Q1, R⁶ für Ethyl, X für Chlor, W für C-Y, Y für Methylsulfonyl, V für Wasserstoff und Z für Chlor steht, und R die in Tabelle 1 angegebenen Bedeutungen hat:

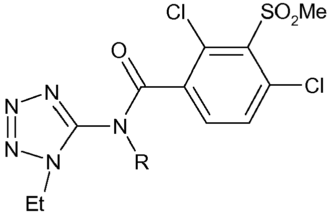

Tabelle 36: Erfindungsgemäße Verbindungen der Formel (I), worin Q für Q1, R⁶ für Methyl, X für Chlor, W für C-Y, Y für Methylsulfonyl, V für Wasserstoff und Z für Trifluormethyl steht, und R die in Tabelle 1 angegebenen Bedeutungen hat:

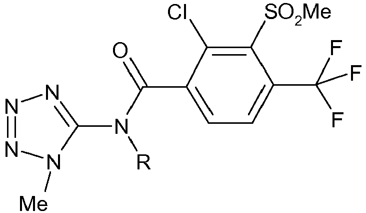

Tabelle 37: Erfindungsgemäße Verbindungen der Formel (I), worin Q für Q1, R⁶ für Ethyl, X für Chlor, W für C-Y, Y für Methylsulfonyl, V für Wasserstoff und Z für Trifluormethyl steht, und R die in Tabelle 1 angegebenen Bedeutungen hat:

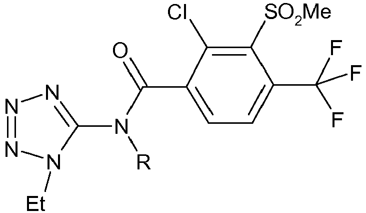

Tabelle 38: Erfindungsgemäße Verbindungen der Formel (I), worin Q für Q1, R⁶ für Methyl, X für Chlor, W für C-Y, Y für Methylsulfonyl, V für Wasserstoff und Z für Methylsulfonyl steht, und R die in Tabelle 1 angegebenen Bedeutungen hat:

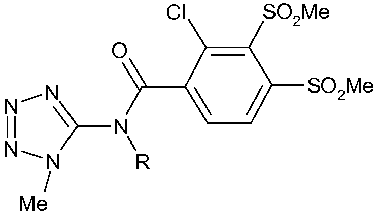

Tabelle 39: Erfindungsgemäße Verbindungen der Formel (I), worin Q für Q1, R⁶ für Ethyl, X für Chlor, W für C-Y, Y für Methylsulfonyl, V für Wasserstoff und Z für Methylsulfonyl steht, und R die in Tabelle 1 angegebenen Bedeutungen hat:

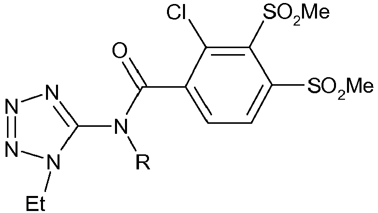

Tabelle 40: Erfindungsgemäße Verbindungen der Formel (I), worin Q für Q1, R⁶ für Methyl, X für Chlor, W für C-Y, Y für Ethylsulfonyl, V für Wasserstoff und Z für Chlor steht, und R die in Tabelle 1 angegebenen Bedeutungen hat:

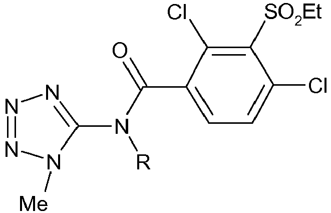

Tabelle 41: Erfindungsgemäße Verbindungen der Formel (I), worin Q für Q1, R⁶ für Methyl, X für Chlor, W für C-Y, Y für Ethylsulfinyl, V für Wasserstoff und Z für Chlor steht, und R die in Tabelle 1 angegebenen Bedeutungen hat:

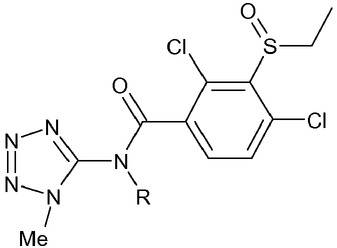

Tabelle 42: Erfindungsgemäße Verbindungen der Formel (I), worin Q für Q1, R⁶ für Methyl, X für Chlor, W für C-Y, Y für Ethylsulfinyl, V für Wasserstoff und Z für Methyl steht, und R die in Tabelle 1 angegebenen Bedeutungen hat:

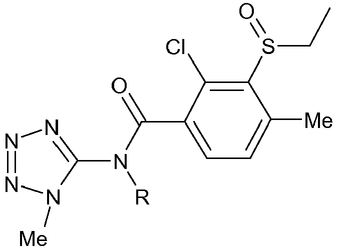

Tabelle 43: Erfindungsgemäße Verbindungen der Formel (I), worin Q für Q1, R⁶ für Methyl, X für Chlor, W für C-Y, Y für Methylsulfonyl, V für Wasserstoff und Z für Methyl steht, und R die in Tabelle 1 angegebenen Bedeutungen hat:

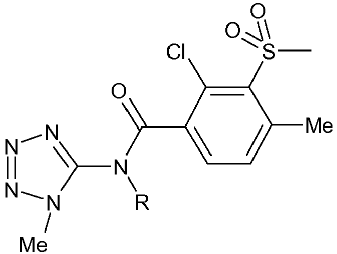

Tabelle 44: Erfindungsgemäße Verbindungen der Formel (I), worin Q für Q1, R⁶ für Methyl, X für Chlor, W für C-Y, Y für Ethylsulfanyl, V für Wasserstoff und Z für Methyl steht, und R die in Tabelle 1 angegebenen Bedeutungen hat:

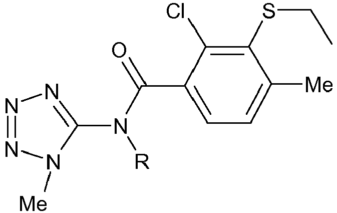

Tabelle 45: Erfindungsgemäße Verbindungen der Formel (I), worin Q für Q1, R⁶ für Methyl, X für Chlor, W für C-Y, Y für Ethylsulfonyl, V für Wasserstoff und Z für Methyl steht, und R die in Tabelle 1 angegebenen Bedeutungen hat:

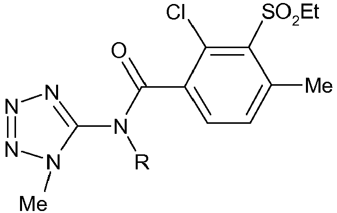

Tabelle 46: Erfindungsgemäße Verbindungen der Formel (I), worin Q für Q1, R⁶ für Methyl, X für Chlor, W für C-Y, Y für 2-Methoxyethylsulfinyl, V für Wasserstoff und Z für Chlor steht, und R die in Tabelle 1 angegebenen Bedeutungen hat:

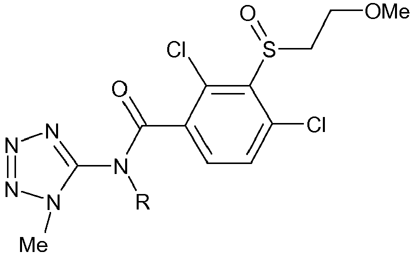

Tabelle 47: Erfindungsgemäße Verbindungen der Formel (I), worin Q für Q1, R⁶ für Methyl, X für Brom, W für C-Y, Y für Methylsulfanyl, V für Wasserstoff und Z für Methyl steht, und R die in Tabelle 1 angegebenen Bedeutungen hat:

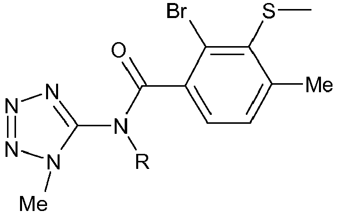

Tabelle 48: Erfindungsgemäße Verbindungen der Formel (I), worin Q für Q1, R⁶ für Methyl, X für Brom, W für C-Y, Y für Methylsulfinyl, V für Wasserstoff und Z für Methyl steht, und R die in Tabelle 1 angegebenen Bedeutungen hat:

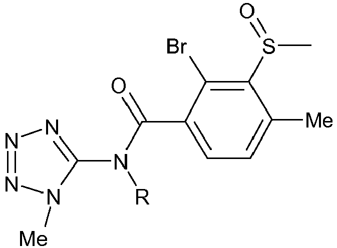

Tabelle 49: Erfindungsgemäße Verbindungen der Formel (I), worin Q für Q1, R⁶ für Methyl, X für Brom, W für C-Y, Y für Methylsulfonyl, V für Wasserstoff und Z für Methyl steht, und R die in Tabelle 1 angegebenen Bedeutungen hat:

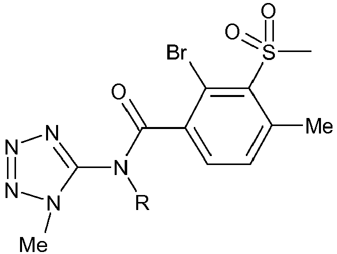

Tabelle 50: Erfindungsgemäße Verbindungen der Formel (I), worin Q für Q1, R⁶ für Methyl, X für Brom, W für C-Y, Y für Ethylsulfanyl, V für Wasserstoff und Z für Methyl steht, und R die in Tabelle 1 angegebenen Bedeutungen hat:

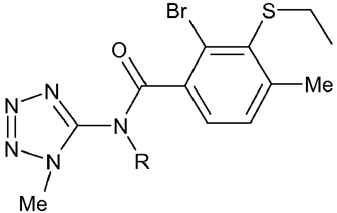

Tabelle 51: Erfindungsgemäße Verbindungen der Formel (I), worin Q für Q1, R⁶ für Methyl, X für Brom, W für C-Y, Y für Ethylsulfinyl, V für Wasserstoff und Z für Methyl steht, und R die in Tabelle 1 angegebenen Bedeutungen hat:

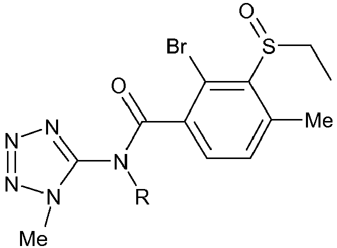

Tabelle 52: Erfindungsgemäße Verbindungen der Formel (I), worin Q für Q1, R⁶ für Methyl, X für Brom, W für C-Y, Y für Ethylsulfonyl, V für Wasserstoff und Z für Methyl steht, und R die in Tabelle 1 angegebenen Bedeutungen hat:

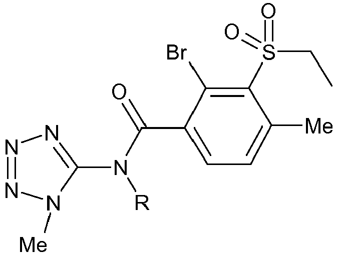

Tabelle 53: Erfindungsgemäße Verbindungen der Formel (I), worin Q für Q1, R⁶ für Methyl, X für Methyl, W für C-Y, Y für 1 H-Pyrazol-1-yl, V für Wasserstoff und Z für Methylsulfonyl steht, und R die in Tabelle 1 angegebenen Bedeutungen hat:

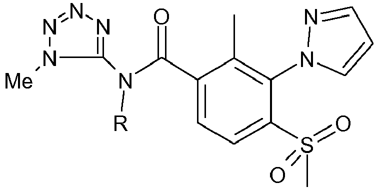

Tabelle 54: Erfindungsgemäße Verbindungen der Formel (I), worin Q für Q1, R⁶ für Ethyl, X für Methyl, W für C-Y, Y für 1 H-Pyrazol-1-yl, V für Wasserstoff und Z für Methylsulfonyl steht, und R die in Tabelle 1 angegebenen Bedeutungen hat:

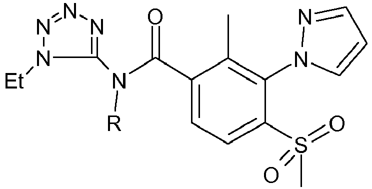

Tabelle 55: Erfindungsgemäße Verbindungen der Formel (I), worin Q für Q1, R⁶ für Methyl, X für Methyl, W für C-Y, Y für 4,5-Dihydro-1,2-oxazol-3-yl, V für Wasserstoff und Z für Methylsulfonyl steht, und R die in Tabelle 1 angegebenen Bedeutungen hat:

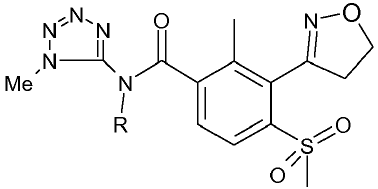

Tabelle 56: Erfindungsgemäße Verbindungen der Formel (I), worin Q für Q1, R⁶ für Ethyl, X für Methyl, W für C-Y, Y für 4,5-Dihydro-1,2-oxazol-3-yl, V für Wasserstoff und Z für Methylsulfonyl steht, und R die in Tabelle 1 angegebenen Bedeutungen hat:

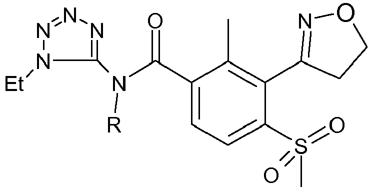

Tabelle 57: Erfindungsgemäße Verbindungen der Formel (I), worin Q für Q1, R⁶ für Methyl, X für Methyl, W für C-Y, Y für (Cyclopropylmethyl)amino, V für Wasserstoff und Z für Methylsulfonyl steht, und R die in Tabelle 1 angegebenen Bedeutungen hat:

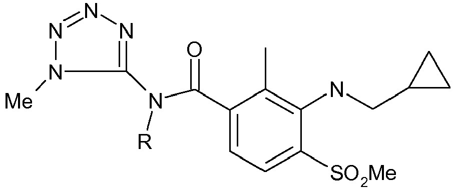

Tabelle 58: Erfindungsgemäße Verbindungen der Formel (I), worin Q für Q1, R⁶ für Methyl, X für Methyl, W für C-Y, Y für Propylamino, V für Wasserstoff und Z für Methylsulfonyl steht, und R die in Tabelle 1 angegebenen Bedeutungen hat:

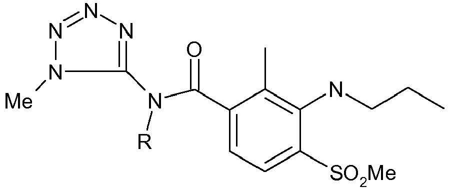

Tabelle 59: Erfindungsgemäße Verbindungen der Formel (I), worin Q für Q1, R⁶ für Methyl, X für Methyl, W für C-Y, Y für Methylsulfanyl, V für Wasserstoff und Z für Methylsulfonyl steht, und R die in Tabelle 1 angegebenen Bedeutungen hat:

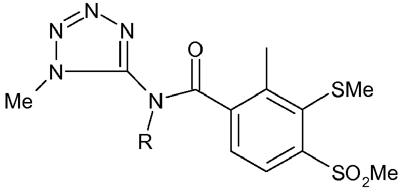

Tabelle 60: Erfindungsgemäße Verbindungen der Formel (I), worin Q für Q1, R⁶ für Methyl, X für Methyl, W für C-Y, Y für Methylsulfonyl, V für Wasserstoff und Z für Methylsulfonyl steht, und R die in Tabelle 1 angegebenen Bedeutungen hat:

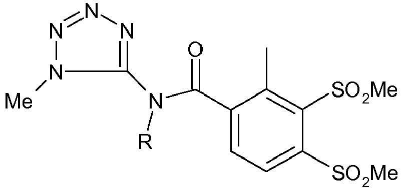

Tabelle 61: Erfindungsgemäße Verbindungen der Formel (I), worin Q für Q1, R⁶ für Ethyl, X für Methyl, W für C-Y, Y für Methylsulfonyl, V für Wasserstoff und Z für Methylsulfonyl steht, und R die in Tabelle 1 angegebenen Bedeutungen hat:

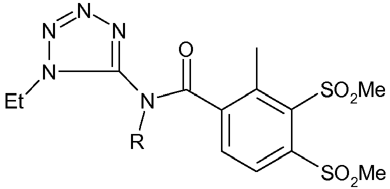

Tabelle 62: Erfindungsgemäße Verbindungen der Formel (I), worin Q für Q1, R⁶ für Methyl, X für Methyl, W für C-Y, Y für Ethylsulfonyl, V für Wasserstoff und Z für Methylsulfonyl steht, und R die in Tabelle 1 angegebenen Bedeutungen hat:

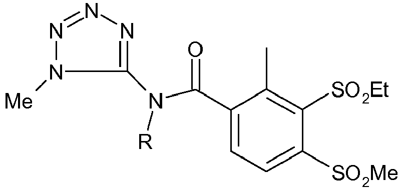

Tabelle 63: Erfindungsgemäße Verbindungen der Formel (I), worin Q für Q1, R⁶ für Methyl, X für Methyl, W für C-Y, Y für Methylsulfanyl, V für Wasserstoff und Z für Trifluormethyl steht, und R die in Tabelle 1 angegebenen Bedeutungen hat:

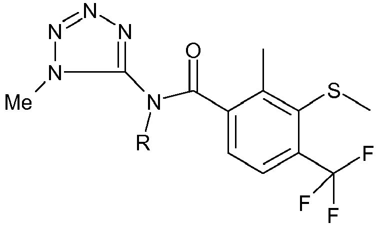

Tabelle 64: Erfindungsgemäße Verbindungen der Formel (I), worin Q für Q1, R⁶ für Ethyl, X für Methyl, W für C-Y, Y für Methylsulfanyl, V für Wasserstoff und Z für Trifluormethyl steht, und R die in Tabelle 1 angegebenen Bedeutungen hat:

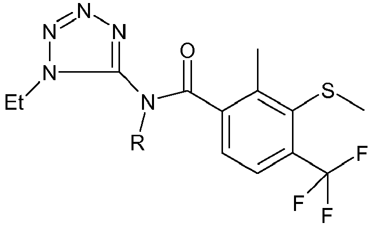

Tabelle 65: Erfindungsgemäße Verbindungen der Formel (I), worin Q für Q1, R⁶ für Methyl, X für Methyl, W für C-Y, Y für Methylsulfanyl, V für Wasserstoff und Z für Chlor steht, und R die in Tabelle 1 angegebenen Bedeutungen hat:

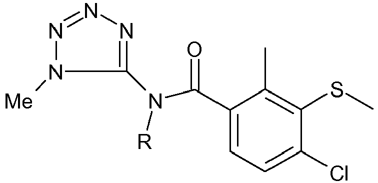

Tabelle 66: Erfindungsgemäße Verbindungen der Formel (I), worin Q für Q1, R⁶ für Ethyl, X für Methyl, W für C-Y, Y für Methylsulfanyl, V für Wasserstoff und Z für Chlor steht, und R die in Tabelle 1 angegebenen Bedeutungen hat:

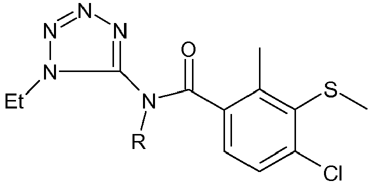

Tabelle 67: Erfindungsgemäße Verbindungen der Formel (I), worin Q für Q1, R⁶ für Methyl, X für Methyl, W für C-Y, Y für Methylsulfanyl, V für Wasserstoff und Z für Brom steht, und R die in Tabelle 1 angegebenen Bedeutungen hat:

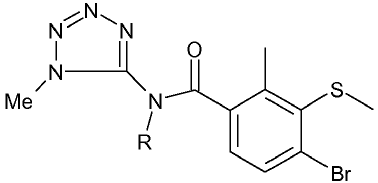

Tabelle 68: Erfindungsgemäße Verbindungen der Formel (I), worin Q für Q1, R⁶ für Methyl, X für Methyl, W für C-Y, Y für Methylsulfinyl, V für Wasserstoff und Z für Brom steht, und R die in Tabelle 1 angegebenen Bedeutungen hat:

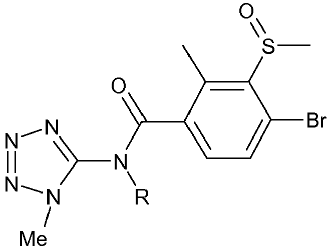

Tabelle 69: Erfindungsgemäße Verbindungen der Formel (I), worin Q für Q1, R⁶ für Methyl, X für Methyl, W für C-Y, Y für Ethylsulfinyl, V für Wasserstoff und Z für Brom steht, und R die in Tabelle 1 angegebenen Bedeutungen hat:

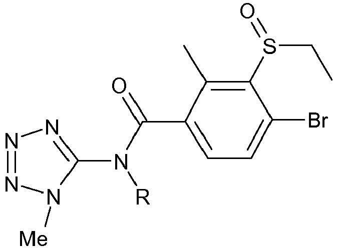

Tabelle 70: Erfindungsgemäße Verbindungen der Formel (I), worin Q für Q1, R⁶ für Methyl, X für Methyl, W für C-Y, Y für Ethylsulfonyl, V für Wasserstoff und Z für Brom steht, und R die in Tabelle 1 angegebenen Bedeutungen hat:

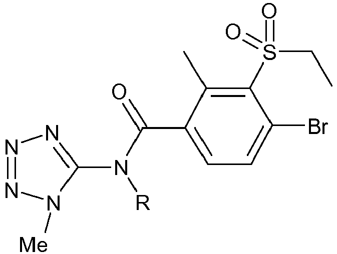

Tabelle 71: Erfindungsgemäße Verbindungen der Formel (I), worin Q für Q1, R⁶ für Methyl, X für Methyl, W für C-Y, Y für Methylsulfanyl, V für Wasserstoff und Z für Iod steht, und R die in Tabelle 1 angegebenen Bedeutungen hat:

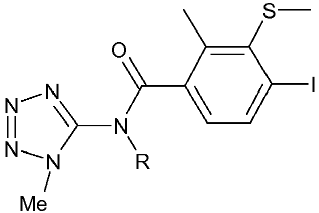

Tabelle 72: Erfindungsgemäße Verbindungen der Formel (I), worin Q für Q1, R⁶ für Methyl, X für Methyl, W für C-Y, Y für Methylsulfinyl, V für Wasserstoff und Z für Iod steht, und R die in Tabelle 1 angegebenen Bedeutungen hat:

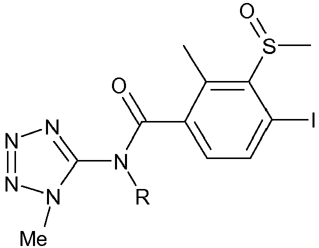

Tabelle 73: Erfindungsgemäße Verbindungen der Formel (I), worin Q für Q1, R⁶ für Methyl, X für Methyl, W für C-Y, Y für Methylsulfonyl, V für Wasserstoff und Z für Iod steht, und R die in Tabelle 1 angegebenen Bedeutungen hat:

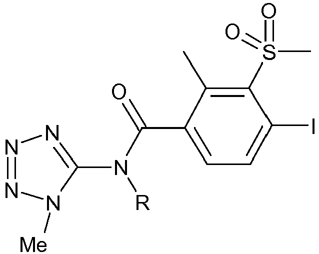

Tabelle 74: Erfindungsgemäße Verbindungen der Formel (I), worin Q für Q1, R⁶ für Methyl, X für Methyl, W für C-Y, Y für Ethylsulfanyl, V für Wasserstoff und Z für Iod steht, und R die in Tabelle 1 angegebenen Bedeutungen hat:

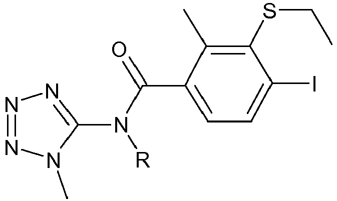

Tabelle 75: Erfindungsgemäße Verbindungen der Formel (I), worin Q für Q1, R⁶ für Ethyl, X für Methyl, W für C-Y, Y für Ethylsulfanyl, V für Wasserstoff und Z für Iod steht, und R die in Tabelle 1 angegebenen Bedeutungen hat:

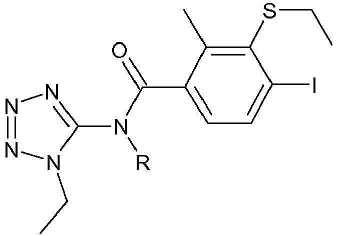

Tabelle 76: Erfindungsgemäße Verbindungen der Formel (I), worin Q für Q1, R⁶ für Methyl, X für Methyl, W für C-Y, Y für Methylsulfinyl, V für Wasserstoff und Z für Trifluormethyl steht, und R die in Tabelle 1 angegebenen Bedeutungen hat:

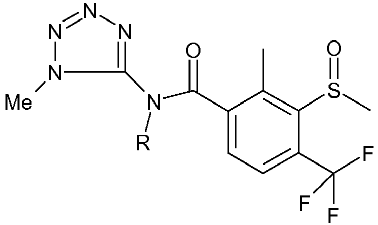

Tabelle 77: Erfindungsgemäße Verbindungen der Formel (I), worin Q für Q1, R⁶ für n-Propyl, X für Methyl, W für C-Y, Y für Methylsulfinyl, V für Wasserstoff und Z für Trifluormethyl steht, und R die in Tabelle 1 angegebenen Bedeutungen hat:

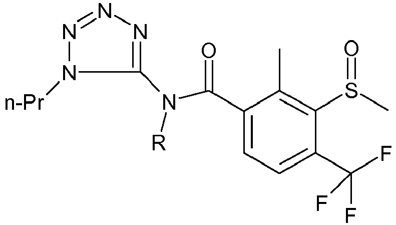

Tabelle 78: Erfindungsgemäße Verbindungen der Formel (I), worin Q für Q1, R⁶ für Allyl, X für Methyl, W für C-Y, Y für Methylsulfinyl, V für Wasserstoff und Z für Trifluormethyl steht, und R die in Tabelle 1 angegebenen Bedeutungen hat:

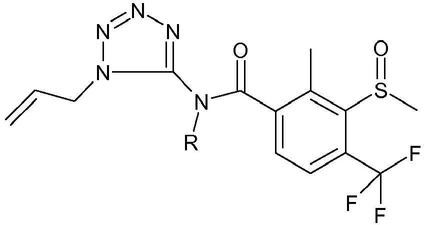

Tabelle 79: Erfindungsgemäße Verbindungen der Formel (I), worin Q für Q1, R⁶ für Ethyl; X für Methyl, W für C-Y, Y für Methylsulfinyl, V für Wasserstoff und Z für Chlor steht, und R die in Tabelle 1 angegebenen Bedeutungen hat:

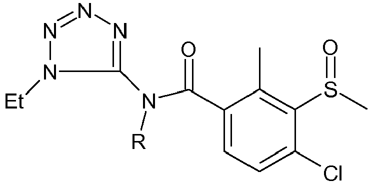

Tabelle 80: Erfindungsgemäße Verbindungen der Formel (I), worin Q für Q1, R⁶ für Methyl, X für Methyl, W für C-Y, Y für Methylsulfonyl, V für Wasserstoff und Z für Trifluormethyl steht, und R die in Tabelle 1 angegebenen Bedeutungen hat:

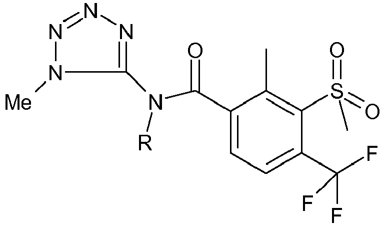

Tabelle 81: Erfindungsgemäße Verbindungen der Formel (I), worin Q für Q1, R⁶ für Ethyl, X für Methyl, W für C-Y, Y für Methylsulfonyl, V für Wasserstoff und Z für Trifluormethyl steht, und R die in Tabelle 1 angegebenen Bedeutungen hat:

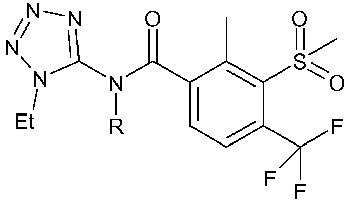

Tabelle 82: Erfindungsgemäße Verbindungen der Formel (I), worin Q für Q1, R⁶ für n-Propyl, X für Methyl, W für C-Y, Y für Methylsulfonyl, V für Wasserstoff und Z für Trifluormethyl steht, und R die in Tabelle 1 angegebenen Bedeutungen hat:

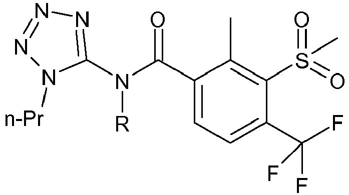

Tabelle 83: Erfindungsgemäße Verbindungen der Formel (I), worin Q für Q1, R⁶ für Methoxymethyl; X für Methyl, W für C-Y, Y für Methylsulfonyl, V für Wasserstoff und Z für Trifluormethyl steht, und R die in Tabelle 1 angegebenen Bedeutungen hat:

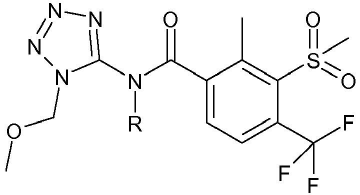

Tabelle 84: Erfindungsgemäße Verbindungen der Formel (I), worin Q für Q1, R⁶ für Allyl; X für Methyl, W für C-Y, Y für Methylsulfonyl, V für Wasserstoff und Z für Trifluormethyl steht, und R die in Tabelle 1 angegebenen Bedeutungen hat:

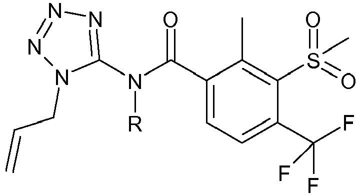

Tabelle 85: Erfindungsgemäße Verbindungen der Formel (I), worin Q für Q1, R⁶ für Propargyl, X für Methyl, W für C-Y, Y für Methylsulfonyl, V für Wasserstoff und Z für Trifluormethyl steht, und R die in Tabelle 1 angegebenen Bedeutungen hat:

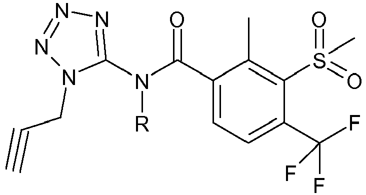

Tabelle 86: Erfindungsgemäße Verbindungen der Formel (I), worin Q für Q1, R⁶ für Ethyl, X für Methyl, W für C-Y, Y für Methylsulfonyl, V für Wasserstoff und Z für Chlor steht, und R die in Tabelle 1 angegebenen Bedeutungen hat:

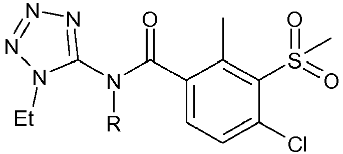

Tabelle 87: Erfindungsgemäße Verbindungen der Formel (I), worin Q für Q1, R⁶ für Methyl, X für Methyl, W für C-Y, Y für Methylsulfonyl, V für Wasserstoff und Z für Brom steht, und R die in Tabelle 1 angegebenen Bedeutungen hat:

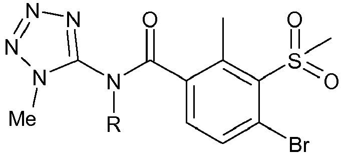

Tabelle 88: Erfindungsgemäße Verbindungen der Formel (I), worin Q für Q1, R⁶ für Methyl, X für Methyl, W für C-Y, Y für Ethylsulfanyl, V für Wasserstoff und Z für Brom steht, und R die in Tabelle 1 angegebenen Bedeutungen hat:

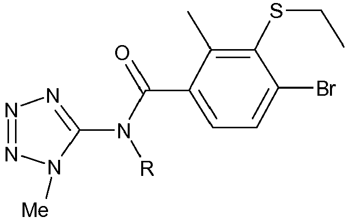

Tabelle 89: Erfindungsgemäße Verbindungen der Formel (I), worin Q für Q1, R⁶ für Methyl, X für Methyl, W für C-Y, Y für Ethylsulfanyl, V für Wasserstoff und Z für Trifluormethyl steht, und R die in Tabelle 1 angegebenen Bedeutungen hat:

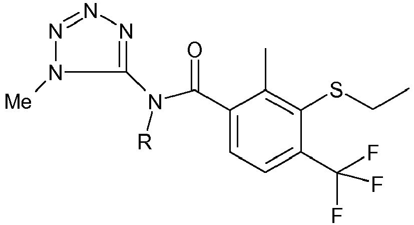

Tabelle 90: Erfindungsgemäße Verbindungen der Formel (I), worin Q für Q1, R⁶ für Methyl, X für Methyl, W für C-Y, Y für Ethylsulfanyl, V für Wasserstoff und Z für Chlor steht, und R die in Tabelle 1 angegebenen Bedeutungen hat:

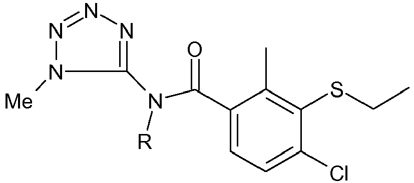

Tabelle 91: Erfindungsgemäße Verbindungen der Formel (I), worin Q für Q1, R⁶ für Ethyl, X für Methyl, W für C-Y, Y für Ethylsulfanyl, V für Wasserstoff und Z für Trifluormethyl steht, und R die in Tabelle 1 angegebenen Bedeutungen hat:

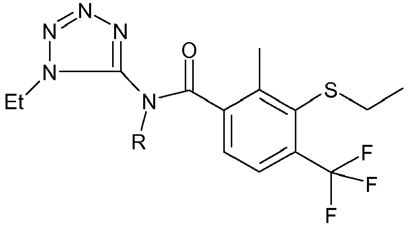

Tabelle 92: Erfindungsgemäße Verbindungen der Formel (I), worin Q für Q1, R⁶ für Ethyl, X für Methyl, W für C-Y, Y für Ethylsulfinyl, V für Wasserstoff und Z für Trifluormethyl steht, und R die in Tabelle 1 angegebenen Bedeutungen hat:

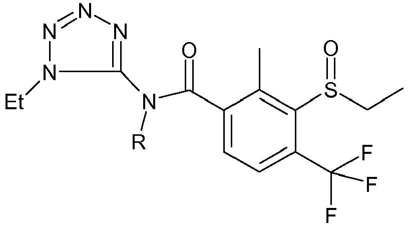

Tabelle 93: Erfindungsgemäße Verbindungen der Formel (I), worin Q für Q1, R⁶ für Methyl, X für Methyl, W für C-Y, Y für Ethylsulfinyl, V für Wasserstoff und Z für Chlor steht, und R die in Tabelle 1 angegebenen Bedeutungen hat:

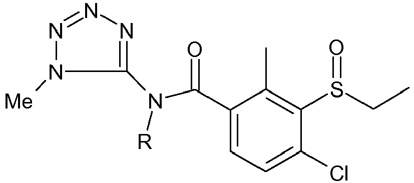

Tabelle 94: Erfindungsgemäße Verbindungen der Formel (I), worin Q für Q1, R⁶ für Ethyl, X für Methyl, W für C-Y, Y für Ethylsulfonyl, V für Wasserstoff und Z für Trifluormethyl steht, und R die in Tabelle 1 angegebenen Bedeutungen hat:

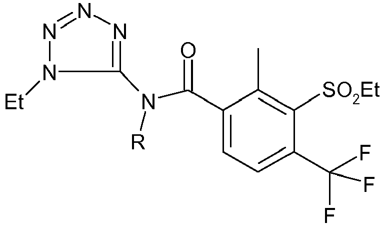

Tabelle 95: Erfindungsgemäße Verbindungen der Formel (I), worin Q für Q1, R⁶ für Methyl, X für Methyl, W für C-Y, Y für Ethylsulfonyl, V für Wasserstoff und Z für Chlor steht, und R die in Tabelle 1 angegebenen Bedeutungen hat:

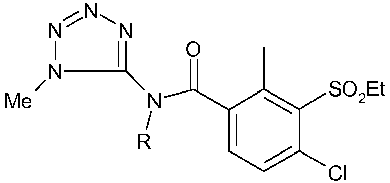

Tabelle 96: Erfindungsgemäße Verbindungen der Formel (I), worin Q für Q1, R⁶ für Ethyl, X für Methyl, W für C-Y, Y für Ethylsulfonyl, V für Wasserstoff und Z für Chlor steht, und R die in Tabelle 1 angegebenen Bedeutungen hat:

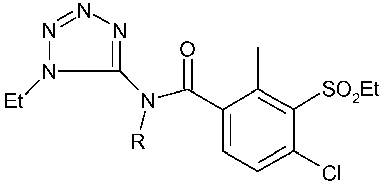

Tabelle 97: Erfindungsgemäße Verbindungen der Formel (I), worin Q für Q1, R⁶ für Methyl, X für Methyl, W für C-Y, Y für 2-Methoxyethylsulfonyl, V für Wasserstoff und Z für Methylsulfonyl steht, und R die in Tabelle 1 angegebenen Bedeutungen hat:

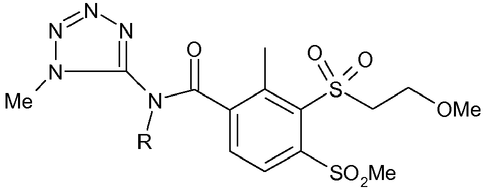

Tabelle 98: Erfindungsgemäße Verbindungen der Formel (I), worin Q für Q1, R⁶ für Methyl, X für Ethyl, W für C-Y, Y für Methylsulfanyl, V für Wasserstoff und Z für Trifluormethyl steht, und R die in Tabelle 1 angegebenen Bedeutungen hat:

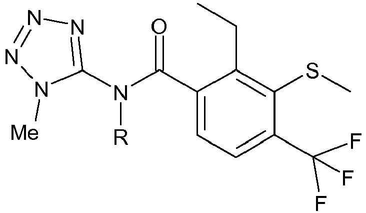

Tabelle 99: Erfindungsgemäße Verbindungen der Formel (I), worin Q für Q1, R⁶ für Methyl, X für n-Propyl, W für C-Y, Y für Methylsulfanyl, V für Wasserstoff und Z für Trifluormethyl steht, und R die in Tabelle 1 angegebenen Bedeutungen hat:

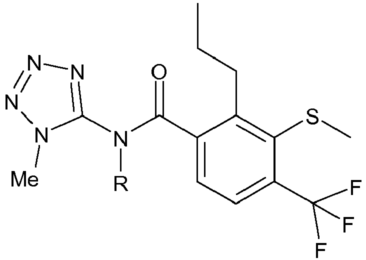

Tabelle 100: Erfindungsgemäße Verbindungen der Formel (I), worin Q für Q1, R⁶ für Ethyl, X für Ethyl, W für C-Y, Y für Methylsulfanyl, V für Wasserstoff und Z für Trifluormethyl steht, und R die in Tabelle 1 angegebenen Bedeutungen hat:

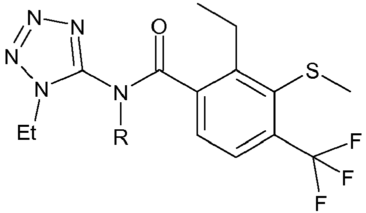

Tabelle 101: Erfindungsgemäße Verbindungen der Formel (I), worin Q für Q1, R⁶ für Methyl, X für Ethyl, W für C-Y, Y für Methylsulfanyl, V für Wasserstoff und Z für Chlor steht, und R die in Tabelle 1 angegebenen Bedeutungen hat:

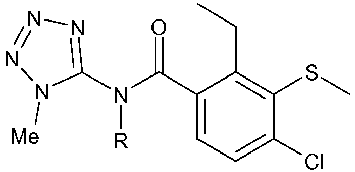

Tabelle 102: Erfindungsgemäße Verbindungen der Formel (I), worin Q für Q1, R⁶ für Ethyl, X für Ethyl, W für C-Y, Y für Methylsulfanyl, V für Wasserstoff und Z für Brom steht, und R die in Tabelle 1 angegebenen Bedeutungen hat:

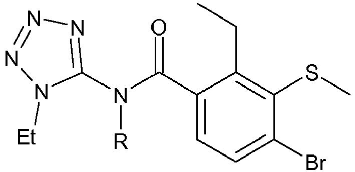

Tabelle 103: Erfindungsgemäße Verbindungen der Formel (I), worin Q für Q1, R⁶ für Methyl, X für Ethyl, W für C-Y, Y für Methylsulfinyl, V für Wasserstoff und Z für Trifluormethyl steht, und R die in Tabelle 1 angegebenen Bedeutungen hat:

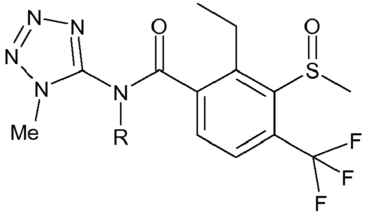

Tabelle 104: Erfindungsgemäße Verbindungen der Formel (I), worin Q für Q1, R⁶ für Ethyl, X für Ethyl, W für C-Y, Y für Methylsulfinyl, V für Wasserstoff und Z für Trifluormethyl steht, und R die in Tabelle 1 angegebenen Bedeutungen hat:

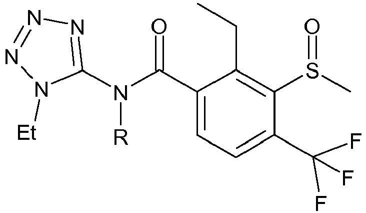

Tabelle 105: Erfindungsgemäße Verbindungen der Formel (I), worin Q für Q1, R⁶ für Methyl, X für n-Propyl; W für C-Y, Y für Methylsulfinyl, V für Wasserstoff und Z für Trifluormethyl steht, und R die in Tabelle 1 angegebenen Bedeutungen hat:

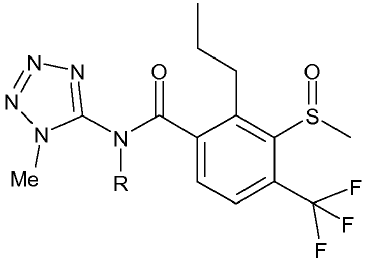

Tabelle 106: Erfindungsgemäße Verbindungen der Formel (I), worin Q für Q1, R⁶ für Methyl, X für Ethyl, W für C-Y, Y für Methylsulfinyl, V für Wasserstoff und Z für Chlor steht, und R die in Tabelle 1 angegebenen Bedeutungen hat:

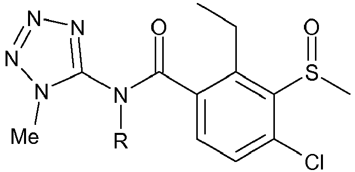

Tabelle 107: Erfindungsgemäße Verbindungen der Formel (I), worin Q für Q1, R⁶ für Ethyl, X für Ethyl, W für C-Y, Y für Methylsulfinyl, V für Wasserstoff und Z für Chlor steht, und R die in Tabelle 1 angegebenen Bedeutungen hat:

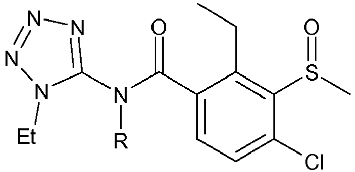

Tabelle 108: Erfindungsgemäße Verbindungen der Formel (I), worin Q für Q1, R⁶ für Methyl, X für Ethyl, W für C-Y, Y für Methylsulfinyl, V für Wasserstoff und Z für Brom steht, und R die in Tabelle 1 angegebenen Bedeutungen hat:

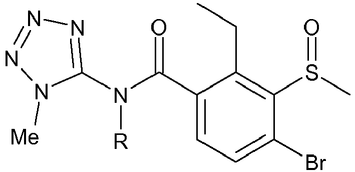

Tabelle 109: Erfindungsgemäße Verbindungen der Formel (I), worin Q für Q1, R⁶ für Methyl, X für Ethyl, W für C-Y, Y für Ethylsulfinyl, V für Wasserstoff und Z für Trifluormethyl steht, und R die in Tabelle 1 angegebenen Bedeutungen hat:

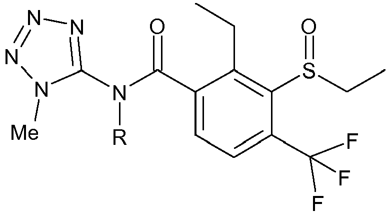

Tabelle 110: Erfindungsgemäße Verbindungen der Formel (I), worin Q für Q1, R⁶ für Methyl, X für Ethyl, W für C-Y, Y für Methylsulfanyl, V für Wasserstoff und Z für Brom steht, und R die in Tabelle 1 angegebenen Bedeutungen hat:

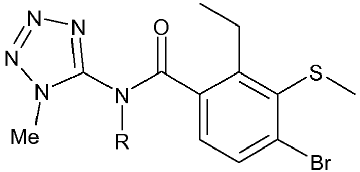

Tabelle 111: Erfindungsgemäße Verbindungen der Formel (I), worin Q für Q1, R⁶ für Methyl, X für Ethyl, W für C-Y, Y für Methylsulfonyl, V für Wasserstoff und Z für Trifluormethyl steht, und R die in Tabelle 1 angegebenen Bedeutungen hat:

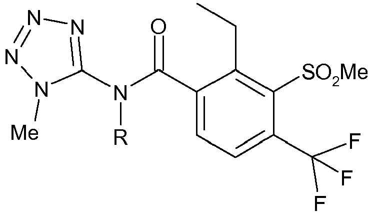

Tabelle 112: Erfindungsgemäße Verbindungen der Formel (I), worin Q für Q1, R⁶ für Ethyl, X für Ethyl, W für C-Y, Y für Methylsulfonyl, V für Wasserstoff und Z für Trifluormethyl steht, und R die in Tabelle 1 angegebenen Bedeutungen hat:

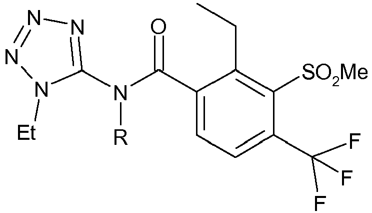

Tabelle 113: Erfindungsgemäße Verbindungen der Formel (I), worin Q für Q1, R⁶ für Methyl, X für Ethyl, W für C-Y, Y für Methylsulfonyl, V für Wasserstoff und Z für Chlor steht, und R die in Tabelle 1 angegebenen Bedeutungen hat:

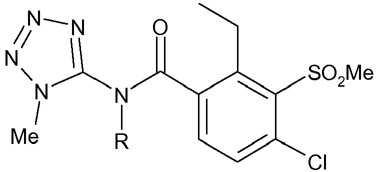

Tabelle 114: Erfindungsgemäße Verbindungen der Formel (I), worin Q für Q1, R⁶ für Methyl, X für Ethyl, W für C-Y, Y für Methylsulfonyl, V für Wasserstoff und Z für Brom steht, und R die in Tabelle 1 angegebenen Bedeutungen hat:

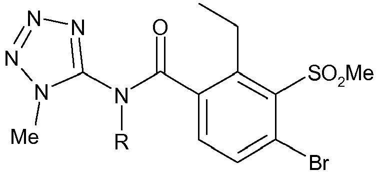

Tabelle 115: Erfindungsgemäße Verbindungen der Formel (I), worin Q für Q1, R⁶ für Methyl, X für n-Propyl; W für C-Y, Y für Methylsulfinyl, V für Wasserstoff und Z für Trifluormethyl steht, und R die in Tabelle 1 angegebenen Bedeutungen hat:

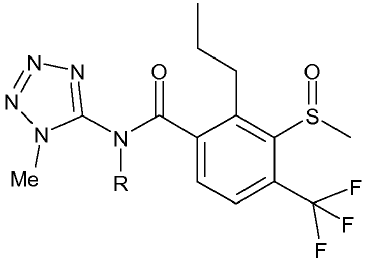

Tabelle 116: Erfindungsgemäße Verbindungen der Formel (I), worin Q für Q1, R⁶ für Methyl, X für Isopropyl; W für C-Y, Y für Methylsulfanyl, V für Wasserstoff und Z für Chlor steht, und R die in Tabelle 1 angegebenen Bedeutungen hat:

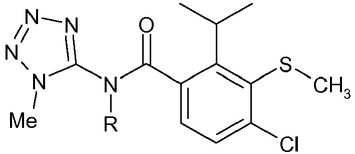

Tabelle 117: Erfindungsgemäße Verbindungen der Formel (I), worin Q für Q1, R⁶ für Methyl, X für Cyclopropyl; W für C-Y, Y für Methylsulfanyl, V für Wasserstoff und Z für Trifluormethyl steht, und R die in Tabelle 1 angegebenen Bedeutungen hat:

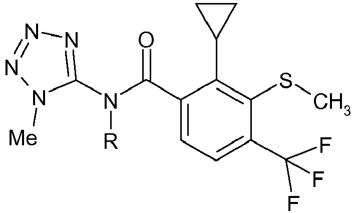

Tabelle 118: Erfindungsgemäße Verbindungen der Formel (I), worin Q für Q1, R⁶ für Methyl, X für Methoxy; W für C-Y, Y für Methylsulfanyl, V für Wasserstoff und Z für Trifluormethyl steht, und R die in Tabelle 1 angegebenen Bedeutungen hat:

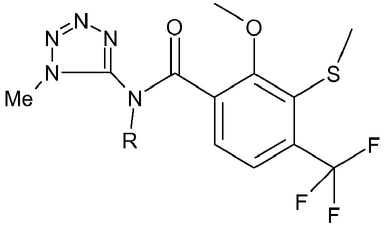

Tabelle 119: Erfindungsgemäße Verbindungen der Formel (I), worin Q für Q1, R⁶ für Methyl, X für Methoxy; W für C-Y, Y für Methylsulfinyl, V für Wasserstoff und Z für Trifluormethyl steht, und R die in Tabelle 1 angegebenen Bedeutungen hat:

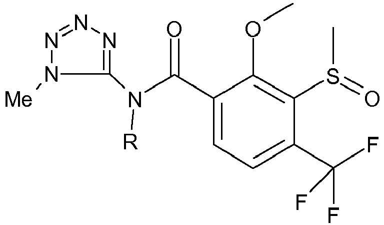

Tabelle 120: Erfindungsgemäße Verbindungen der Formel (I), worin Q für Q1, R⁶ für Methyl, X für Methoxy; W für C-Y, Y für Methylsulfonyl, V für Wasserstoff und Z für Trifluormethyl steht, und R die in Tabelle 1 angegebenen Bedeutungen hat:

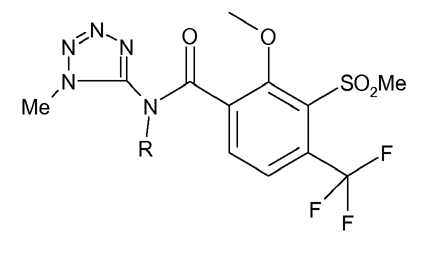

Tabelle 121: Erfindungsgemäße Verbindungen der Formel (I), worin Q für Q1, R⁶ für Methyl, X für Methoxy; W für C-Y, Y für Methylsulfanyl, V für Wasserstoff und Z für Methylsulfonyl steht, und R die in Tabelle 1 angegebenen Bedeutungen hat:

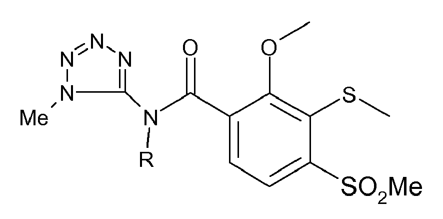

Tabelle 122: Erfindungsgemäße Verbindungen der Formel (I), worin Q für Q1, R⁶ für Methyl, X für Methoxy; W für C-Y, Y für Methylsulfinyl, V für Wasserstoff und Z für Methylsulfonyl steht, und R die in Tabelle 1 angegebenen Bedeutungen hat:

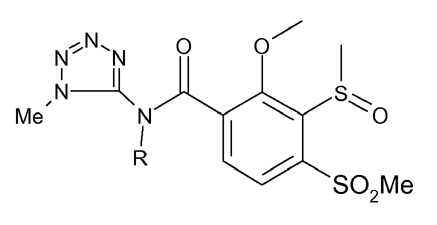

Tabelle 123: Erfindungsgemäße Verbindungen der Formel (I), worin Q für Q1, R⁶ für Methyl, X für Methoxy; W für C-Y, Y für Methylsulfonyl, V für Wasserstoff und Z für Methylsulfonyl steht, und R die in Tabelle 1 angegebenen Bedeutungen hat:

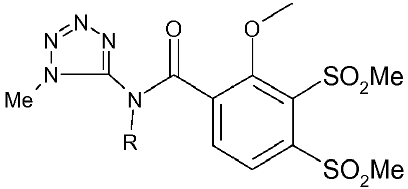

Tabelle 124: Erfindungsgemäße Verbindungen der Formel (I), worin Q für Q1, R⁶ für Methyl, X für Methoxy; W für C-Y, Y für Ethylsulfanyl, V für Wasserstoff und Z für Trifluormethyl steht, und R die in Tabelle 1 angegebenen Bedeutungen hat:

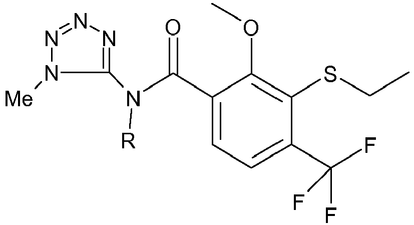

Tabelle 125: Erfindungsgemäße Verbindungen der Formel (I), worin Q für Q1, R⁶ für Ethyl, X für Ethoxy; W für C-Y, Y für Methylsulfanyl, V für Wasserstoff und Z für Chlor steht, und R die in Tabelle 1 angegebenen Bedeutungen hat:

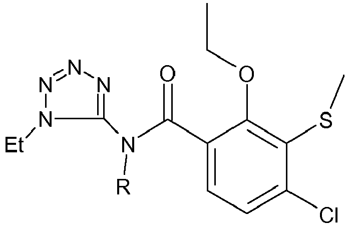

Tabelle 126: Erfindungsgemäße Verbindungen der Formel (I), worin Q für Q1, R⁶ für Methyl, X für Methoxymethyl; W für C-Y, Y für Methylsulfanyl, V für Wasserstoff und Z für Methylsulfonyl steht, und R die in Tabelle 1 angegebenen Bedeutungen hat:

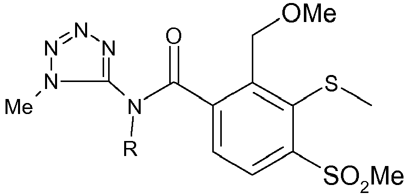

Tabelle 127: Erfindungsgemäße Verbindungen der Formel (I), worin Q für Q1, R⁶ für Methyl, X für Methoxymethyl; W für C-Y, Y für Methylsulfinyl, V für Wasserstoff und Z für Methylsulfonyl steht, und R die in Tabelle 1 angegebenen Bedeutungen hat:

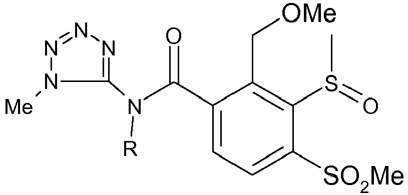

Tabelle 128: Erfindungsgemäße Verbindungen der Formel (I), worin Q für Q1, R⁶ für Methyl, X für Methoxymethyl; W für C-Y, Y für Methylsulfonyl, V für Wasserstoff und Z für Methylsulfonyl steht, und R die in Tabelle 1 angegebenen Bedeutungen hat:

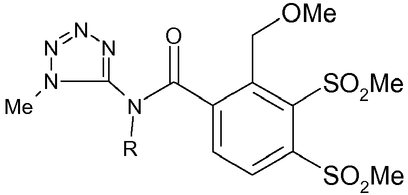

Tabelle 129: Erfindungsgemäße Verbindungen der Formel (I), worin Q für Q1, R⁶ für Methyl, X für Chlor; W für C-Y, Y für Wasserstoff, V für Methyl und Z für Methylsulfanyl steht, und R die in Tabelle 1 angegebenen Bedeutungen hat: 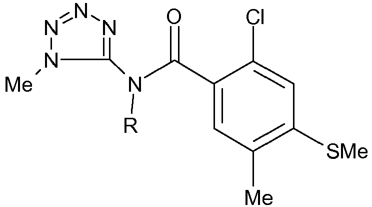 Tabelle 130: Erfindungsgemäße Verbindungen der Formel (I), worin Q für Q1, R⁶ für Methyl, X für Chlor; W für C-Y, Y für Methylsulfinyl, V für Methyl und Z für Wasserstoff steht, und R die in Tabelle 1 angegebenen Bedeutungen hat:

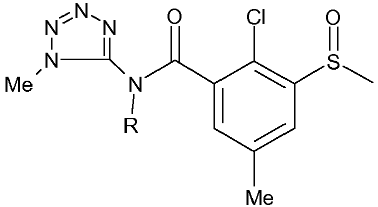

Tabelle 131: Erfindungsgemäße Verbindungen der Formel (I), worin Q für Q1, R⁶ für Methyl, X für Chlor; W für C-Y, Y für Methylsulfonyl, V für Methyl und Z für Wasserstoff steht, und R die in Tabelle 1 angegebenen Bedeutungen hat:

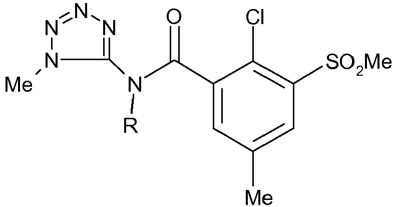

Tabelle 132: Erfindungsgemäße Verbindungen der Formel (I), worin Q für Q1, R⁶ für Methyl, X für Chlor; W für C-Y, Y für Methyl; V für Methyl und Z für Methylsulfanyl steht, und R die in Tabelle 1 angegebenen Bedeutungen hat:

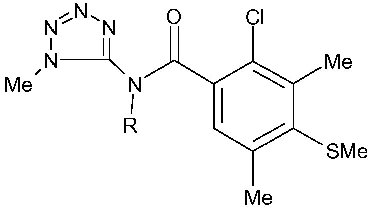

Tabelle 133: Erfindungsgemäße Verbindungen der Formel (I), worin Q für Q1, R⁶ für Methyl, X für Chlor; W für C-Y, Y für Wasserstoff; V für Wasserstoff und Z für Chlor steht, und R die in Tabelle 1 angegebenen Bedeutungen hat:

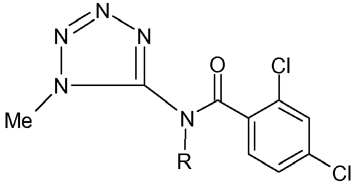

Tabelle 134: Erfindungsgemäße Verbindungen der Formel (I), worin Q für Q1, R⁶ für Methyl, X für Chlor; W für C-Y, Y für Wasserstoff; V für Wasserstoff und Z für Methylsulfanyl steht, und R die in Tabelle 1 angegebenen Bedeutungen hat:

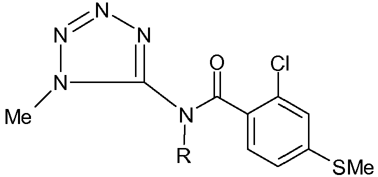

Tabelle 135: Erfindungsgemäße Verbindungen der Formel (I), worin Q für Q1, R⁶ für Methyl, X für Chlor; W für C-Y, Y für Wasserstoff; V für Wasserstoff und Z für Methylsulfonyl steht, und R die in Tabelle 1 angegebenen Bedeutungen hat:

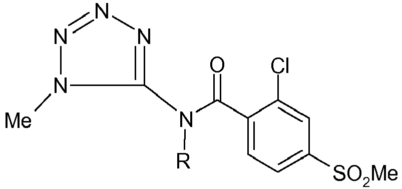

Tabelle 136: Erfindungsgemäße Verbindungen der Formel (I), worin Q für Q1, R⁶ für Methyl, X für Chlor; W für C-Y, Y für Wasserstoff; V für Wasserstoff und Z für Trifluormethyl steht, und R die in Tabelle 1 angegebenen Bedeutungen hat:

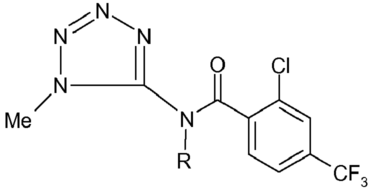

Tabelle 137: Erfindungsgemäße Verbindungen der Formel (I), worin Q für Q1, R⁶ für Methyl, X für Brom; W für C-Y, Y für Wasserstoff; V für Wasserstoff und Z für Methylsulfanyl steht, und R die in Tabelle 1 angegebenen Bedeutungen hat:

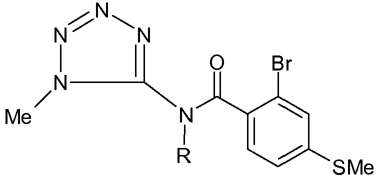

Tabelle 138: Erfindungsgemäße Verbindungen der Formel (I), worin Q für Q1, R⁶ für Methyl, X für Methylsulfonyl; W für C-Y, Y für Wasserstoff; V für Wasserstoff und Z für Trifluormethyl steht, und R die in Tabelle 1 angegebenen Bedeutungen hat:

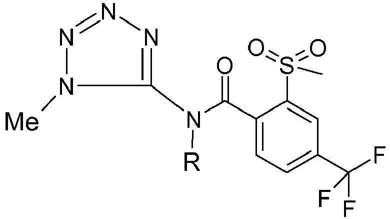

Tabelle 139: Erfindungsgemäße Verbindungen der Formel (I), worin Q für Q1, R⁶ für Methyl, X für Brom; W für C-Y, Y für Wasserstoff; V für Wasserstoff und Z für Trifluormethyl steht, und R die in Tabelle 1 angegebenen Bedeutungen hat:

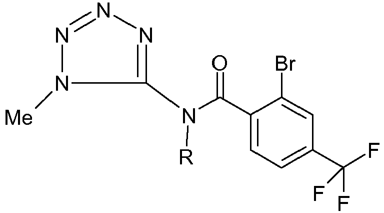

Tabelle 140: Erfindungsgemäße Verbindungen der Formel (I), worin Q für Q1, R⁶ für Methyl, X für Brom; W für C-Y, Y für Wasserstoff; V für Wasserstoff und Z für Methylsulfonyl steht, und R die in Tabelle 1 angegebenen Bedeutungen hat:

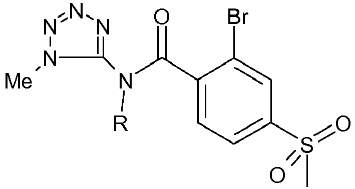

Tabelle 141: Erfindungsgemäße Verbindungen der Formel (I), worin Q für Q1, R⁶ für Methyl, X für (Methylsulfanyl)methyl; W für C-Y, Y für Wasserstoff; V für Wasserstoff und Z für Trifluormethyl steht, und R die in Tabelle 1 angegebenen Bedeutungen hat:

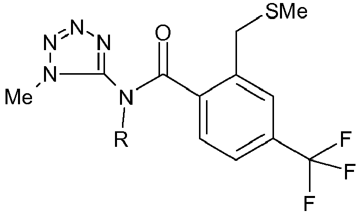

Tabelle 142: Erfindungsgemäße Verbindungen der Formel (I), worin Q für Q1, R⁶ für Methyl, X für Chlor; W für N,V für Wasserstoff und Z für Trifluormethyl steht, und R die in Tabelle 1 angegebenen Bedeutungen hat:

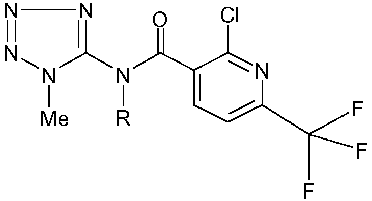

Tabelle 143: Erfindungsgemäße Verbindungen der Formel (I), worin Q für Q1, R⁶ für Methyl, X für Brom; W für N,V für Wasserstoff und Z für Trifluormethyl steht, und R die in Tabelle 1 angegebenen Bedeutungen hat:

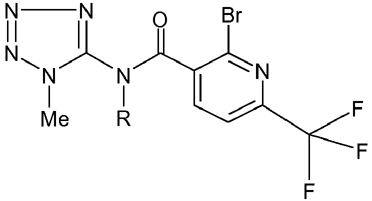

Tabelle 144: Erfindungsgemäße Verbindungen der Formel (I), worin Q für Q1, R⁶ für Methyl, X für Methyl; W für N;V für Wasserstoff und Z für Trifluormethyl steht, und R die in Tabelle 1 angegebenen Bedeutungen hat:

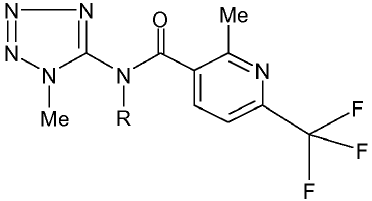

Tabelle 145: Erfindungsgemäße Verbindungen der Formel (I), worin Q für Q1, R⁶ für Methyl, X für Methylsulfonyl; W für N,;V für Wasserstoff und Z für Trifluormethyl steht, und R die in Tabelle 1 angegebenen Bedeutungen hat:

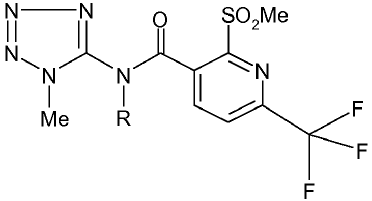

Tabelle 146: Erfindungsgemäße Verbindungen der Formel (I), worin Q für Q1, R⁶ für Methyl, X für Methoxymethyl; W für N,V für Wasserstoff und Z für Trifluormethyl steht, und R die in Tabelle 1 angegebenen Bedeutungen hat:

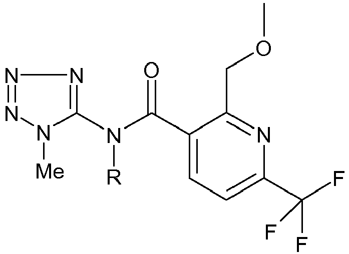

Tabelle 147: Erfindungsgemäße Verbindungen der Formel (I), worin Q für Q1, R⁶ für Ethyl, X für Methoxymethyl; W für N,V für Wasserstoff und Z für Trifluormethyl steht, und R die in Tabelle 1 angegebenen Bedeutungen hat:

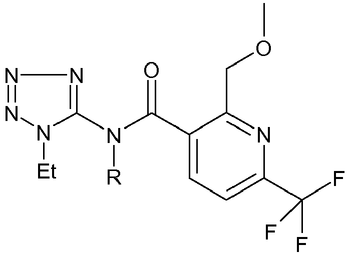

Tabelle 148: Erfindungsgemäße Verbindungen der Formel (I), worin Q für Q1, R⁶ für Ethyl, X für (2-Methoxyethyl)methyl; W für N,V für Wasserstoff und Z für Trifluormethyl steht, und R die in Tabelle 1 angegebenen Bedeutungen hat:

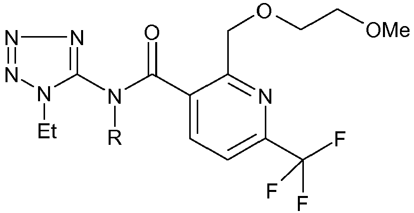

Tabelle 149: Erfindungsgemäße Verbindungen der Formel (I), worin Q für Q1, R⁶ für Methyl, X für (3-Methoxypropyloxy)methyl; W für N,V für Wasserstoff und Z für Trifluormethyl steht, und R die in Tabelle 1 angegebenen Bedeutungen hat:

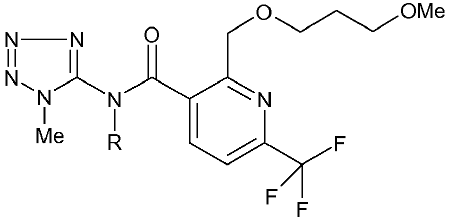

Tabelle 150: Erfindungsgemäße Verbindungen der Formel (I), worin Q für Q1, R⁶ für Methyl, X für (Cyclopropylmethoxy)methyl; W für N,V für Wasserstoff und Z für Trifluormethyl steht, und R die in Tabelle 1 angegebenen Bedeutungen hat:

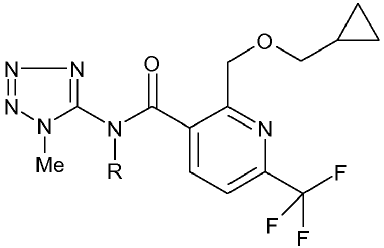

Tabelle 151: Erfindungsgemäße Verbindungen der Formel (I), worin Q für Q1, R⁶ für Methyl, X für (Methylsulfanyl)methyl; W für N,V für Wasserstoff und Z für Trifluormethyl steht, und R die in Tabelle 1 angegebenen Bedeutungen hat: 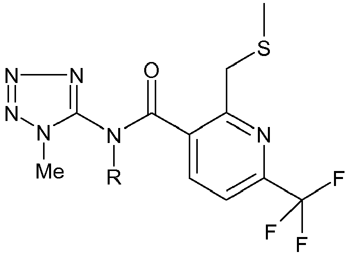 Q = Q2 Triazole

Tabelle 152: Erfindungsgemäße Verbindungen der Formel (I), worin Q für Q2, R⁷ für Methyl, X für Nitro; W für C-Y, Y für Wasserstoff, V für Wasserstoff und Z für Trifluormethyl steht, und R die in Tabelle 1 angegebenen Bedeutungen hat:

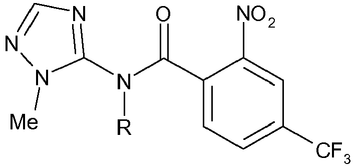

Tabelle 153: Erfindungsgemäße Verbindungen der Formel (I), worin Q für Q2, R⁷ für Methyl, X für Brom; W für C-Y, Y für Wasserstoff, V für Wasserstoff und Z für Trifluormethyl steht, und R die in Tabelle 1 angegebenen Bedeutungen hat:

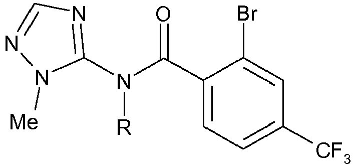

Tabelle 154: Erfindungsgemäße Verbindungen der Formel (I), worin Q für Q2, R⁷ für Methyl, X für Methylsulfonyl; W für C-Y, Y für Wasserstoff, V für Wasserstoff und Z für Trifluormethyl steht, und R die in Tabelle 1 angegebenen Bedeutungen hat:

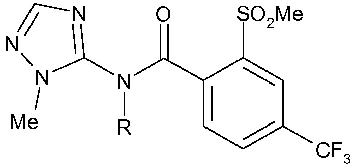

Tabelle 155: Erfindungsgemäße Verbindungen der Formel (I), worin Q für Q2, R⁷ für Methyl, X für Chlor; W für N, V für Wasserstoff und Z für Trifluormethyl steht, und R die in Tabelle 1 angegebenen Bedeutungen hat:

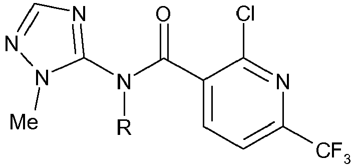

Tabelle 156: Erfindungsgemäße Verbindungen der Formel (I), worin Q für Q2, R⁷ für Methyl, X für Methoxymethyl; W für N, V für Wasserstoff und Z für Trifluormethyl steht, und R die in Tabelle 1 angegebenen Bedeutungen hat:

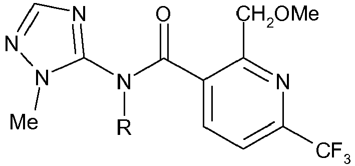

Tabelle 157: Erfindungsgemäße Verbindungen der Formel (I), worin Q für Q2, R⁷ für Methyl, X für (2-Methoxyethoxy)methyl; W für N, V für Wasserstoff und Z für Trifluormethyl steht, und R die in Tabelle 1 angegebenen Bedeutungen hat:

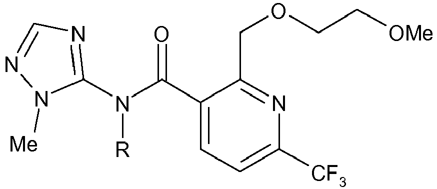

Tabelle 158: Erfindungsgemäße Verbindungen der Formel (I), worin Q für Q2, R⁷ für Methyl, X für Chlor; W für C-Y, Y für (2,2,2-Trifluorethoxy)methyl, V für Wasserstoff und Z für Methylsulfonyl steht, und R die in Tabelle 1 angegebenen Bedeutungen hat:

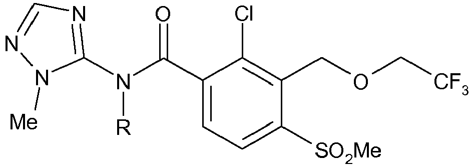

Tabelle 159: Erfindungsgemäße Verbindungen der Formel (I), worin Q für Q2, R⁷ für Methyl, X für Chlor; W für C-Y, Y für 5-Cyanmethyl-4,5-dihydro-1,2-oxazol-3-yl, V für Wasserstoff und Z für Ethylsulfonyl steht, und R die in Tabelle 1 angegebenen Bedeutungen hat:

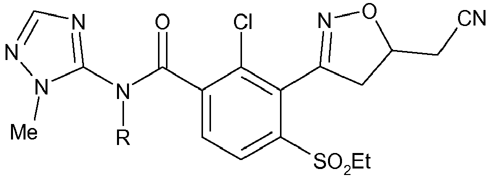

Tabelle 160: Erfindungsgemäße Verbindungen der Formel (I), worin Q für Q2, R⁷ für Methyl, X für Chlor; W für C-Y, Y für 4,5-dihydro-1,2-oxazol-3-yl, V für Wasserstoff und Z für Methylsulfonyl steht, und R die in Tabelle 1 angegebenen Bedeutungen hat:

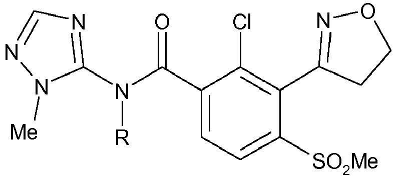

Tabelle 161: Erfindungsgemäße Verbindungen der Formel (I), worin Q für Q2, R⁷ für Methyl, X für Chlor; W für C-Y, Y für 5-(Methoxymethyl)-4,5-dihydro-1,2-oxazol-3-yl, V für Wasserstoff und Z für Ethylsulfonyl steht, und R die in Tabelle 1 angegebenen Bedeutungen hat:

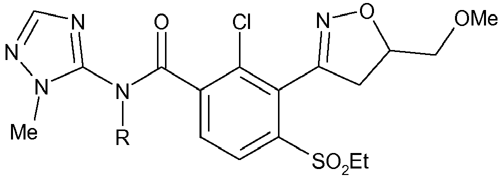

Tabelle 162: Erfindungsgemäße Verbindungen der Formel (I), worin Q für Q2, R⁷ für Methyl, X für Chlor; W für C-Y, Y für Methylsulfanyl, V für Wasserstoff und Z für Methyl steht, und R die in Tabelle 1 angegebenen Bedeutungen hat:

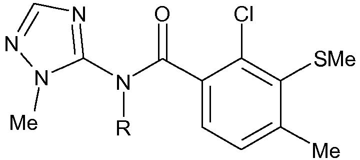

Tabelle 163: Erfindungsgemäße Verbindungen der Formel (I), worin Q für Q2, R⁷ für Methyl, X für Chlor; W für C-Y, Y für Methylsulfanyl, V für Wasserstoff und Z für Chlor steht, und R die in Tabelle 1 angegebenen Bedeutungen hat:

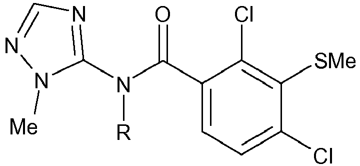

Tabelle 164: Erfindungsgemäße Verbindungen der Formel (I), worin Q für Q2, R⁷ für Methyl, X für Chlor; W für C-Y, Y für Methylsulfinyl, V für Wasserstoff und Z für Chlor steht, und R die in Tabelle 1 angegebenen Bedeutungen hat:

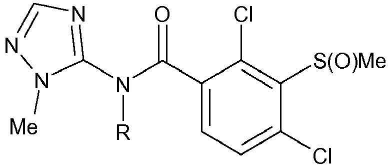

Tabelle 165: Erfindungsgemäße Verbindungen der Formel (I), worin Q für Q2, R⁷ für Methyl, X für Chlor; W für C-Y, Y für Methylsulfonyl, V für Wasserstoff und Z für Chlor steht, und R die in Tabelle 1 angegebenen Bedeutungen hat:

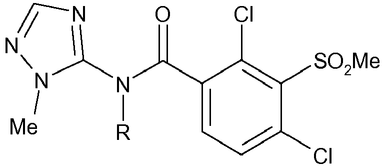

Tabelle 166: Erfindungsgemäße Verbindungen der Formel (I), worin Q für Q2, R⁷ für Methyl, X für Chlor; W für C-Y, Y für Methylsulfanyl, V für Wasserstoff und Z für Methylsulfonyl steht, und R die in Tabelle 1 angegebenen Bedeutungen hat:

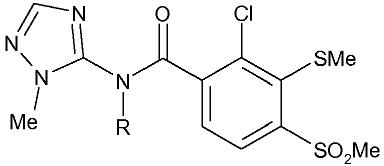

Tabelle 167: Erfindungsgemäße Verbindungen der Formel (I), worin Q für Q2, R⁷ für Methyl, X für Chlor; W für C-Y, Y für Methylsulfinyl, V für Wasserstoff und Z für Methylsulfonyl steht, und R die in Tabelle 1 angegebenen Bedeutungen hat:

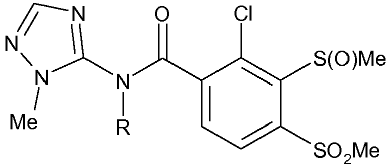

Tabelle 168: Erfindungsgemäße Verbindungen der Formel (I), worin Q für Q2, R⁷ für Methyl, X für Chlor; W für C-Y, Y für Methylsulfonyl, V für Wasserstoff und Z für Methylsulfonyl steht, und R die in Tabelle 1 angegebenen Bedeutungen hat:

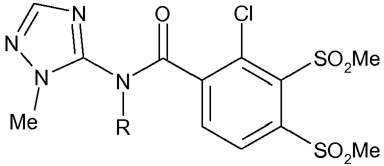

Tabelle 169: Erfindungsgemäße Verbindungen der Formel (I), worin Q für Q2, R⁷ für Methyl, X für Chlor; W für C-Y, Y für Methylsulfanyl, V für Wasserstoff und Z für Trifluormethyl steht, und R die in Tabelle 1 angegebenen Bedeutungen hat:

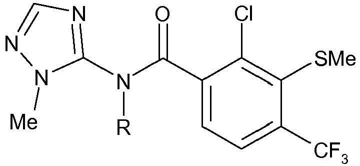

Tabelle 170: Erfindungsgemäße Verbindungen der Formel (I), worin Q für Q2, R⁷ für Methyl, X für Chlor; W für C-Y, Y für Methylsulfinyl, V für Wasserstoff und Z für Trifluormethyl steht, und R die in Tabelle 1 angegebenen Bedeutungen hat:

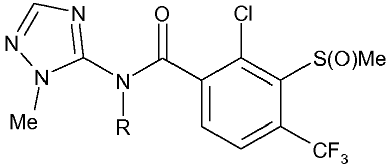

Tabelle 171: Erfindungsgemäße Verbindungen der Formel (I), worin Q für Q2, R⁷ für Methyl, X für Chlor; W für C-Y, Y für Methylsulfonyl, V für Wasserstoff und Z für Trifluormethyl steht, und R die in Tabelle 1 angegebenen Bedeutungen hat:

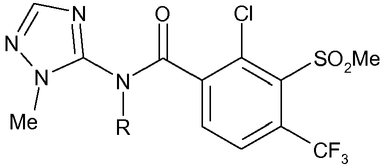

Tabelle 172: Erfindungsgemäße Verbindungen der Formel (I), worin Q für Q2, R⁷ für Methyl, X für Chlor; W für C-Y, Y für 2-Methoxy, V für Wasserstoff und Z für Methylsulfonyl steht, und R die in Tabelle 1 angegebenen Bedeutungen hat:

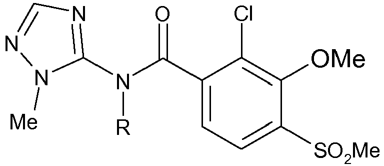

Tabelle 173: Erfindungsgemäße Verbindungen der Formel (I), worin Q für Q2, R⁷ für Methyl, X für Chlor; W für C-Y, Y für Propoxy, V für Wasserstoff und Z für Methylsulfonyl steht, und R die in Tabelle 1 angegebenen Bedeutungen hat:

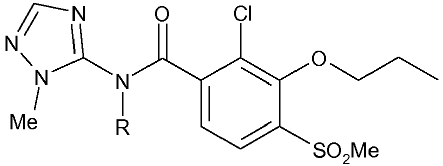

Tabelle 174: Erfindungsgemäße Verbindungen der Formel (I), worin Q für Q2, R⁷ für Methyl, X für Chlor; W für C-Y, Y für Cyclopropylmethoxy, V für Wasserstoff und Z für Methylsulfonyl steht, und R die in Tabelle 1 angegebenen Bedeutungen hat:

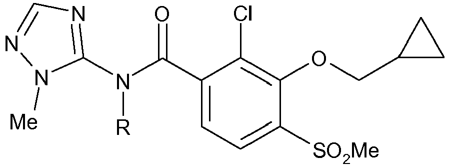

Tabelle 175: Erfindungsgemäße Verbindungen der Formel (I), worin Q für Q2, R⁷ für Methyl, X für Chlor; W für C-Y, Y für 2-Methoxyethoxy, V für Wasserstoff und Z für Methylsulfonyl steht, und R die in Tabelle 1 angegebenen Bedeutungen hat:

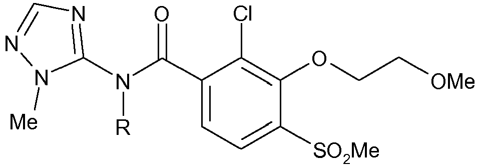

Tabelle 176: Erfindungsgemäße Verbindungen der Formel (I), worin Q für Q2, R⁷ für Methyl, X für Chlor; W für C-Y, Y für Methoxymethyl, V für Wasserstoff und Z für Methylsulfonyl steht, und R die in Tabelle 1 angegebenen Bedeutungen hat:

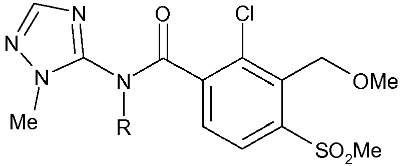

Tabelle 177: Erfindungsgemäße Verbindungen der Formel (I), worin Q für Q2, R⁷ für Methyl, X für Chlor; W für C-Y, Y für (2-Methoxyethoxy)methyl, V für Wasserstoff und Z für Methylsulfonyl steht, und R die in Tabelle 1 angegebenen Bedeutungen hat:

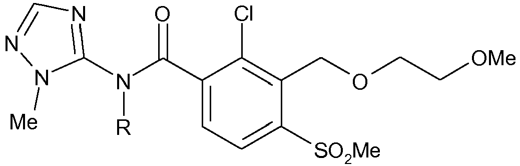

Tabelle 178: Erfindungsgemäße Verbindungen der Formel (I), worin Q für Q2, R⁷ für Methyl, X für Chlor; W für C-Y, Y für Chlor, V für Wasserstoff und Z für Methylsulfonyl steht, und R die in Tabelle 1 angegebenen Bedeutungen hat:

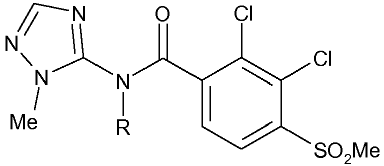

Tabelle 179: Erfindungsgemäße Verbindungen der Formel (I), worin Q für Q2, R⁷ für Methyl, X für Chlor; W für C-Y, Y für (Tetrahydrofuran-2-ylmethoxy)methyl, V für Wasserstoff und Z für Methylsulfonyl steht, und R die in Tabelle 1 angegebenen Bedeutungen hat:

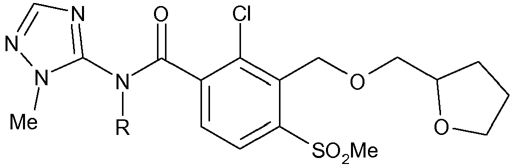

Tabelle 180: Erfindungsgemäße Verbindungen der Formel (I), worin Q für Q2, R⁷ für Methyl, X für Chlor; W für C-Y, Y für 2-Fluorethoxy, V für Wasserstoff und Z für Methylsulfonyl steht, und R die in Tabelle 1 angegebenen Bedeutungen hat:

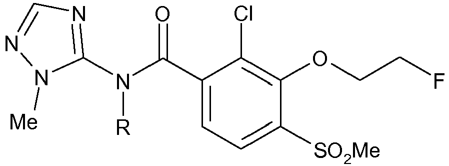

Tabelle 181: Erfindungsgemäße Verbindungen der Formel (I), worin Q für Q2, R⁷ für Methyl, X für Chlor; W für C-Y, Y für (3-Methoxy)propoxy, V für Wasserstoff und Z für Ethylsulfonyl steht, und R die in Tabelle 1 angegebenen Bedeutungen hat:

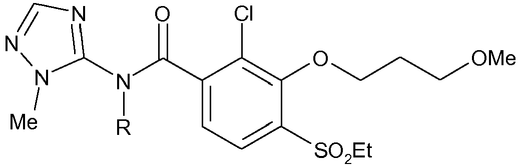

Tabelle 182: Erfindungsgemäße Verbindungen der Formel (I), worin Q für Q2, R⁷ für Methyl, X für Brom; W für C-Y, Y für Methylsulfanyl, V für Wasserstoff und Z für Methyl steht, und R die in Tabelle 1 angegebenen Bedeutungen hat:

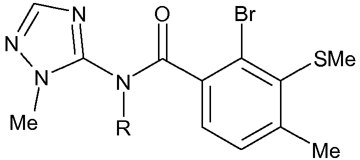

Tabelle 183: Erfindungsgemäße Verbindungen der Formel (I), worin Q für Q2, R⁷ für Methyl, X für Brom; W für C-Y, Y für Methylsulfinyl, V für Wasserstoff und Z für Methyl steht, und R die in Tabelle 1 angegebenen Bedeutungen hat:

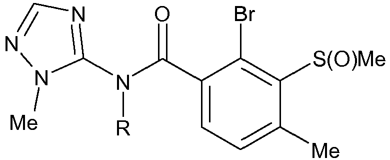

Tabelle 184: Erfindungsgemäße Verbindungen der Formel (I), worin Q für Q2, R⁷ für Methyl, X für Brom; W für C-Y, Y für Methylsulfonyl, V für Wasserstoff und Z für Methyl steht, und R die in Tabelle 1 angegebenen Bedeutungen hat:

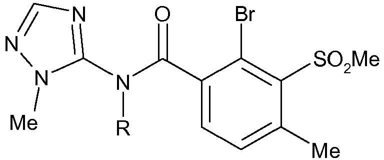

Tabelle 185: Erfindungsgemäße Verbindungen der Formel (I), worin Q für Q2, R⁷ für Methyl, X für Methyl; W für C-Y, Y für 4,5-dihydro-1,2-oxazol-3-yl, V für Wasserstoff und Z für Methylsulfonyl steht, und R die in Tabelle 1 angegebenen Bedeutungen hat:

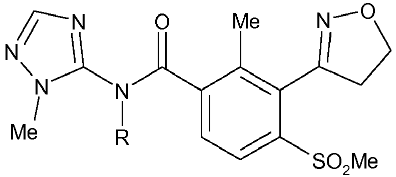

Tabelle 186: Erfindungsgemäße Verbindungen der Formel (I), worin Q für Q2, R⁷ für Methyl, X für Methyl; W für C-Y, Y für 1 H-Pyrazol-1-yl, V für Wasserstoff und Z für Trifluormethyl steht, und R die in Tabelle 1 angegebenen Bedeutungen hat:

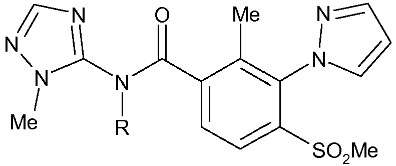

Tabelle 187: Erfindungsgemäße Verbindungen der Formel (I), worin Q für Q2, R⁷ für Methyl, X für Methyl; W für C-Y, Y für Methylsulfanyl, V für Wasserstoff und Z für Trifluormethyl steht, und R die in Tabelle 1 angegebenen Bedeutungen hat:

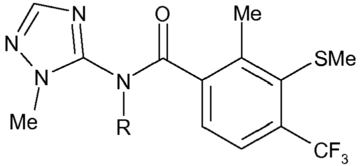

Tabelle 188: Erfindungsgemäße Verbindungen der Formel (I), worin Q für Q2, R⁷ für Methyl, X für Methyl; W für C-Y, Y für Methylsulfinyl, V für Wasserstoff und Z für Trifluormethyl steht, und R die in Tabelle 1 angegebenen Bedeutungen hat:

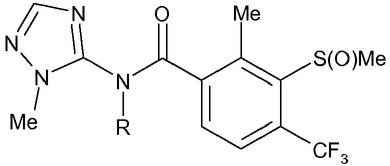

Tabelle 189: Erfindungsgemäße Verbindungen der Formel (I), worin Q für Q2, R⁷ für Methyl, X für Methyl; W für C-Y, Y für Methylsulfonyl, V für Wasserstoff und Z für Trifluormethyl steht, und R die in Tabelle 1 angegebenen Bedeutungen hat:

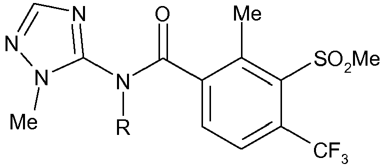

Tabelle 190: Erfindungsgemäße Verbindungen der Formel (I), worin Q für Q2, R⁷ für Methyl, X für Methyl; W für C-Y, Y für Methylsulfanyl, V für Wasserstoff und Z für Methylsulfonyl steht, und R die in Tabelle 1 angegebenen Bedeutungen hat:

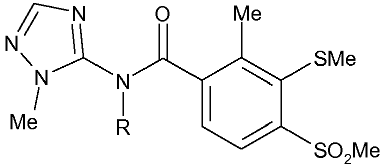

Tabelle 191: Erfindungsgemäße Verbindungen der Formel (I), worin Q für Q2, R⁷ für Methyl, X für Methyl; W für C-Y, Y für Methylsulfinyl, V für Wasserstoff und Z für Methylsulfonyl steht, und R die in Tabelle 1 angegebenen Bedeutungen hat:

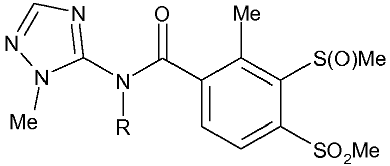

Tabelle 192: Erfindungsgemäße Verbindungen der Formel (I), worin Q für Q2, R⁷ für Methyl, X für Methyl; W für C-Y, Y für Methylsulfonyl, V für Wasserstoff und Z für Methylsulfonyl steht, und R die in Tabelle 1 angegebenen Bedeutungen hat:

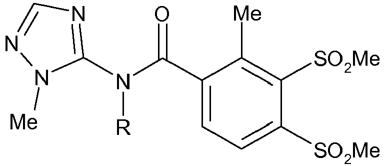

Tabelle 193: Erfindungsgemäße Verbindungen der Formel (I), worin Q für Q2, R⁷ für Methyl, X für Methyl; W für C-Y, Y für Ethylsulfonyl, V für Wasserstoff und Z für Methylsulfonyl steht, und R die in Tabelle 1 angegebenen Bedeutungen hat:

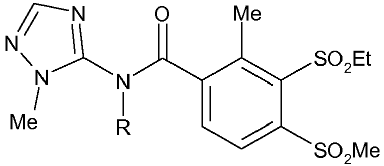

Tabelle 194: Erfindungsgemäße Verbindungen der Formel (I), worin Q für Q2, R⁷ für Methyl, X für Methoxy; W für C-Y, Y für Methylsulfanyl, V für Wasserstoff und Z für Methylsulfonyl steht, und R die in Tabelle 1 angegebenen Bedeutungen hat:

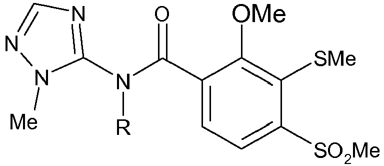

Tabelle 195: Erfindungsgemäße Verbindungen der Formel (I), worin Q für Q2, R⁷ für Methyl, X für Methoxy; W für C-Y, Y für Methylsulfinyl, V für Wasserstoff und Z für Methylsulfonyl steht, und R die in Tabelle 1 angegebenen Bedeutungen hat:

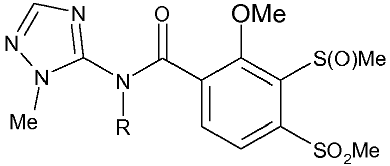

Tabelle 196: Erfindungsgemäße Verbindungen der Formel (I), worin Q für Q2, R⁷ für Methyl, X für Methoxy; W für C-Y, Y für Methylsulfonyl, V für Wasserstoff und Z für Methylsulfonyl steht, und R die in Tabelle 1 angegebenen Bedeutungen hat:

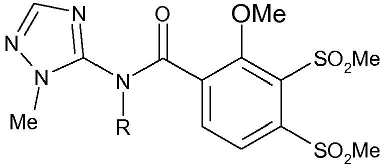

Tabelle 197: Erfindungsgemäße Verbindungen der Formel (I), worin Q für Q2, R⁷ für Methyl, X für Methoxy; W für C-Y, Y für Methylsulfanyl, V für Wasserstoff und Z für Trifluormethyl steht, und R die in Tabelle 1 angegebenen Bedeutungen hat:

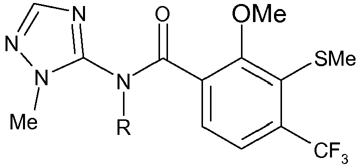

Tabelle 198: Erfindungsgemäße Verbindungen der Formel (I), worin Q für Q2, R⁷ für Methyl, X für Methoxy; W für C-Y, Y für Methylsulfinyl, V für Wasserstoff und Z für Trifluormethyl steht, und R die in Tabelle 1 angegebenen Bedeutungen hat:

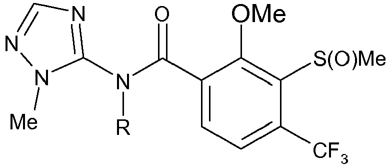

Tabelle 199: Erfindungsgemäße Verbindungen der Formel (I), worin Q für Q2, R7 für Methyl, X für Methoxy; W für C-Y, Y für Methylsulfonyl, V für Wasserstoff und Z für Trifluormethyl steht, und R die in Tabelle 1 angegebenen Bedeutungen hat:

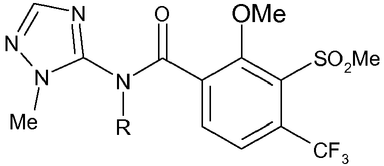

Tabelle 200: Erfindungsgemäße Verbindungen der Formel (I), worin Q für Q2, R⁷ für Methyl, X für Methoxymethyl; W für C-Y, Y für Methylsulfanyl, V für Wasserstoff und Z für Methylsulfonyl steht, und R die in Tabelle 1 angegebenen Bedeutungen hat:

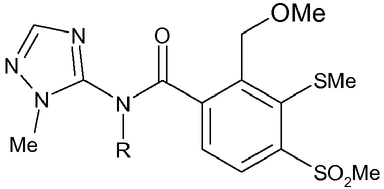

Tabelle 201: Erfindungsgemäße Verbindungen der Formel (I), worin Q für Q2, R⁷ für Methyl, X für Methoxymethyl; W für C-Y, Y für Methylsulfinyl, V für Wasserstoff und Z für Methylsulfonyl steht, und R die in Tabelle 1 angegebenen Bedeutungen hat:

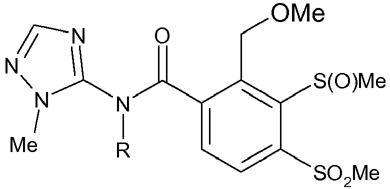

Tabelle 202: Erfindungsgemäße Verbindungen der Formel (I), worin Q für Q2, R⁷ für Methyl, X für Methoxymethyl; W für C-Y, Y für Methylsulfonyl, V für Wasserstoff und Z für Methylsulfonyl steht, und R die in Tabelle 1 angegebenen Bedeutungen hat:

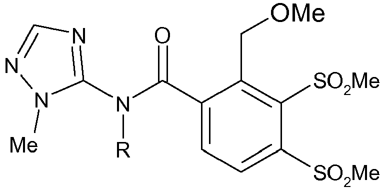

Tabelle 203: Erfindungsgemäße Verbindungen der Formel (I), worin Q für Q2, R⁷ für Methyl, X für Ethyl; W für C-Y, Y für Methylsulfanyl, V für Wasserstoff und Z für Trifluormethyl steht, und R die in Tabelle 1 angegebenen Bedeutungen hat:

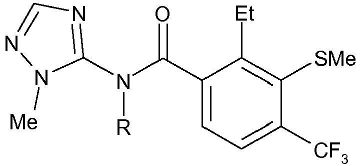

Tabelle 204: Erfindungsgemäße Verbindungen der Formel (I), worin Q für Q2, R⁷ für Methyl, X für Trifluormethyl; W für C-Y, Y für 2-Methoxyethoxy, V für Wasserstoff und Z für Methylsulfonyl steht, und R die in Tabelle 1 angegebenen Bedeutungen hat:

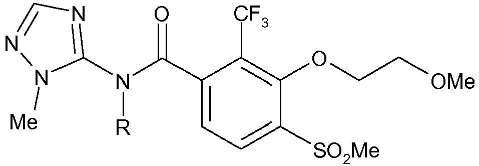

Tabelle 205: Erfindungsgemäße Verbindungen der Formel (I), worin Q für Q3, R⁸ für Methyl, X für Chlor; W für N, V für Wasserstoff und Z für Trifluormethyl steht, und R die in Tabelle 1 angegebenen Bedeutungen hat:

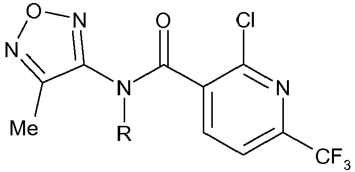

Tabelle 206: Erfindungsgemäße Verbindungen der Formel (I), worin Q für Q3, R⁸ für Methyl, X für Methyl; W für N, V für Wasserstoff und Z für Trifluormethyl steht, und R die in Tabelle 1 angegebenen Bedeutungen hat:

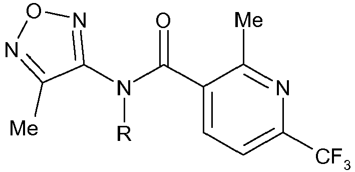

Tabelle 207: Erfindungsgemäße Verbindungen der Formel (I), worin Q für Q3, R⁸ für Methyl, X für Methoxymethyl; W für N, V für Wasserstoff und Z für Trifluormethyl steht, und R die in Tabelle 1 angegebenen Bedeutungen hat:

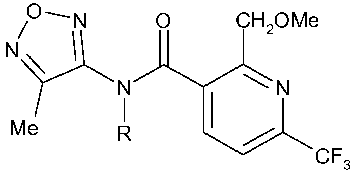

Tabelle 208: Erfindungsgemäße Verbindungen der Formel (I), worin Q für Q3, R⁸ für Methyl, X für Chlor; W für C-Y, Y für (2,2,2-Trifluorethoxy)methyl, V für Wasserstoff und Z für Methylsulfonyl steht, und R die in Tabelle 1 angegebenen Bedeutungen hat:

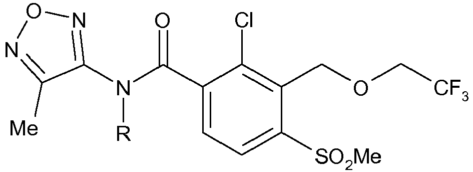

Tabelle 209: Erfindungsgemäße Verbindungen der Formel (I), worin Q für Q3, R⁸ für Methyl, X für Chlor; W für C-Y, Y für 1H-1,2,3-Triazol-1-yl, V für Wasserstoff und Z für Methylsulfonyl steht, und R die in Tabelle 1 angegebenen Bedeutungen hat:

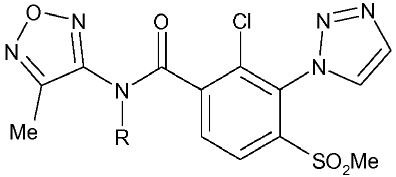

Tabelle 210: Erfindungsgemäße Verbindungen der Formel (I), worin Q für Q3, R⁸ für Methyl, X für Chlor; W für C-Y, Y für 1 H-Pyrazol-1-yl, V für Wasserstoff und Z für Methylsulfonyl steht, und R die in Tabelle 1 angegebenen Bedeutungen hat:

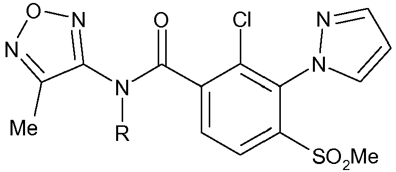

Tabelle 211: Erfindungsgemäße Verbindungen der Formel (I), worin Q für Q3, R⁸ für Chlor, X für Chlor; W für C-Y, Y für 1 H-Pyrazol-1-yl, V für Wasserstoff und Z für Methylsulfonyl steht, und R die in Tabelle 1 angegebenen Bedeutungen hat:

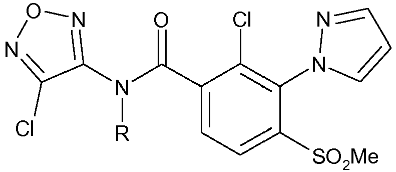

Tabelle 212: Erfindungsgemäße Verbindungen der Formel (I), worin Q für Q3, R⁸ für Chlor, X für Chlor; W für C-Y, Y für 4-Methyl-1 H-pyrazol-1-yl, V für Wasserstoff und Z für Methylsulfonyl steht, und R die in Tabelle 1 angegebenen Bedeutungen hat:

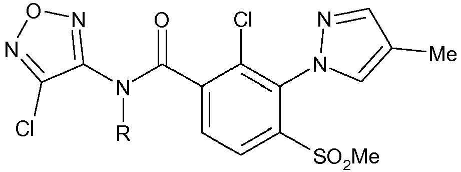

Tabelle 213: Erfindungsgemäße Verbindungen der Formel (I), worin Q für Q3, R⁸ für Ethyl, X für Chlor, W für C-Y, Y für 1 H-Pyrazol-1-yl, V für Wasserstoff und Z für Methylsulfonyl steht, und R die in Tabelle 1 angegebenen Bedeutungen hat:

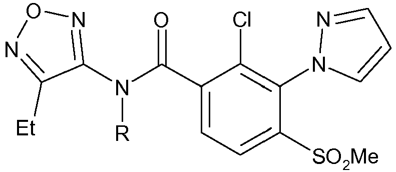

Tabelle 214: Erfindungsgemäße Verbindungen der Formel (I), worin Q für Q3, R⁸ für Chlor, X für Chlor; W für C-Y, Y für Methylsulfanyl, V für Wasserstoff und Z für Trifluormethyl steht, und R die in Tabelle 1 angegebenen Bedeutungen hat:

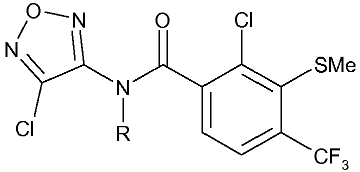

Tabelle 215: Erfindungsgemäße Verbindungen der Formel (I), worin Q für Q3, R⁸ für Methyl, X für Chlor; W für C-Y, Y für Methylsulfanyl, V für Wasserstoff und Z für Trifluormethyl steht, und R die in Tabelle 1 angegebenen Bedeutungen hat:

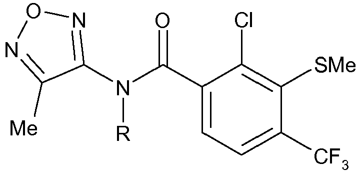

Tabelle 216: Erfindungsgemäße Verbindungen der Formel (I), worin Q für Q3, R⁸ für Methyl, X für Chlor; W für C-Y, Y für Methylsulfinyl, V für Wasserstoff und Z für Trifluormethyl steht, und R die in Tabelle 1 angegebenen Bedeutungen hat:

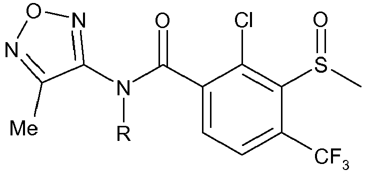

Tabelle 217: Erfindungsgemäße Verbindungen der Formel (I), worin Q für Q3, R⁸ für Chlor, X für Chlor; W für C-Y, Y für Methylsulfinyl, V für Wasserstoff und Z für Trifluormethyl steht, und R die in Tabelle 1 angegebenen Bedeutungen hat:

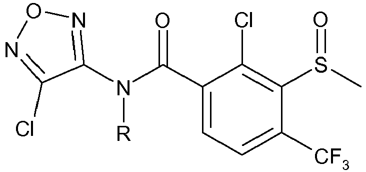

Tabelle 218: Erfindungsgemäße Verbindungen der Formel (I), worin Q für Q3, R⁸ für Methyl, X für Chlor; W für C-Y, Y für Methylsulfonyl, V für Wasserstoff und Z für Trifluormethyl steht, und R die in Tabelle 1 angegebenen Bedeutungen hat:

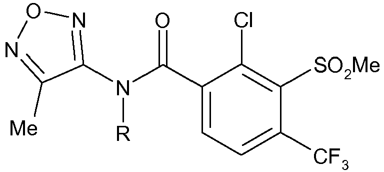

Tabelle 219: Erfindungsgemäße Verbindungen der Formel (I), worin Q für Q3, R⁸ für Chlor, X für Chlor; W für C-Y, Y für Methylsulfonyl, V für Wasserstoff und Z für Trifluormethyl steht, und R die in Tabelle 1 angegebenen Bedeutungen hat:

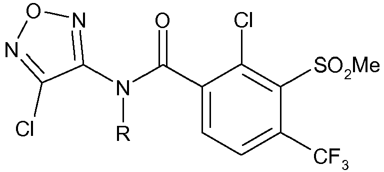

Tabelle 220: Erfindungsgemäße Verbindungen der Formel (I), worin Q für Q3, R⁸ für Methyl, X für Chlor; W für C-Y, Y für Methylsulfanyl, V für Wasserstoff und Z für Methylsulfonyl steht, und R die in Tabelle 1 angegebenen Bedeutungen hat:

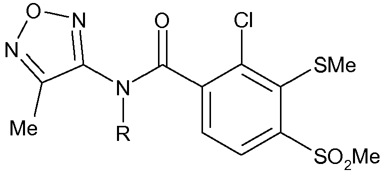

Tabelle 221: Erfindungsgemäße Verbindungen der Formel (I), worin Q für Q3, R⁸ für Methyl, X für Chlor; W für C-Y, Y für Methylsulfinyl, V für Wasserstoff und Z für Methylsulfonyl steht, und R die in Tabelle 1 angegebenen Bedeutungen hat:

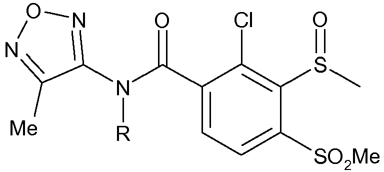

Tabelle 222: Erfindungsgemäße Verbindungen der Formel (I), worin Q für Q3, R⁸ für Chlor, X für Chlor, W für C-Y, Y für Methylsulfinyl, V für Wasserstoff und Z für Methylsulfonyl steht, und R die in Tabelle 1 angegebenen Bedeutungen hat:

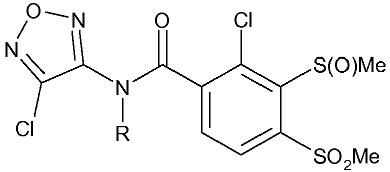

Tabelle 223: Erfindungsgemäße Verbindungen der Formel (I), worin Q für Q3, R⁸ für Methoxymethyl, X für Chlor, W für C-Y, Y für Methylsulfinyl, V für Wasserstoff und Z für Methylsulfonyl steht, und R die in Tabelle 1 angegebenen Bedeutungen hat:

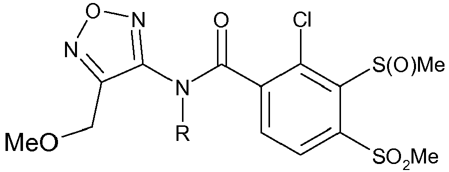

Tabelle 224: Erfindungsgemäße Verbindungen der Formel (I), worin Q für Q3, R⁸ für Methyl, X für Chlor, W für C-Y, Y für Ethylsulfinyl, V für Wasserstoff und Z für Methylsulfonyl steht, und R die in Tabelle 1 angegebenen Bedeutungen hat:

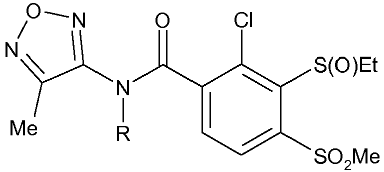

Tabelle 225: Erfindungsgemäße Verbindungen der Formel (I), worin Q für Q3, R⁸ für Methyl, X für Chlor, W für C-Y, Y für Methylsulfonyl, V für Wasserstoff und Z für Methylsulfonyl steht, und R die in Tabelle 1 angegebenen Bedeutungen hat:

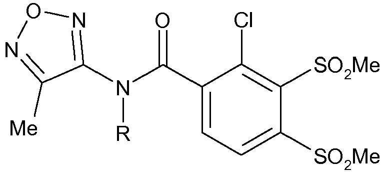

Tabelle 226: Erfindungsgemäße Verbindungen der Formel (I), worin Q für Q3, R⁸ für Chlor, X für Chlor, W für C-Y, Y für Methylsulfonyl, V für Wasserstoff und Z für Methylsulfonyl steht, und R die in Tabelle 1 angegebenen Bedeutungen hat:

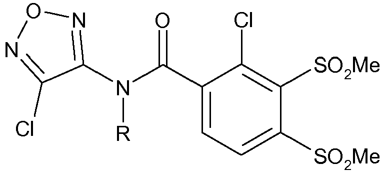

Tabelle 227: Erfindungsgemäße Verbindungen der Formel (I), worin Q für Q3, R⁸ für Methoxymethyl, X für Chlor; W für C-Y, Y für Methylsulfonyl, V für Wasserstoff und Z für Methylsulfonyl steht, und R die in Tabelle 1 angegebenen Bedeutungen hat:

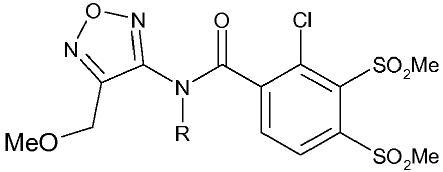

Tabelle 228: Erfindungsgemäße Verbindungen der Formel (I), worin Q für Q3, R⁸ für Methyl, X für Chlor; W für C-Y, Y für 5-Cyanmethyl-4,5-dihydro-1,2-oxazol-3-yl, V für Wasserstoff und Z für Ethylsulfonyl steht, und R die in Tabelle 1 angegebenen Bedeutungen hat:

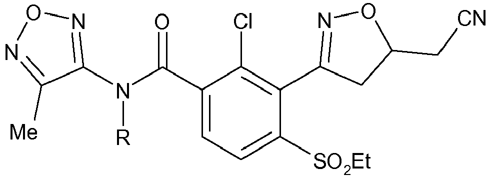

Tabelle 229: Erfindungsgemäße Verbindungen der Formel (I), worin Q für Q3, R⁸ für Methoxy, X für Chlor; W für C-Y, Y für 5-Cyanmethyl-4,5-dihydro-1,2-oxazol-3-yl, V für Wasserstoff und Z für Ethylsulfonyl steht, und R die in Tabelle 1 angegebenen Bedeutungen hat:

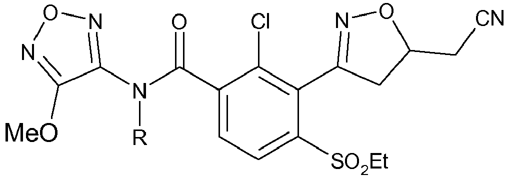

Tabelle 230: Erfindungsgemäße Verbindungen der Formel (I), worin Q für Q3, R⁸ für Methyl, X für Chlor; W für C-Y, Y für 5-Methoxymethyl-4,5-dihydro-1,2-oxazol-3-yl, V für Wasserstoff und Z für Ethylsulfonyl steht, und R die in Tabelle 1 angegebenen Bedeutungen hat:

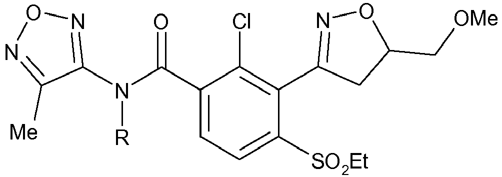

Tabelle 231: Erfindungsgemäße Verbindungen der Formel (I), worin Q für Q3, R⁸ für Methyl, X für Methyl, W für C-Y, Y für 1 H-Pyrazol-1-yl, V für Wasserstoff und Z für Methylsulfonyl steht, und R die in Tabelle 1 angegebenen Bedeutungen hat:

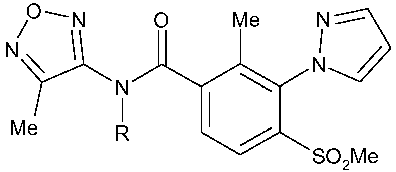

Tabelle 232: Erfindungsgemäße Verbindungen der Formel (I), worin Q für Q3, R⁸ für Ethyl, X für Methyl, W für C-Y, Y für 1 H-Pyrazol-1-yl, V für Wasserstoff und Z für Methylsulfonyl steht, und R die in Tabelle 1 angegebenen Bedeutungen hat:

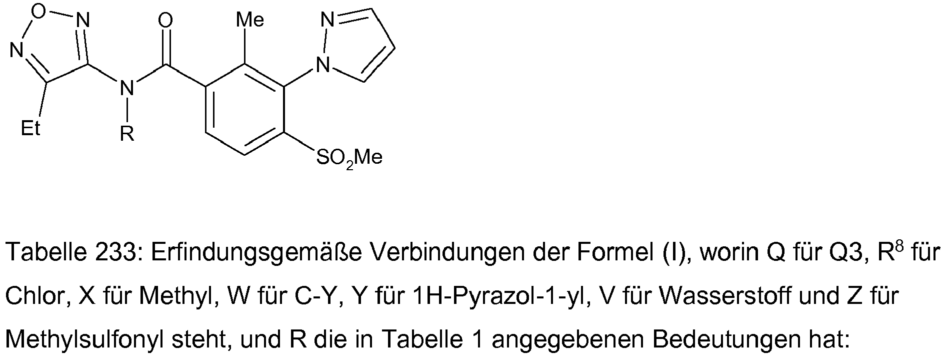

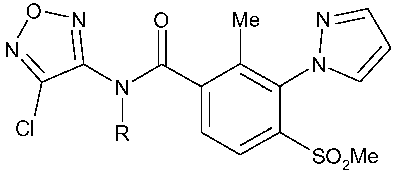

Tabelle 234: Erfindungsgemäße Verbindungen der Formel (I), worin Q für Q3, R⁸ für Methyl, X für Methyl, W für C-Y, Y für Methylsulfanyl, V für Wasserstoff und Z für Methylsulfonyl steht, und R die in Tabelle 1 angegebenen Bedeutungen hat:

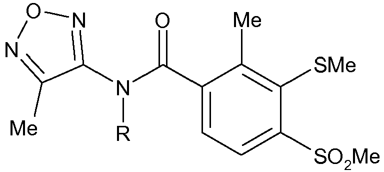

Tabelle 235: Erfindungsgemäße Verbindungen der Formel (I), worin Q für Q3, R⁸ für Chlor, X für Methyl, W für C-Y, Y für Methylsulfanyl, V für Wasserstoff und Z für Methylsulfonyl steht, und R die in Tabelle 1 angegebenen Bedeutungen hat:

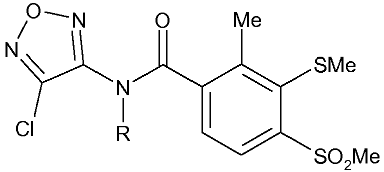

Tabelle 236: Erfindungsgemäße Verbindungen der Formel (I), worin Q für Q3, R⁸ für Methyl, X für Methyl, W für C-Y, Y für Methylsulfinyl, V für Wasserstoff und Z für Methylsulfonyl steht, und R die in Tabelle 1 angegebenen Bedeutungen hat:

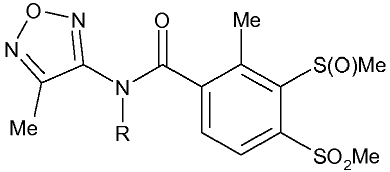

Tabelle 237: Erfindungsgemäße Verbindungen der Formel (I), worin Q für Q3, R⁸ für Chlor, X für Methyl, W für C-Y, Y für Methylsulfinyl, V für Wasserstoff und Z für Methylsulfonyl steht, und R die in Tabelle 1 angegebenen Bedeutungen hat:

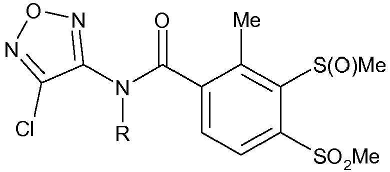

Tabelle 238: Erfindungsgemäße Verbindungen der Formel (I), worin Q für Q3, R⁸ für Methyl, X für Methyl, W für C-Y, Y für Methylsulfonyl, V für Wasserstoff und Z für Methylsulfonyl steht, und R die in Tabelle 1 angegebenen Bedeutungen hat:

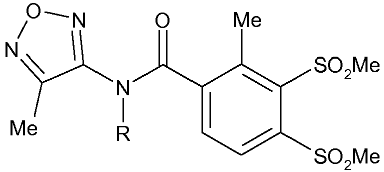

Tabelle 239: Erfindungsgemäße Verbindungen der Formel (I), worin Q für Q3, R⁸ für Chlor, X für Methyl, W für C-Y, Y für Methylsulfonyl, V für Wasserstoff und Z für Methylsulfonyl steht, und R die in Tabelle 1 angegebenen Bedeutungen hat:

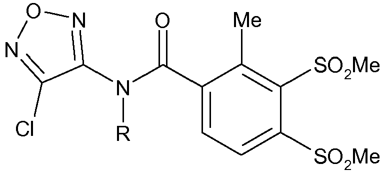

Tabelle 240: Erfindungsgemäße Verbindungen der Formel (I), worin Q für Q3, R⁸ für Cyclopropyl, X für Methyl, W für C-Y, Y für Methylsulfonyl, V für Wasserstoff und Z für Methylsulfonyl steht, und R die in Tabelle 1 angegebenen Bedeutungen hat:

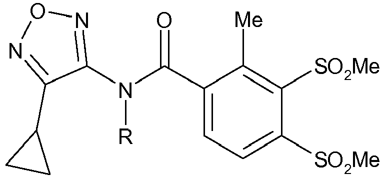

Tabelle 241: Erfindungsgemäße Verbindungen der Formel (I), worin Q für Q3, R⁸ für Methyl, X für Methyl, W für C-Y, Y für (2-Methoxyethyl)sulfonyl, V für Wasserstoff und Z für Methylsulfonyl steht, und R die in Tabelle 1 angegebenen Bedeutungen hat:

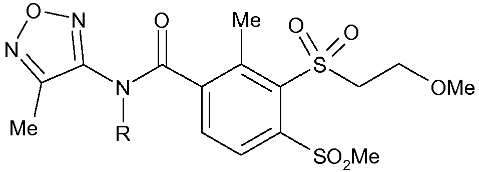

Tabelle 242: Erfindungsgemäße Verbindungen der Formel (I), worin Q für Q3, R⁸ für Chlor, X für Methyl, W für C-Y, Y für (2-Methoxyethyl)sulfonyl, V für Wasserstoff und Z für Methylsulfonyl steht, und R die in Tabelle 1 angegebenen Bedeutungen hat:

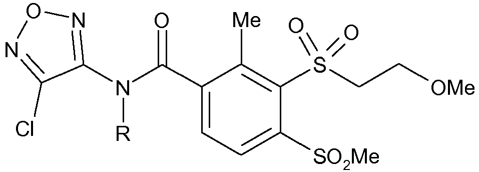

Tabelle 243: Erfindungsgemäße Verbindungen der Formel (I), worin Q für Q3, R⁸ für Methyl, X für Methyl, W für C-Y, Y für Ethylsulfonyl, V für Wasserstoff und Z für Methylsulfonyl steht, und R die in Tabelle 1 angegebenen Bedeutungen hat:

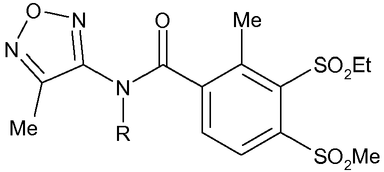

Tabelle 244: Erfindungsgemäße Verbindungen der Formel (I), worin Q für Q3, R⁸ für Ethyl, X für Methyl, W für C-Y, Y für Ethylsulfonyl, V für Wasserstoff und Z für Methylsulfonyl steht, und R die in Tabelle 1 angegebenen Bedeutungen hat:

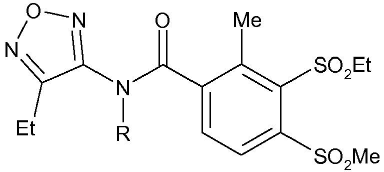

Tabelle 245: Erfindungsgemäße Verbindungen der Formel (I), worin Q für Q3, R⁸ für Chlor, X für Methyl, W für C-Y, Y für Ethylsulfonyl, V für Wasserstoff und Z für Methylsulfonyl steht, und R die in Tabelle 1 angegebenen Bedeutungen hat:

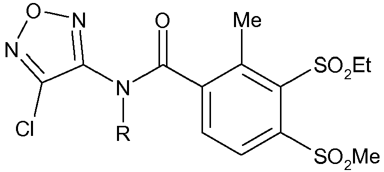

Tabelle 246: Erfindungsgemäße Verbindungen der Formel (I), worin Q für Q3, R⁸ für Amino, X für Methyl, W für C-Y, Y für Ethylsulfonyl, V für Wasserstoff und Z für Methylsulfonyl steht, und R die in Tabelle 1 angegebenen Bedeutungen hat:

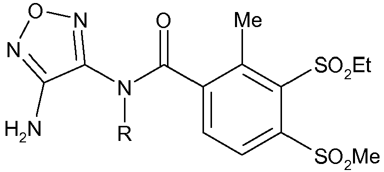

Tabelle 247: Erfindungsgemäße Verbindungen der Formel (I), worin Q für Q3, R⁸ für Acetylamino, X für Methyl, W für C-Y, Y für Methylsulfonyl, V für Wasserstoff und Z für Methylsulfonyl steht, und R die in Tabelle 1 angegebenen Bedeutungen hat:

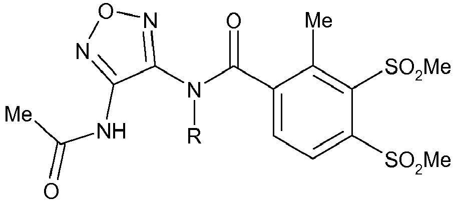

Tabelle 248: Erfindungsgemäße Verbindungen der Formel (I), worin Q für Q3, R⁸ für Methoxy, X für Methyl, W für C-Y, Y für Methylsulfonyl, V für Wasserstoff und Z für Methylsulfonyl steht, und R die in Tabelle 1 angegebenen Bedeutungen hat:

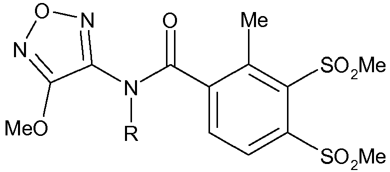

Tabelle 249: Erfindungsgemäße Verbindungen der Formel (I), worin Q für Q3, R⁸ für Methyl, X für Methyl, W für C-Y, Y für Methylsulfanyl, V für Wasserstoff und Z für Trifluormethyl steht, und R die in Tabelle 1 angegebenen Bedeutungen hat:

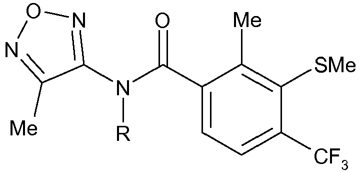

Tabelle 250: Erfindungsgemäße Verbindungen der Formel (I), worin Q für Q3, R⁸ für Chlor, X für Methyl, W für C-Y, Y für Methylsulfanyl, V für Wasserstoff und Z für Trifluormethyl steht, und R die in Tabelle 1 angegebenen Bedeutungen hat:

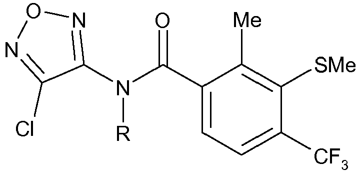

Tabelle 251: Erfindungsgemäße Verbindungen der Formel (I), worin Q für Q3, R⁸ für Methyl, X für Methyl, W für C-Y, Y für Methylsulfinyl, V für Wasserstoff und Z für Trifluormethyl steht, und R die in Tabelle 1 angegebenen Bedeutungen hat:

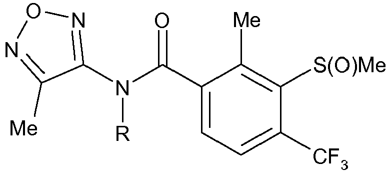

Tabelle 252: Erfindungsgemäße Verbindungen der Formel (I), worin Q für Q3, R⁸ für Chlor, X für Methyl, W für C-Y, Y für Methylsulfinyl, V für Wasserstoff und Z für Trifluormethyl steht, und R die in Tabelle 1 angegebenen Bedeutungen hat:

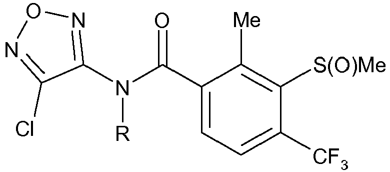

Tabelle 253: Erfindungsgemäße Verbindungen der Formel (I), worin Q für Q3, R⁸ für Methoxymethyl, X für Methyl, W für C-Y, Y für Methylsulfinyl, V für Wasserstoff und Z für Trifluormethyl steht, und R die in Tabelle 1 angegebenen Bedeutungen hat:

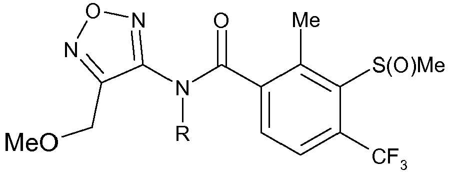

Tabelle 254: Erfindungsgemäße Verbindungen der Formel (I), worin Q für Q3, R⁸ für 1H-1,2,4-Triazol-1-yl, X für Methyl, W für C-Y, Y für Methylsulfinyl, V für Wasserstoff und Z für Trifluormethyl steht, und R die in Tabelle 1 angegebenen Bedeutungen hat:

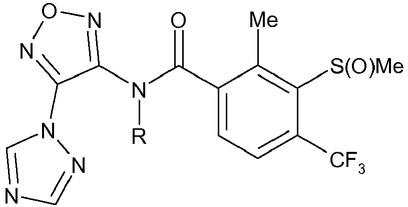

Tabelle 255: Erfindungsgemäße Verbindungen der Formel (I), worin Q für Q3, R⁸ für Methyl, X für Methyl, W für C-Y, Y für Methylsulfonyl, V für Wasserstoff und Z für Trifluormethyl steht, und R die in Tabelle 1 angegebenen Bedeutungen hat:

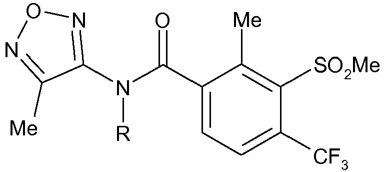

Tabelle 256: Erfindungsgemäße Verbindungen der Formel (I), worin Q für Q3, R⁸ für Chlor, X für Methyl, W für C-Y, Y für Methylsulfonyl, V für Wasserstoff und Z für Trifluormethyl steht, und R die in Tabelle 1 angegebenen Bedeutungen hat:

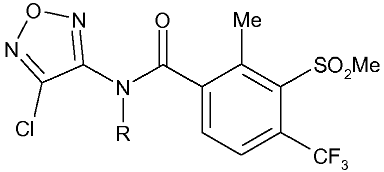

Tabelle 257: Erfindungsgemäße Verbindungen der Formel (I), worin Q für Q3, R⁸ für Methylsulfanyl, X für Methyl, W für C-Y, Y für Methylsulfonyl, V für Wasserstoff und Z für Trifluormethyl steht, und R die in Tabelle 1 angegebenen Bedeutungen hat:

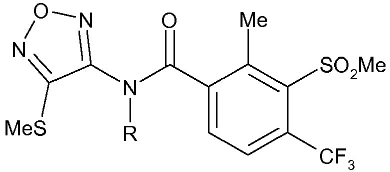

Tabelle 258: Erfindungsgemäße Verbindungen der Formel (I), worin Q für Q3, R⁸ für Nitro, X für Methyl, W für C-Y, Y für Methylsulfonyl, V für Wasserstoff und Z für Trifluormethyl steht, und R die in Tabelle 1 angegebenen Bedeutungen hat:

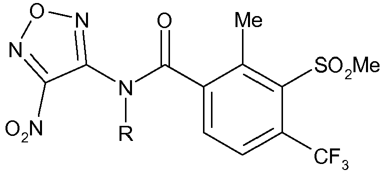

Tabelle 259: Erfindungsgemäße Verbindungen der Formel (I), worin Q für Q3, R⁸ für Methoxymethyl, X für Methyl, W für C-Y, Y für Methylsulfonyl, V für Wasserstoff und Z für Trifluormethyl steht, und R die in Tabelle 1 angegebenen Bedeutungen hat:

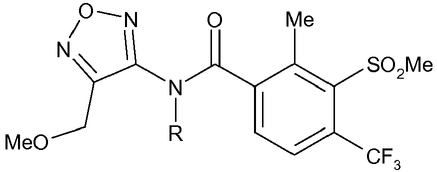

Tabelle 260: Erfindungsgemäße Verbindungen der Formel (I), worin Q für Q3, R⁸ für (1 H-Pyrazol-1-yl)methyl, X für Methyl, W für C-Y, Y für Methylsulfonyl, V für Wasserstoff und Z für Trifluormethyl steht, und R die in Tabelle 1 angegebenen Bedeutungen hat:

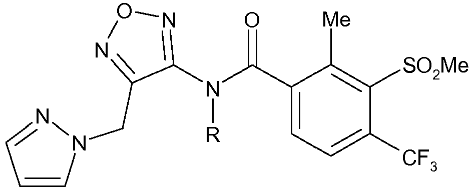

Tabelle 261: Erfindungsgemäße Verbindungen der Formel (I), worin Q für Q3, R⁸ für Acetylamino, X für Methyl, W für C-Y, Y für Methylsulfonyl, V für Wasserstoff und Z für Trifluormethyl steht, und R die in Tabelle 1 angegebenen Bedeutungen hat:

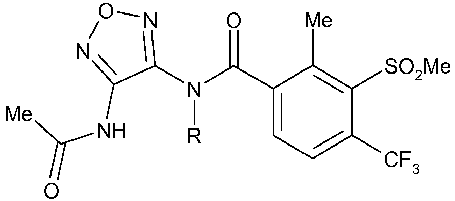

Tabelle 262: Erfindungsgemäße Verbindungen der Formel (I), worin Q für Q3, R⁸ für (2,2-Dimethylpropanoyl)amino, X für Methyl, W für C-Y, Y für Methylsulfonyl, V für Wasserstoff und Z für Trifluormethyl steht, und R die in Tabelle 1 angegebenen Bedeutungen hat:

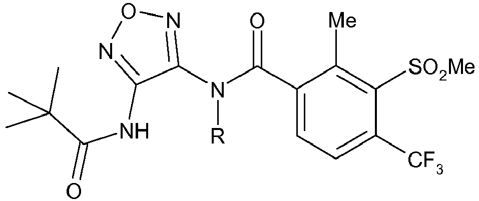

Tabelle 263: Erfindungsgemäße Verbindungen der Formel (I), worin Q für Q3, R⁸ für Methyl, X für Methyl, W für C-Y, Y für Ethylsulfinyl, V für Wasserstoff und Z für Trifluormethyl steht, und R die in Tabelle 1 angegebenen Bedeutungen hat:

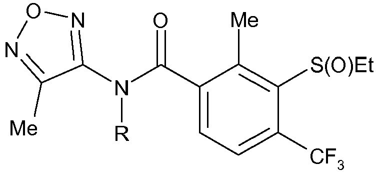

Tabelle 264: Erfindungsgemäße Verbindungen der Formel (I), worin Q für Q3, R⁸ für Chlor, X für Methyl, W für C-Y, Y für Ethylsulfanyl, V für Wasserstoff und Z für Trifluormethyl steht, und R die in Tabelle 1 angegebenen Bedeutungen hat:

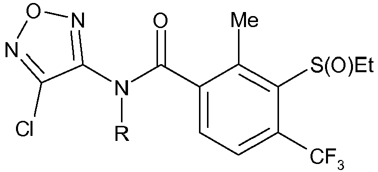

Tabelle 265: Erfindungsgemäße Verbindungen der Formel (I), worin Q für Q3, R⁸ für Methyl, X für Ethyl, W für C-Y, Y für Methylsulfonyl, V für Wasserstoff und Z für Trifluormethyl steht, und R die in Tabelle 1 angegebenen Bedeutungen hat:

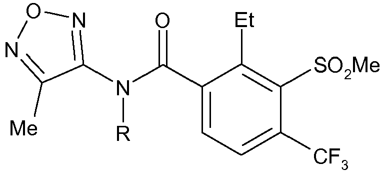

Tabelle 266: Erfindungsgemäße Verbindungen der Formel (I), worin Q für Q3, R⁸ für Methyl, X für Methoxy, W für C-Y, Y für Methylsulfanyl, V für Wasserstoff und Z für Methylsulfonyl steht, und R die in Tabelle 1 angegebenen Bedeutungen hat:

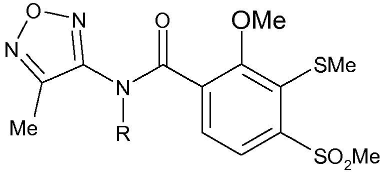

Tabelle 267: Erfindungsgemäße Verbindungen der Formel (I), worin Q für Q3, R⁸ für Methyl, X für Methoxy, W für C-Y, Y für Methylsulfinyl, V für Wasserstoff und Z für Methylsulfonyl steht, und R die in Tabelle 1 angegebenen Bedeutungen hat:

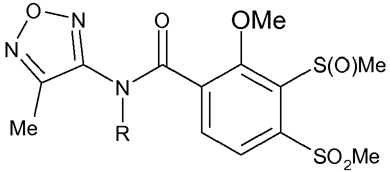

Tabelle 268: Erfindungsgemäße Verbindungen der Formel (I), worin Q für Q3, R⁸ für Methyl, X für Methoxy, W für C-Y, Y für Methylsulfonyl, V für Wasserstoff und Z für Methylsulfonyl steht, und R die in Tabelle 1 angegebenen Bedeutungen hat:

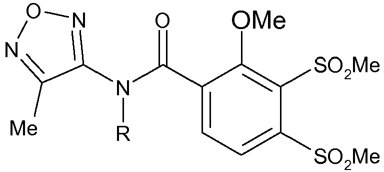

Tabelle 269: Erfindungsgemäße Verbindungen der Formel (I), worin Q für Q3, R⁸ für Methyl, X für Methoxy, W für C-Y, Y für Methylsulfanyl, V für Wasserstoff und Z für Trifluormethyl steht, und R die in Tabelle 1 angegebenen Bedeutungen hat:

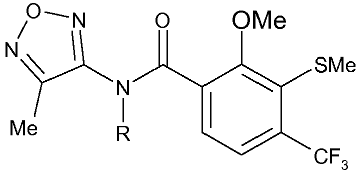

Tabelle 270: Erfindungsgemäße Verbindungen der Formel (I), worin Q für Q3, R⁸ für Methyl, X für Methoxy, W für C-Y, Y für Methylsulfinyl, V für Wasserstoff und Z für Trifluormethyl steht, und R die in Tabelle 1 angegebenen Bedeutungen hat:

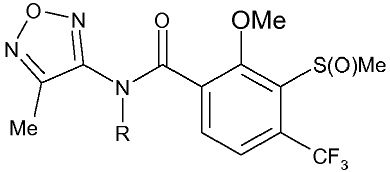

Tabelle 271: Erfindungsgemäße Verbindungen der Formel (I), worin Q für Q3, R⁸ für Methyl, X für Methoxy, W für C-Y, Y für Methylsulfonyl, V für Wasserstoff und Z für Trifluormethyl steht, und R die in Tabelle 1 angegebenen Bedeutungen hat:

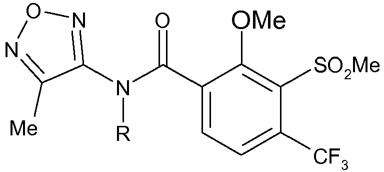

Tabelle 272: Erfindungsgemäße Verbindungen der Formel (I), worin Q für Q3, R⁸ für Methyl, X für Methoxymethyl, W für C-Y, Y für Methylsulfanyl, V für Wasserstoff und Z für Methylsulfonyl steht, und R die in Tabelle 1 angegebenen Bedeutungen hat:

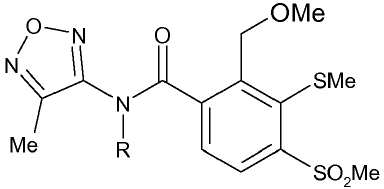

Tabelle 273: Erfindungsgemäße Verbindungen der Formel (I), worin Q für Q3, R⁸ für Methyl, X für Methoxymethyl, W für C-Y, Y für Methylsulfinyl, V für Wasserstoff und Z für Methylsulfonyl steht, und R die in Tabelle 1 angegebenen Bedeutungen hat:

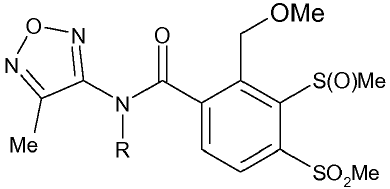

Tabelle 274: Erfindungsgemäße Verbindungen der Formel (I), worin Q für Q3, R⁸ für Methyl, X für Methoxymethyl, W für C-Y, Y für Methylsulfonyl, V für Wasserstoff und Z für Methylsulfonyl steht, und R die in Tabelle 1 angegebenen Bedeutungen hat:

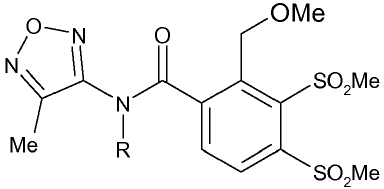

Tabelle 275: Erfindungsgemäße Verbindungen der Formel (I), worin Q für Q3, R⁸ für Methyl, X für Trifluormethyl, W für C-Y, Y für Methylsulfanyl, V für Wasserstoff und Z für Trifluormethyl steht, und R die in Tabelle 1 angegebenen Bedeutungen hat:

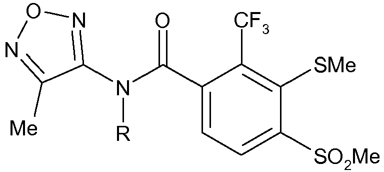

Tabelle 276: Erfindungsgemäße Verbindungen der Formel (I), worin Q für Q4, R⁹ für Methyl, X für Chlor, W für N, V für Wasserstoff und Z für Trifluormethyl steht, und R die in Tabelle 1 angegebenen Bedeutungen hat:

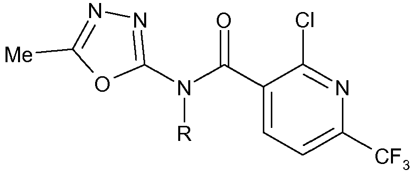

Tabelle 277: Erfindungsgemäße Verbindungen der Formel (I), worin Q für Q4, R⁹ für Ethyl, X für Chlor, W für N, V für Wasserstoff und Z für Trifluormethyl steht, und R die in Tabelle 1 angegebenen Bedeutungen hat:

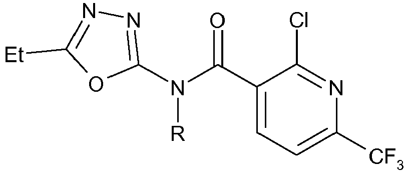

Tabelle 278: Erfindungsgemäße Verbindungen der Formel (I), worin Q für Q4, R⁹ für Methoxymethyl, X für Chlor, W für N, V für Wasserstoff und Z für Trifluormethyl steht, und R die in Tabelle 1 angegebenen Bedeutungen hat:

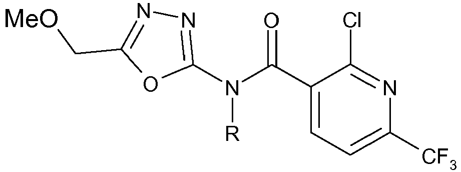

Tabelle 279: Erfindungsgemäße Verbindungen der Formel (I), worin Q für Q4, R⁹ für Ethyl, X für Methoxymethyl, W für N, V für Wasserstoff und Z für Trifluormethyl steht, und R die in Tabelle 1 angegebenen Bedeutungen hat:

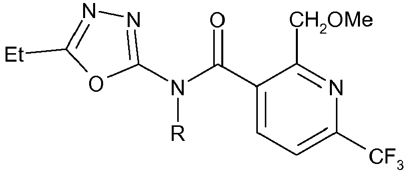

Tabelle 280: Erfindungsgemäße Verbindungen der Formel (I), worin Q für Q4, R⁹ für Wasserstoff, X für Chlor, W für C-Y, Y für (2,2,2-Trifluorethoxy)methyl, V für Wasserstoff und Z für Methylsulfonyl steht, und R die in Tabelle 1 angegebenen Bedeutungen hat:

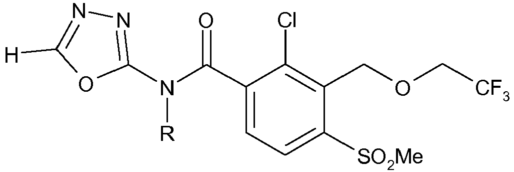

Tabelle 281: Erfindungsgemäße Verbindungen der Formel (I), worin Q für Q4, R⁹ für Methyl, X für Chlor, W für C-Y, Y für (2,2,2-Trifluorethoxy)methyl, V für Wasserstoff und Z für Methylsulfonyl steht, und R die in Tabelle 1 angegebenen Bedeutungen hat:

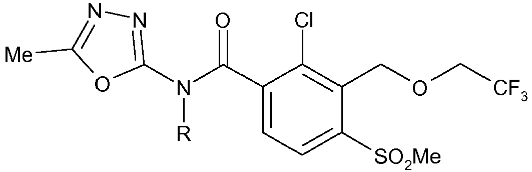

Tabelle 282: Erfindungsgemäße Verbindungen der Formel (I), worin Q für Q4, R⁹ für Isopropyl, X für Chlor, W für C-Y, Y für (2,2,2-Trifluorethoxy)methyl, V für Wasserstoff und Z für Methylsulfonyl steht, und R die in Tabelle 1 angegebenen Bedeutungen hat:

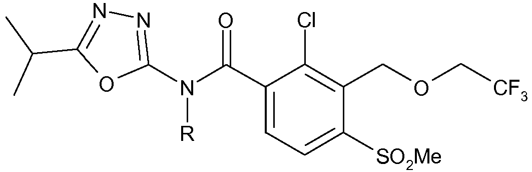

Tabelle 283: Erfindungsgemäße Verbindungen der Formel (I), worin Q für Q4, R⁹ für Methyl, X für Chlor, W für C-Y, Y für Propoxy, V für Wasserstoff und Z für Methylsulfonyl steht, und R die in Tabelle 1 angegebenen Bedeutungen hat:

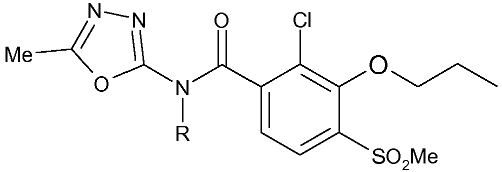

Tabelle 284: Erfindungsgemäße Verbindungen der Formel (I), worin Q für Q4, R⁹ für Methyl, X für Chlor, W für C-Y, Y für Cyclopropylmethoxy, V für Wasserstoff und Z für Methylsulfonyl steht, und R die in Tabelle 1 angegebenen Bedeutungen hat:

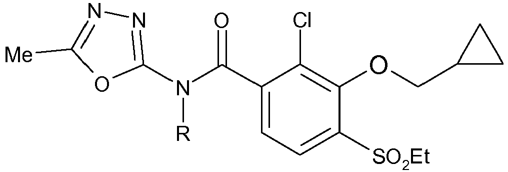

Tabelle 285: Erfindungsgemäße Verbindungen der Formel (I), worin Q für Q4, R⁹ für Methyl, X für Chlor, W für C-Y, Y für 5-Cyanmethyl-4,5-dihydro-1,2-oxazol-3-yl, V für Wasserstoff und Z für Ethylsulfonyl steht, und R die in Tabelle 1 angegebenen Bedeutungen hat:

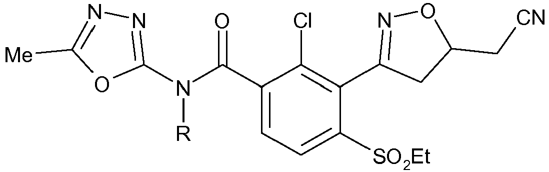

Tabelle 286: Erfindungsgemäße Verbindungen der Formel (I), worin Q für Q4, R⁹ für Ethyl, X für Chlor, W für C-Y, Y für 5-Cyanmethyl-4,5-dihydro-1,2-oxazol-3-yl, V für Wasserstoff und Z für Ethylsulfonyl steht, und R die in Tabelle 1 angegebenen Bedeutungen hat:

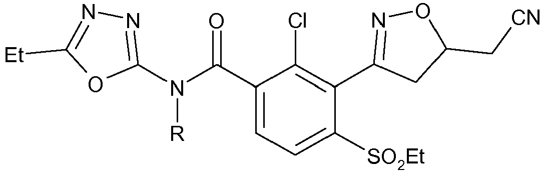

Tabelle 287: Erfindungsgemäße Verbindungen der Formel (I), worin Q für Q4, R⁹ für Methyl, X für Chlor, W für C-Y, Y für Methylsulfanyl, V für Wasserstoff und Z für Methylsulfonyl steht, und R die in Tabelle 1 angegebenen Bedeutungen hat:

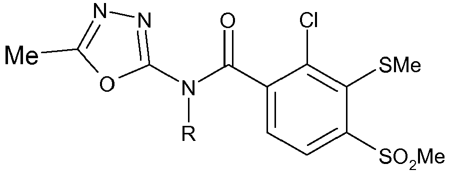

Tabelle 288: Erfindungsgemäße Verbindungen der Formel (I), worin Q für Q4, R⁹ für Ethyl, X für Chlor, W für C-Y, Y für Methylsulfanyl, V für Wasserstoff und Z für Methylsulfonyl steht, und R die in Tabelle 1 angegebenen Bedeutungen hat:

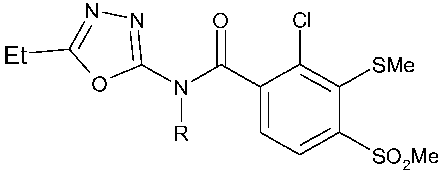

Tabelle 289: Erfindungsgemäße Verbindungen der Formel (I), worin Q für Q4, R⁹ für Methoxymethyl, X für Chlor, W für C-Y, Y für Methylsulfanyl, V für Wasserstoff und Z für Methylsulfonyl steht, und R die in Tabelle 1 angegebenen Bedeutungen hat:

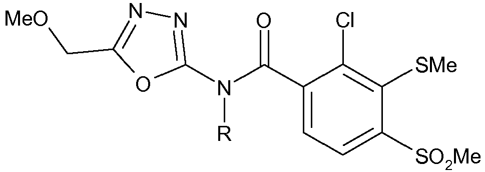

Tabelle 290: Erfindungsgemäße Verbindungen der Formel (I), worin Q für Q4, R⁹ für Methyl, X für Chlor, W für C-Y, Y für Methylsulfinyl, V für Wasserstoff und Z für Methylsulfonyl steht, und R die in Tabelle 1 angegebenen Bedeutungen hat:

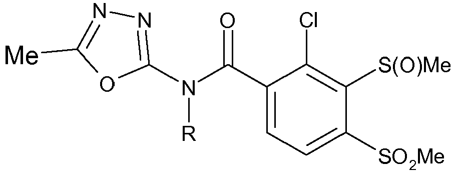

Tabelle 291: Erfindungsgemäße Verbindungen der Formel (I), worin Q für Q4, R⁹ für Ethyl, X für Chlor, W für C-Y, Y für Methylsulfinyl, V für Wasserstoff und Z für Methylsulfonyl steht, und R die in Tabelle 1 angegebenen Bedeutungen hat:

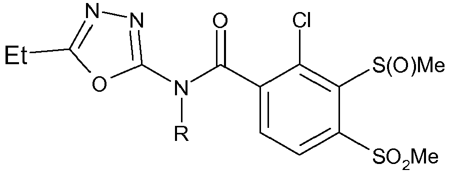

Tabelle 292: Erfindungsgemäße Verbindungen der Formel (I), worin Q für Q4, R⁹ für Methoxymethyl, X für Chlor, W für C-Y, Y für Methylsulfinyl, V für Wasserstoff und Z für Methylsulfonyl steht, und R die in Tabelle 1 angegebenen Bedeutungen hat:

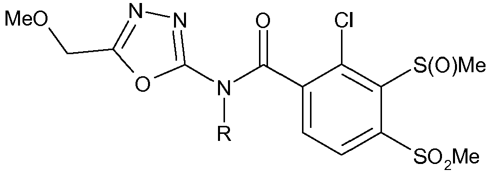

Tabelle 293: Erfindungsgemäße Verbindungen der Formel (I), worin Q für Q4, R⁹ für Methyl, X für Chlor, W für C-Y, Y für Methylsulfonyl, V für Wasserstoff und Z für Methylsulfonyl steht, und R die in Tabelle 1 angegebenen Bedeutungen hat:

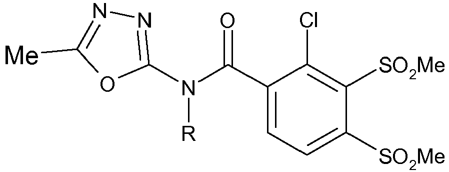

Tabelle 294: Erfindungsgemäße Verbindungen der Formel (I), worin Q für Q4, R⁹ für Ethyl, X für Chlor, W für C-Y, Y für Methylsulfonyl, V für Wasserstoff und Z für Methylsulfonyl steht, und R die in Tabelle 1 angegebenen Bedeutungen hat:

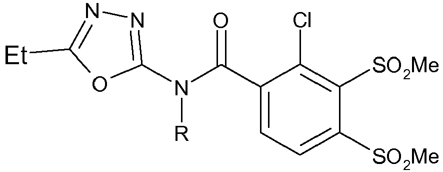

Tabelle 295: Erfindungsgemäße Verbindungen der Formel (I), worin Q für Q4, R⁹ für Methoxymethyl, X für Chlor, W für C-Y, Y für Methylsulfonyl, V für Wasserstoff und Z für Methylsulfonyl steht, und R die in Tabelle 1 angegebenen Bedeutungen hat:

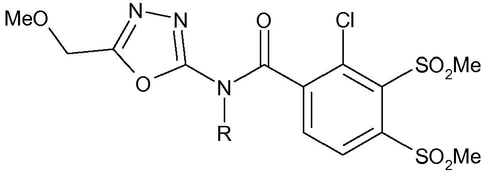

Tabelle 296: Erfindungsgemäße Verbindungen der Formel (I), worin Q für Q4, R⁹ für Methyl, X für Chlor, W für C-Y, Y für Methylsulfanyl, V für Wasserstoff und Z für Trifluormethyl steht, und R die in Tabelle 1 angegebenen Bedeutungen hat:

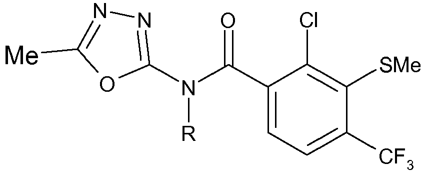

Tabelle 297: Erfindungsgemäße Verbindungen der Formel (I), worin Q für Q4, R⁹ für Ethyl, X für Chlor, W für C-Y, Y für Methylsulfanyl, V für Wasserstoff und Z für Trifluormethyl steht, und R die in Tabelle 1 angegebenen Bedeutungen hat:

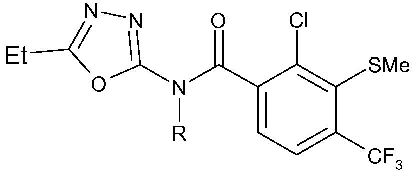

Tabelle 298: Erfindungsgemäße Verbindungen der Formel (I), worin Q für Q4, R⁹ für Methoxymethyl, X für Chlor, W für C-Y, Y für Methylsulfanyl, V für Wasserstoff und Z für Trifluormethyl steht, und R die in Tabelle 1 angegebenen Bedeutungen hat:

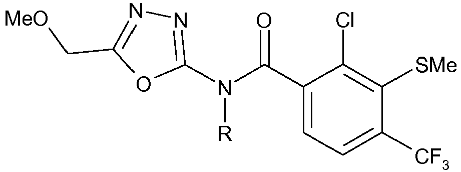

Tabelle 299: Erfindungsgemäße Verbindungen der Formel (I), worin Q für Q4, R⁹ für Methyl, X für Chlor, W für C-Y, Y für Methylsulfinyl, V für Wasserstoff und Z für Trifluormethyl steht, und R die in Tabelle 1 angegebenen Bedeutungen hat:

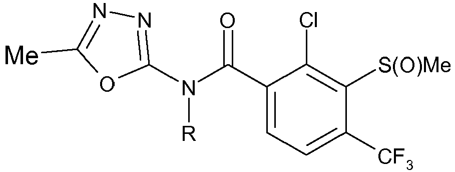

Tabelle 300: Erfindungsgemäße Verbindungen der Formel (I), worin Q für Q4, R⁹ für Ethyl, X für Chlor, W für C-Y, Y für Methylsulfinyl, V für Wasserstoff und Z für Trifluormethyl steht, und R die in Tabelle 1 angegebenen Bedeutungen hat:

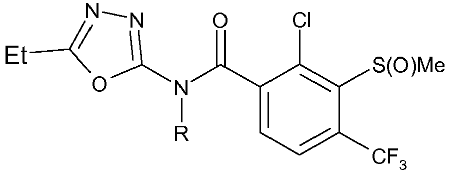

Tabelle 301: Erfindungsgemäße Verbindungen der Formel (I), worin Q für Q4, R⁹ für Methoxymethyl, X für Chlor, W für C-Y, Y für Methylsulfinyl, V für Wasserstoff und Z für Trifluormethyl steht, und R die in Tabelle 1 angegebenen Bedeutungen hat:

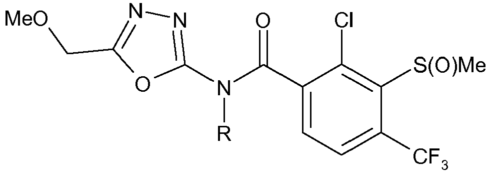

Tabelle 302: Erfindungsgemäße Verbindungen der Formel (I), worin Q für Q4, R⁹ für Methyl, X für Chlor, W für C-Y, Y für Methylsulfonyl, V für Wasserstoff und Z für Trifluormethyl steht, und R die in Tabelle 1 angegebenen Bedeutungen hat:

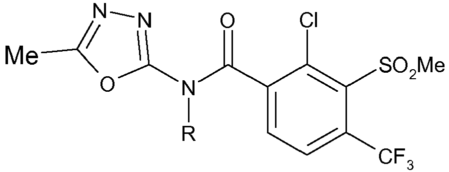

Tabelle 303: Erfindungsgemäße Verbindungen der Formel (I), worin Q für Q4, R⁹ für Ethyl, X für Chlor, W für C-Y, Y für Methylsulfonyl, V für Wasserstoff und Z für Trifluormethyl steht, und R die in Tabelle 1 angegebenen Bedeutungen hat:

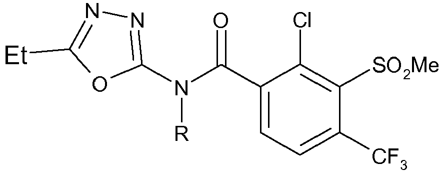

Tabelle 304: Erfindungsgemäße Verbindungen der Formel (I), worin Q für Q4, R⁹ für Methoxymethyl, X für Chlor, W für C-Y, Y für Methylsulfonyl, V für Wasserstoff und Z für Trifluormethyl steht, und R die in Tabelle 1 angegebenen Bedeutungen hat:

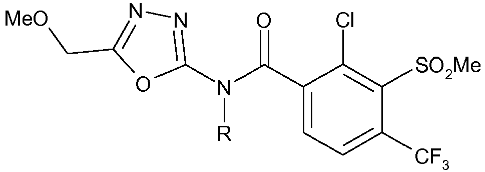

Tabelle 305: Erfindungsgemäße Verbindungen der Formel (I), worin Q für Q4, R⁹ für Ethyl, X für Fluor, W für C-Y, Y für Methylsulfinyl, V für Wasserstoff und Z für Trifluormethyl steht, und R die in Tabelle 1 angegebenen Bedeutungen hat:

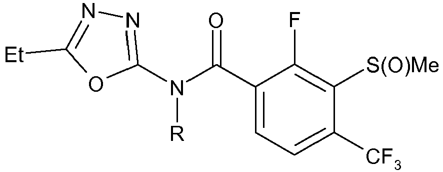

Tabelle 306: Erfindungsgemäße Verbindungen der Formel (I), worin Q für Q4, R⁹ für Methyl, X für Brom, W für C-Y, Y für Methylsulfanyl, V für Wasserstoff und Z für Methyl steht, und R die in Tabelle 1 angegebenen Bedeutungen hat:

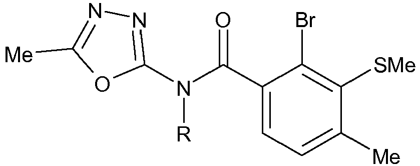

Tabelle 307: Erfindungsgemäße Verbindungen der Formel (I), worin Q für Q4, R⁹ für Methyl, X für Brom, W für C-Y, Y für Methylsulfinyl, V für Wasserstoff und Z für Methyl steht, und R die in Tabelle 1 angegebenen Bedeutungen hat:

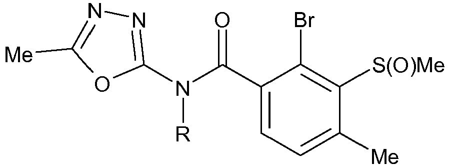

Tabelle 308: Erfindungsgemäße Verbindungen der Formel (I), worin Q für Q4, R⁹ für Methyl, X für Methyl, W für C-Y, Y für Methylsulfanyl, V für Wasserstoff und Z für Trifluormethyl steht, und R die in Tabelle 1 angegebenen Bedeutungen hat:

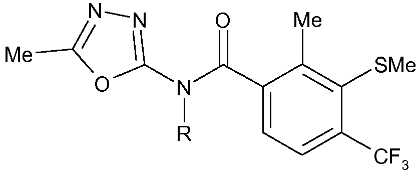

Tabelle 309: Erfindungsgemäße Verbindungen der Formel (I), worin Q für Q4, R⁹ für Ethyl, X für Methyl, W für C-Y, Y für Methylsulfanyl, V für Wasserstoff und Z für Trifluormethyl steht, und R die in Tabelle 1 angegebenen Bedeutungen hat:

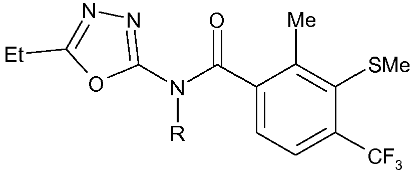

Tabelle 310: Erfindungsgemäße Verbindungen der Formel (I), worin Q für Q4, R⁹ für Methoxymethyl, X für Methyl, W für C-Y, Y für Methylsulfanyl, V für Wasserstoff und Z für Trifluormethyl steht, und R die in Tabelle 1 angegebenen Bedeutungen hat:

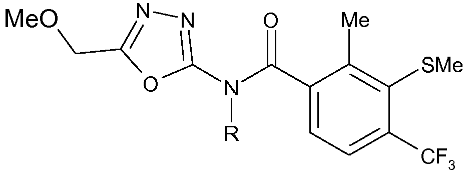

Tabelle 311: Erfindungsgemäße Verbindungen der Formel (I), worin Q für Q4, R⁹ für Methyl, X für Methyl, W für C-Y, Y für Methylsulfinyl, V für Wasserstoff und Z für Trifluormethyl steht, und R die in Tabelle 1 angegebenen Bedeutungen hat:

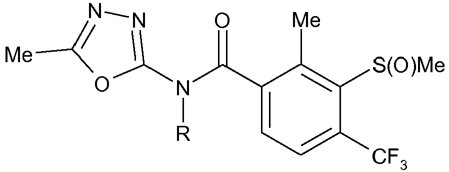

Tabelle 312: Erfindungsgemäße Verbindungen der Formel (I), worin Q für Q4, R⁹ für Cyclopropyl, X für Methyl, W für C-Y, Y für Methylsulfinyl, V für Wasserstoff und Z für Trifluormethyl steht, und R die in Tabelle 1 angegebenen Bedeutungen hat:

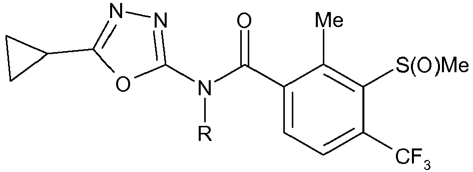

Tabelle 313: Erfindungsgemäße Verbindungen der Formel (I), worin Q für Q4, R⁹ für Trifluormethyl, X für Methyl, W für C-Y, Y für Methylsulfinyl, V für Wasserstoff und Z für Trifluormethyl steht, und R die in Tabelle 1 angegebenen Bedeutungen hat:

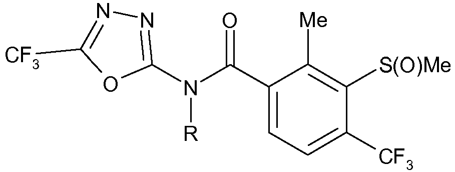

Tabelle 314: Erfindungsgemäße Verbindungen der Formel (I), worin Q für Q4, R⁹ für Methyl, X für Methyl, W für C-Y, Y für Methylsulfonyl, V für Wasserstoff und Z für Trifluormethyl steht, und R die in Tabelle 1 angegebenen Bedeutungen hat:

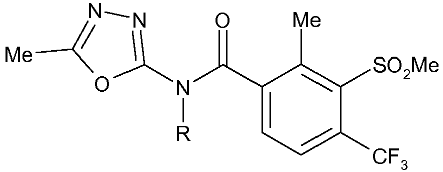

Tabelle 315: Erfindungsgemäße Verbindungen der Formel (I), worin Q für Q4, R⁹ für Ethyl, X für Methyl, W für C-Y, Y für Methylsulfonyl, V für Wasserstoff und Z für Trifluormethyl steht, und R die in Tabelle 1 angegebenen Bedeutungen hat:

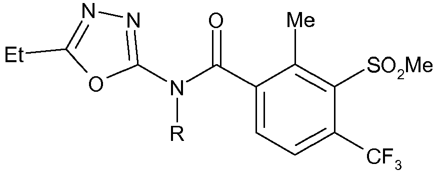

Tabelle 316: Erfindungsgemäße Verbindungen der Formel (I), worin Q für Q4, R⁹ für Methoxymethyl, X für Methyl, W für C-Y, Y für Methylsulfonyl, V für Wasserstoff und Z für Trifluormethyl steht, und R die in Tabelle 1 angegebenen Bedeutungen hat:

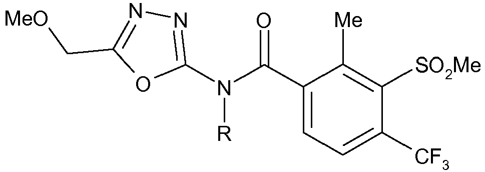

Tabelle 317: Erfindungsgemäße Verbindungen der Formel (I), worin Q für Q4, R⁹ für Ethoxymethyl, X für Methyl, W für C-Y, Y für Methylsulfonyl, V für Wasserstoff und Z für Trifluormethyl steht, und R die in Tabelle 1 angegebenen Bedeutungen hat:

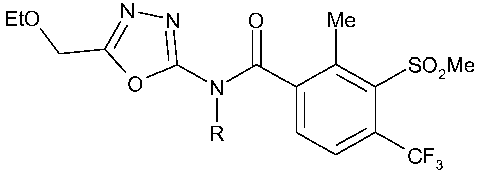

Tabelle 318: Erfindungsgemäße Verbindungen der Formel (I), worin Q für Q4, R⁹ für Trifluormethyl, X für Methyl, W für C-Y, Y für Methylsulfonyl, V für Wasserstoff und Z für Trifluormethyl steht, und R die in Tabelle 1 angegebenen Bedeutungen hat:

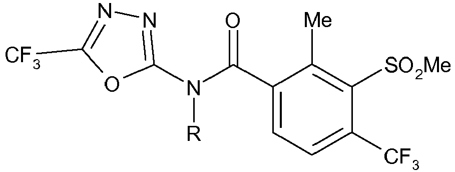

Tabelle 319: Erfindungsgemäße Verbindungen der Formel (I), worin Q für Q4, R⁹ für 2,2,2-Trifluorethyl, X für Methyl, W für C-Y, Y für Methylsulfonyl, V für Wasserstoff und Z für Trifluormethyl steht, und R die in Tabelle 1 angegebenen Bedeutungen hat:

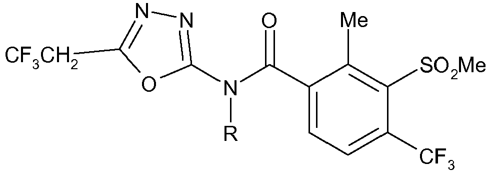

Tabelle 320: Erfindungsgemäße Verbindungen der Formel (I), worin Q für Q4, R⁹ für Methyl, X für Methyl, W für C-Y, Y für Methylsulfanyl, V für Wasserstoff und Z für Methylsulfonyl steht, und R die in Tabelle 1 angegebenen Bedeutungen hat:

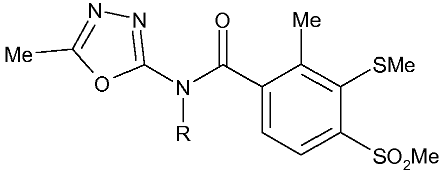

Tabelle 321: Erfindungsgemäße Verbindungen der Formel (I), worin Q für Q4, R⁹ für Ethyl, X für Methyl, W für C-Y, Y für Methylsulfanyl, V für Wasserstoff und Z für Methylsulfonyl steht, und R die in Tabelle 1 angegebenen Bedeutungen hat:

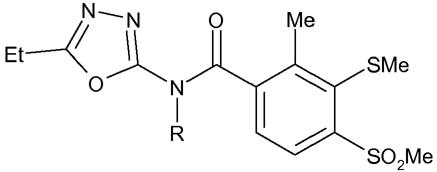

Tabelle 322: Erfindungsgemäße Verbindungen der Formel (I), worin Q für Q4, R⁹ für Methoxymethyl, X für Methyl, W für C-Y, Y für Methylsulfanyl, V für Wasserstoff und Z für Methylsulfonyl steht, und R die in Tabelle 1 angegebenen Bedeutungen hat:

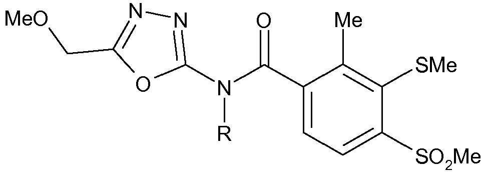

Tabelle 323: Erfindungsgemäße Verbindungen der Formel (I), worin Q für Q4, R⁹ für Methyl, X für Methyl, W für C-Y, Y für Methylsulfinyl, V für Wasserstoff und Z für Methylsulfonyl steht, und R die in Tabelle 1 angegebenen Bedeutungen hat:

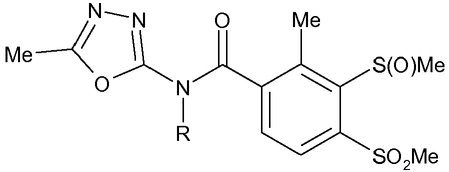

Tabelle 324: Erfindungsgemäße Verbindungen der Formel (I), worin Q für Q4, R⁹ für Ethyl, X für Methyl, W für C-Y, Y für Methylsulfinyl, V für Wasserstoff und Z für Methylsulfonyl steht, und R die in Tabelle 1 angegebenen Bedeutungen hat:

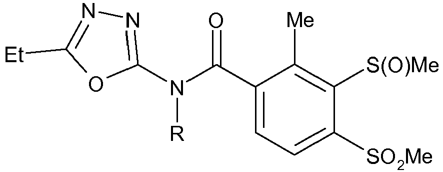

Tabelle 325: Erfindungsgemäße Verbindungen der Formel (I), worin Q für Q4, R⁹ für Methoxymethyl, X für Methyl, W für C-Y, Y für Methylsulfinyl, V für Wasserstoff und Z für Methylsulfonyl steht, und R die in Tabelle 1 angegebenen Bedeutungen hat:

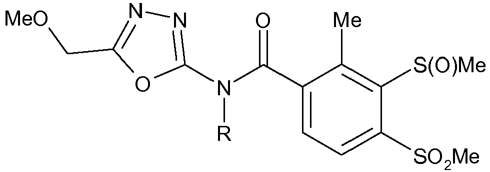

Tabelle 326: Erfindungsgemäße Verbindungen der Formel (I), worin Q für Q4, R⁹ für Methyl, X für Methyl, W für C-Y, Y für Methylsulfonyl, V für Wasserstoff und Z für Methylsulfonyl steht, und R die in Tabelle 1 angegebenen Bedeutungen hat:

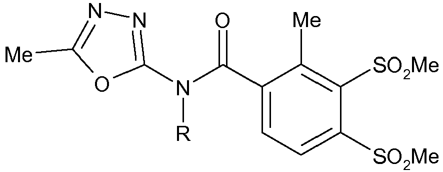

Tabelle 327: Erfindungsgemäße Verbindungen der Formel (I), worin Q für Q4, R⁹ für Ethyl, X für Methyl, W für C-Y, Y für Methylsulfonyl, V für Wasserstoff und Z für Methylsulfonyl steht, und R die in Tabelle 1 angegebenen Bedeutungen hat:

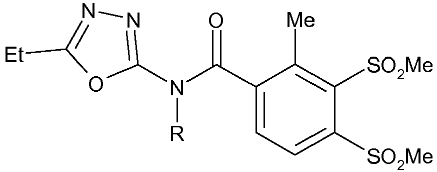

Tabelle 328: Erfindungsgemäße Verbindungen der Formel (I), worin Q für Q4, R⁹ für Methoxymethyl, X für Methyl, W für C-Y, Y für Methylsulfonyl, V für Wasserstoff und Z für Methylsulfonyl steht, und R die in Tabelle 1 angegebenen Bedeutungen hat:

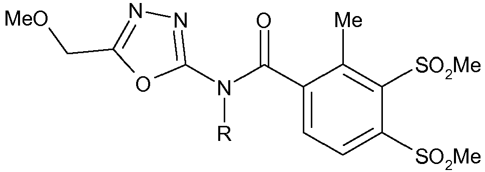

Tabelle 329: Erfindungsgemäße Verbindungen der Formel (I), worin Q für Q4, R⁹ für Methyl, X für Methoxy, W für C-Y, Y für Methylsulfanyl, V für Wasserstoff und Z für Trifluormethyl steht, und R die in Tabelle 1 angegebenen Bedeutungen hat:

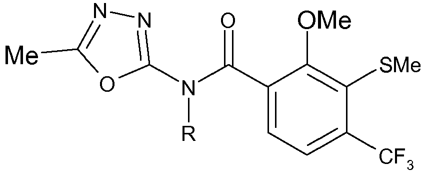

Tabelle 330: Erfindungsgemäße Verbindungen der Formel (I), worin Q für Q4, R⁹ für Methyl, X für Methoxy, W für C-Y, Y für Methylsulfinyl, V für Wasserstoff und Z für Trifluormethyl steht, und R die in Tabelle 1 angegebenen Bedeutungen hat:

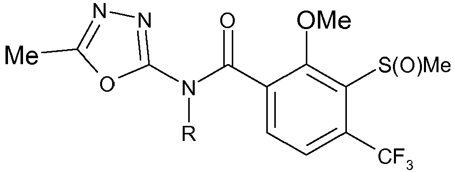

Tabelle 331: Erfindungsgemäße Verbindungen der Formel (I), worin Q für Q4, R⁹ für Methyl, X für Methoxy, W für C-Y, Y für Methylsulfonyl, V für Wasserstoff und Z für Trifluormethyl steht, und R die in Tabelle 1 angegebenen Bedeutungen hat:

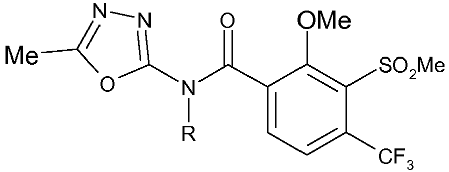

Tabelle 332: Erfindungsgemäße Verbindungen der Formel (I), worin Q für Q4, R⁹ für Methyl, X für Methoxy, W für C-Y, Y für Methylsulfanyl, V für Wasserstoff und Z für Methylsulfonyl steht, und R die in Tabelle 1 angegebenen Bedeutungen hat:

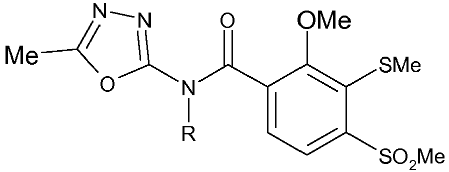

Tabelle 333: Erfindungsgemäße Verbindungen der Formel (I), worin Q für Q4, R⁹ für Methyl, X für Methoxy, W für C-Y, Y für Methylsulfinyl, V für Wasserstoff und Z für Methylsulfonyl steht, und R die in Tabelle 1 angegebenen Bedeutungen hat:

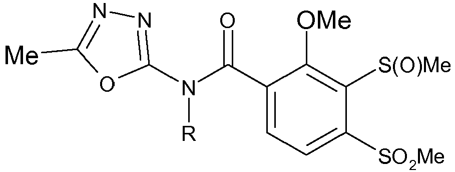

Tabelle 334: Erfindungsgemäße Verbindungen der Formel (I), worin Q für Q4, R⁹ für Methyl, X für Methoxy, W für C-Y, Y für Methylsulfonyl, V für Wasserstoff und Z für Methylsulfonyl steht, und R die in Tabelle 1 angegebenen Bedeutungen hat:

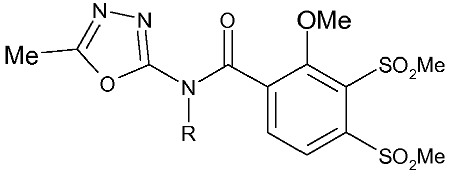

Tabelle 335: Erfindungsgemäße Verbindungen der Formel (I), worin Q für Q4, R⁹ für Methyl, X für Methoxymethyl, W für C-Y, Y für Methylsulfanyl, V für Wasserstoff und Z für Methylsulfonyl steht, und R die in Tabelle 1 angegebenen Bedeutungen hat:

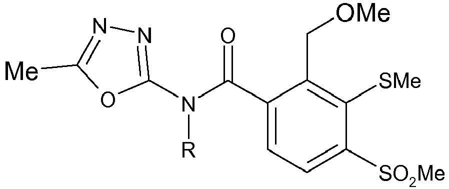

Tabelle 336: Erfindungsgemäße Verbindungen der Formel (I), worin Q für Q4, R⁹ für Methyl, X für Methoxymethyl, W für C-Y, Y für Methylsulfinyl, V für Wasserstoff und Z für Methylsulfonyl steht, und R die in Tabelle 1 angegebenen Bedeutungen hat:

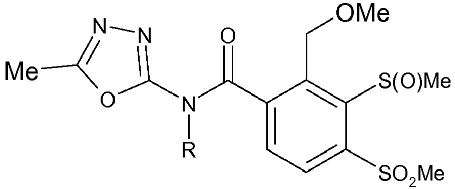

Tabelle 337: Erfindungsgemäße Verbindungen der Formel (I), worin Q für Q4, R⁹ für Methyl, X für Methoxymethyl, W für C-Y, Y für Methylsulfonyl, V für Wasserstoff und Z für Methylsulfonyl steht, und R die in Tabelle 1 angegebenen Bedeutungen hat:

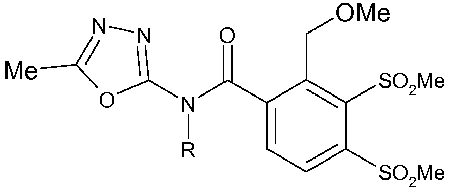

Ganz besonders bevorzugt sind die in denTabelle A, B, C und D angebenenen Verbindungen der allgemeinen Formel (I), die analog der hier genannten Methoden erhältlich sind:

**Tabelle A: Erfindungsgemäße Verbindungen der Formel (I), worin Q für Q1 steht, V für Wasserstoff steht, und R, R⁶, X, W, Z und V die in Tabelle A angegebenen Bedeutungen haben**

| | | | | | | |
|---|---|---|---|---|---|---|
| 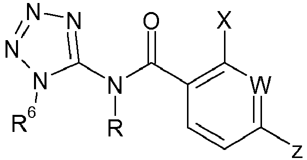 | | | | | | |

| Nr. | R | R⁶ | X | W | Z | Physikalische Daten (¹H-NMR, DMSO-d₆, 400 MHz) |
|---|---|---|---|---|---|---|
| A-1 | CH₂ OEt | Me | Me | C-SO₂Me | CF₃ | 7.94; 7.78; 7.67 und 7.42; (4d, 2 H); 5.33 und 4.90 (2sb, 2H), 4.08 und 4.00 (2s, 3H), 3.86-3.72 und 3.41-3.16 (2m, 5H), 2.88 und 2.85 (2s, 3H), 1.31-1.02 (m, 3H) |
| A-2 | Et | Me | Me | C-SO₂Me | CF₃ | 7.98; 7.69; 7.67 und 7.37; (4d, 2 H); 4.11-3.58 (m, 5H), 3.28 und 3.3,18 (2s, 3H), 3.83 (s, 3H), 1.32 und 1.12 (2t, 3H) |
| A-3 | Me | Me | Me | C-SO₂Me | CF₃ | 7.98, 7.69, 7.38 (3bd,2H), 4.07, 3.91 (2bs,3H), 3.51, 3.37 (2bs,3H), 3.28, 3.20 (2bs,3H), 2.84 (s,3H) |
| A-4 | Bn | Me | Me | C-SO₂Me | CF₃ | 8.23, 8.05, 7.80, 7.76 (4d,2H), 7.41-7.11 (m,5H), 5.12, 4.83 (2bs,2H), 4.02, 3.96 (2s,3H), 3.34 (s,3H), 2.68, 2.52 (2s,3H) |
| A-5 | Me | Bn | Me | C-SO₂Me | CF₃ | 8.06, 7.89, 7.61, 7.18 (4d,2H), 7.43-7.29 (m,5H), 5.73, 5.66 (2bs,2H), 3.46, 3.42 (2s,3H), 3.34, 3.13 (2s,3H), 2.70, 2.65 (2s,3H) |
| A-6 | Prg | Me | Me | C-SOMe | CF₃ | 7.79-7.25; (m, 2 H); 5.31 (bs, 2H), 3.95 (s, 3H), 2.96 (2s, 6H) |
| A-7 | CH₂C O₂Me | Me | Me | C-SOMe | CF₃ | 7.72; 7.59; 7.46 und 7.31; (4d, 2 H); 5.02-4.93 und 4.60-4.31 (2m, 2H), 4.19 und 3.69 (2s, 3H), 3.82 (s, 3H), 3.01 (s, 3H), 2.97 (s, 3H) 4.19 und 3.69 (2s, 3H), 3.82 (s, 3H), 3.01 (s, 3H), 2.97 (s, 3H) |
| A-8 | CH₂C O₂Me | Me | Me | C-SO₂Me | CF₃ | 7.94; 7.72; 7.68 und 7.47; (4d, 2 H); 4.80-4.33 (m, 2H), 4.19; 3.91; 3.84 und 3.69 (4s, 6H), 3.38 und 3.33 (2s, 3H), 2.90 und 2.82 (2s, 3H) |
| A-9 | Allyl | Me | Me | C-SO₂Me | CF₃ | 7.95; 7.69; 7.66 und 7.40; (4d, 2 H); 5.99-5.87 und 5.79-5.65 (2m, 1H), 5.33; 5.32; 5.19 und 5.01 (4d, 2H), 4.61 -4.13 (m, 2H), 4.05 und 3.85 (2s, 3H), 3.28 und 3.19 (2s, 3H), 2.91 und 2.85 (2s, 3H), |
| A-10 | Prg | Prg | Cl | C-Cl | SO₂M | 8.02; (d, 1H); 7.67 (d, 1H), 5.60 (bs, 2H), 4.86 (bs, 2H), 3.52 (b, 1H), 3.43 (s, 3H), 3.34 (t, 1H) |
| A-11 | Me | Me | Me | C-SO₂Me | SO₂M | 8.29; 8.12; 8.02; 7.75 (4db, 2 H); 3.67-3.54 (m, 6H); 3.46; 3.25 (2s, 3H); 2.70; 2.64 (2s, 3H), 2.47; 2.42 (2s, 3H) |
| A-12 | Allyl | Me | Cl | C-(4,5-dihydro-5-acetonitril-1,2-oxazol)-4yl | SO₂Et | 8.23; 8.15; 7.90 und 7.82; (4d, 2 H); 5.88 (m, 1H), 5.42 - 4.98 (m, 3H), 4.55 (bs, 1H), 4.29 (d, 1H), 4.10 and 4.04 (2s, 3H), 3.67 - 2.98 (m, 7H), 1.18 and 1.04 (2t, 3H) |
| A-13 | Me | CO₂Me | Cl | C-OH | SO₂M | 9.39 (bs, 1H), 7.80 (d, 1H), 7.16 (d, 1H), 4.08 (s, 3H), 3.21 (s, 3H). |
| A-14 | Me | CO₂Me | SO₂M | C-H | CF₃ | 8.40 (s, 1H), 8.31 (d, 1H), 8.15 (d, 1H), 4.14 (s, 3H), 3.67 (s, 3H); 3.38 (s, 3H). |
| A-15 | Me | CO₂Et | SO₂M | C-H | CF₃ | ¹H-NMR (400 MHz; DMSO-d₆): 8.40 (s, 1H), 8.32 (d, 1H), 8.16 (d, 1H), 4.13 (s, 3H), 4.10 (q, 2H);3.38 (s, 3H), 0.93 (t, 3H). |
| A-16 | Me | CO₂-i-Pr | SO₂M | C-H | CF₃ | 8.39 (s, 1H), 8.32 (d, 1H), 8.18 (d, 1H), 4.81 (m, 1H), 4.12 (s, 1H), 3.38 (s, 3H); 0.95 (d, 6H) |
| A-17 | Me | CO₂CH₂CHM e₂ | SO₂M | C-H | CF₃ | 8.40 (s, 1H), 8.31 (d, 1H), 8.19 (d, 1H), 4.14 (s, 3H), 3.88 (d, 2H), 3.38 (s, 3H); 1.62 (m, 1H), 0.62 (d, 6H) |
| A-18 | Me | CO₂CH₂-t-Bu | SO₂M | C-H | CF₃ | 8.40 (s, 1H), 8.32 (d, 1H), 8.22 (d, 1H), 4.15 (s, 3H), 3.38 (s, 3H); 0.63 (s, 9H) |

**Tabelle C: Erfindungsgemäße Verbindungen der Formel (I), worin Q für Q1 steht, V für Wasserstoff steht. und R. R⁸. X. W und Z die in Tabelle C angegebenen Bedeutungen haben**

| | | | | | | |
|---|---|---|---|---|---|---|
| 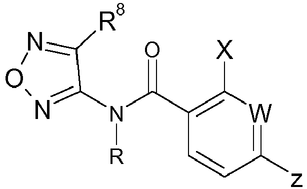 | | | | | | |

| Nr. | R | R⁸ | X | W | Z | Physikalische Daten (¹H-NMR, DMSO-d₆, 400 MHz) |
|---|---|---|---|---|---|---|
| C-1 | Allyl | Me | Me | C-SO₂Me | CF₃ | 8.10-8.01 (b, 1H); 7.83; 7.74 (2d, 1H); 5.98-5.75 (m, 1H); 5.38; 5.26 (2d, 1H); 5.14; 5.02 (2d, 1H); 4.19 (d, 2H); 3.46; 3.30 (2s; 3H), 2.89; 2.72 (2s; 3H); 2.43 (s, 3H) |
| C-2 | CH₂CH₂CN | Me | Me | C-SO₂Me | CF₃ | 8.12; 8.11; 7.87; 7.72 (4d, 2 H); 4.01-3.82 (m, 2H), 3.49-3.37 (m, 3H); 2.99-2.79 (m, 2H), 2.72 (s, 3H), 2.42; 2.41 (2s, 3H) |
| C-4 | CH₂CO₂Me | Me | Me | C-SO₂Me | CF₃ | 8.85; 7.68; (2d, 2 H); 4.74 (bs, 2H), 3.76; 3.46 (2s, 3H); 3.42; 3.39 (2s, 3H), 2.74; 2.72 (2s, 3H), 2.42; 2.38 (2s, 3H) |
| C-5 | Me | Me | Me | C-SO₂Me | SO₂M | 8.29; 8.12; 8.02; 7.75 (4db, 2 H); 3.67-3.54 (m, 6H); 3.46; 3.25 (2s, 3H); 2.70; 2.64 (2s, 3H), 2.47; 2.42 (2s, 3H) |
| C-6 | CH₂OEt | Me | Me | C-SO₂Me | CF₃ | 8.07 (bs, 1H), 7.85 and 7.77 (2bs, 1H), 5.35 and 4.89 (2bs, 2H), 3.70and 3.33 (2 bs, 3H), 3.42 (bq, 2H), 2.71 (s, 3H), 2.42 (s, 3H), 2.66 and 2.55 (2s, 3H), 2.36 (s, 3H) |
| C-7 | Bn | Me | Me | C-SO₂Me | CF₃ | 8.14,8.02,7.82 and 7.76 (4d, 2H), 7.40 - 7.08 (m, 5H), 5.25 and 4.82 (2bs, 2H), 3.32 (s, 3H), 1.17 and 1.01 (2t, 3H). |
| C-8 | Me | Me | Me | C-SO₂Me | CF₃ | BCAK20931-1-1 |
| | | | | | | ¹H-NMR (400 MHz; DMSO-d₆): 7.96, and 7.36 (2bs, 1H), 7.68 (bs, 1H), 3.48 -3.19 (4s, 6H), 2.82 (bs, 3H), 2.45 and 2.34 (2bs, 3H) |
| C-9 | Me | Et | Me | C-SO₂Me | CF₃ | 7.95 and 7.35 (bd, 1H), 7.68 (bs, 1H), 3.48, 3.30, 3.27 and 3.19 (4bs, 6H), 2.82 - 2.66 (m, 5H), 1.38 (t, 3H). |
| C-10 | Me | Cl | Cl | C-S(O)Me | CF₃ | 7.84 - 7.52 (m, 2H), 3.63 - 3.32 (m, 3H), 3.24 - 2.96 (m, 3H). |
| C-11 | Prg | Cl | Cl | C-SMe | CF₃ | 7.70 (bs, 1H), 7.50 (bs, 1H), 4.80 (bs, 2H), 2.45 - 2.33 (m, 4H). |
| C-12 | Prg | Me | Me | C-SO₂Me | SO₂M | 8.41,8.16,7.81 and 7.45 (4bs, 2H), 4.73 and 4.37 (2bs, 2H), 3.57 - 3.42 (m, 6H), 2.87 - 2.77 (bs, 3H), 2.44 - 2.34 (m, 4H) |
| C-13 | Me | Cl | Cl | C-SMe | CF₃ | 7.68 and 7.49 (2bs, 2H), 3.50 (bs, 3H), 2.33 (bs, 3H). |

**Tabelle D: Erfindungsgemäße Verbindung der Formel (I), worin Q für Q4 steht, V für Wasserstoff steht, und R, R⁹, X, W und Z die in Tabelle D angegebenen Bedeutungen haben**

| | | | | | | |
|---|---|---|---|---|---|---|
| 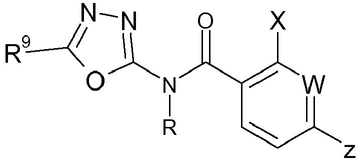 | | | | | | |

| Nr. | R | R⁹ | X | W | Z | Physikalische Daten (¹H-NMR, CDCl₃, 400 MHz) |
|---|---|---|---|---|---|---|
| D-1 | Me | Me | Me | C-SO₂Me | CF₃ | 7.98 (d, 1H), 7.49 (d, 1H), 3.57 (bs, 3H), 3.23 (s, 3H), 2.92 (s, 3H), 2.37 (s, 3H). |

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I) und/oder deren Salze,
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Dabei zeigen beispielsweise die Verbindungen Nr. A-2, A-3, A-4, A-5, A-6, C-1, C-3 und C-5 bei einer Aufwandmenge von 320 g/ha jeweils eine mindestens 80 %-ige Wirkung gegen Stellaria media und Veronica persica.

### 2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Dabei zeigen beispielsweise die Verbindungen Nr. A-2, A-3, A-4, A-5, A-6, A-7, C-1, C-3 und C-5 bei einer Aufwandmenge von 80 g/ha jeweils eine mindestens 80 %-ige Wirkung gegen Stellaria media und Amaranthus retroflexus.

## Patentansprüche

1. N-(1,2,5-Oxadiazol-3-yl)-, N-(1,3,4-Oxadiazol-2-yl)-, N-(Tetrazol-5-yl)- und N-(Triazol-5-yl)-arylcarbonsäureamide der Formel (I) oder deren Salze 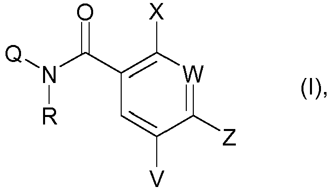 worin
R bedeutet (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₃-C₆)-Halogenalkinyl, (C₃-C₆)-Cycloalkyl, (C₃-C₆)-Halogencycloalkyl, (C₃-C₆)-Cycloalkyl-(C₁-C₆)-alkyl, (C₃-C₆)-Halogencycloalkyl-(C₁-C₆)-alkyl, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-COOR¹, (C₁-C₆)-Alkyl-C(O)R¹, (C₁-C₆)-Alkyl-CN, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R, OR¹, COOR¹, CON(R¹)₂, N(R¹)₂, NR¹COOR¹ NR¹CON(R¹)₂ oder
jeweils durch s Reste aus der Gruppe Methyl, Ethyl, Methoxy, Nitro, Trifluormethyl und Halogen substituiertes Benzyl,
W bedeutet N oder CY,
X und Z bedeuten unabhängig voneinander jeweils Wasserstoff, Nitro, Halogen, Cyano, Formyl, Rhodano, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₃-C₆)-Halogenalkinyl, (C₃-C₆)-Cycloalkyl, (C₃-C₆)-Halogencycloalkyl, (C₃-C₆)-Cycloalkyl-(C₁-C₆)-alkyl, (C₃-C₆)-Halogencycloalkyl-(C₁-C₆)-alkyl, COR¹, OR¹, OCOR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-COOR¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², NR₁R₂, P(O)(OR⁵)₂, oder
jeweils durch s Reste aus der Gruppe Methyl, Ethyl, Methoxy, Nitro, Trifluormethyl und Halogen substituiertes Heteroaryl, Heterocyclyl oder Phenyl,
Y bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹,OCOOR¹, NR¹COOR¹, C(O)N(R¹)₂, NR¹C(O)N(R¹)₂, OC(O)N(R¹)₂, C(O)N(R¹)OR¹, NR¹SO₂R², NR¹COR¹, OR¹, OSO₂R², S(O)ₙR², SO₂OR¹, SO₂N(R¹)₂ (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-CN, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², N(R¹)₂, P(O)(OR⁵)₂, CH₂P(O)(OR⁵)₂, CH=NOR¹, (C₁-C₆)-Alkyl-CH=NOR¹, (C₁-C₆)-Alkyl-O-N=C(R¹)₂, (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die 6 letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
oder
Y und Z bilden gemeinsam mit den beiden Atomen, an die sie gebunden sind, einen 5-, 6- oder 7-gliedrigen, ungesättigten, teilgesättigten oder gesättigten Ring, der neben Kohlenstoffatomen jeweils s Stickstoffatome, n Sauerstoffatome, n Schwefelatome und n Elemente S(O), S(O)₂, C=N-R¹⁷, C(OR¹⁷)₂, C[-O-(CH₂)₂-O-] oder C(O) als Ringglieder umfasst,
dessen Kohlenstoffatome durch s Reste aus der Gruppe bestehend aus Halogen, Cyano, (C₁-C₆)-Alkyl, (C₂-C₁₀)-Alkenyl, (C₂-C₁₀)-Alkinyl, (C₁-C₆)-Haloalkyl, (C₁-C₆)-Alkoxy, Phenoxy, Halogen-(C₁-C₆)-alkoxy, (C₃-C₈)-Cycloalkyl, (C₂-C₈)-Alkoxyalkyl und Phenyl substituiert sind,
dessen Stickstoffatome durch n Reste aus der Gruppe bestehend aus (C₁-C₆)-Alkyl und Phenyl substituiert sind,
und worin die vorstehend genannten Phenylreste durch s Reste aus der Gruppe bestehend aus Cyano, Nitro, Halogen, (C₁-C₆)-Alkyl, (C₁-C₆)-Haloalkyl und (C₁-C₆)-Alkoxy substituiert sind,
V bedeutet Wasserstoff, Nitro, Halogen, Cyano, (C₁-C₄)-Alkyl, (C₁-C₄)-Halogenalkyl, OR¹, S(O)ₙR²,
R¹ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocycl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl wobei die 21 letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
R² bedeutet (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl wobei die 21 letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SOR⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
R³ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R⁴ bedeutet (C₁-C₆)-Alkyl, (C₂-C₆)-Alkenyl oder (C₂-C₆)-Alkinyl, (C₃-C₆)-Cycloalkyl oder (C₃-C₆)-Cycloalkyl-(C₁-C₆)-alkyl,
R⁵ bedeutet (C₁-C₄)-Alkyl,
n bedeutet 0, 1 oder 2,
s bedeutet 0, 1, 2 oder 3,
Q bedeutet einen Rest Q1, Q2, Q3 oder Q4 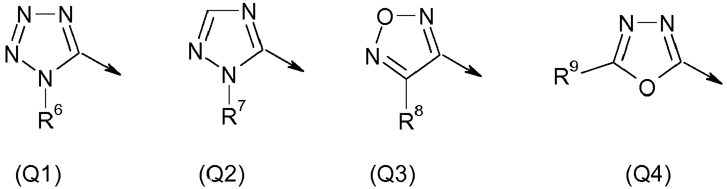
R⁶ bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl,
wobei diese 6 vorstehend genannten Reste jeweils durch s Reste aus der Gruppe bestehend aus Nitro, Cyano, SiR¹²₃, PO(OR¹²)₃, S(O)ₙ-(C₁-C₆)-Alkyl, (C₁-C₆)-Alkoxy, Halogen-(C₁-C₆)-alkoxy, N(R¹⁰)₂, COR¹⁰, COOR¹⁰, OCOR¹⁰, OCO₂R¹⁰, NR¹⁰COR¹⁰, NR¹⁰SO₂R¹¹, (C₃-C₆)-Cycloalkyl, Heteroaryl, Heterocyclyl, Phenyl, D-Heteroaryl, D-Heterocyclyl, D-Phenyl oder D-Benzyl substituiert sind, und wobei die 7 letztgenannten Reste durch s Reste aus der Gruppe Methyl, Ethyl, Methoxy, Trifluormethyl und Halogen substituiert sind, und wobei Heterocyclyl n Oxogruppen trägt,
oder
R⁶ bedeutet jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiertes (C₃-C₇)-Cycloalkyl, Heteroaryl, Heterocyclyl oder Phenyl,
R⁷ bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl,
wobei diese 6 vorstehend genannten Reste jeweils durch s Reste aus der Gruppe bestehend aus Nitro, Cyano, SiR¹²₃, PO(OR¹²)₃, S(O)ₙ-(C₁-C₆)-Alkyl, (C₁-C₆)-Alkoxy, Halogen-(C₁-C₆)-alkoxy, N(R¹⁰)₂, COR¹⁰, COOR¹⁰, OCOR¹⁰, OCO₂R¹⁰, NR¹⁰COR¹⁰, NR¹⁰SO₂R¹¹, (C₃-C₆)-Cycloalkyl, Heteroaryl, Heterocyclyl, Phenyl, D-Heteroaryl, D-Heterocyclyl, D-Phenyl und D-Benzyl substituiert sind, und wobei die 7 letztgenannten Reste durch s Reste aus der Gruppe Methyl, Ethyl, Methoxy, Trifluormethyl und Halogen substituiert sind, und wobei Heterocyclyl n Oxogruppen trägt, oder
R⁷ bedeutet jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiertes (C₃-C₇)-Cycloalkyl, Heteroaryl, Heterocyclyl oder Phenyl,
R⁸ bedeutet Wasserstoff, (C₁-C₆)-Alkyl, (C₃-C₇)-Cycloalkyl, Halogen-(C₁-C₆)-alkyl, (C₁-C₆)-Alkoxy, Halogen-(C₁-C₆)-alkoxy, (C₂-C₆)-Alkenyl, (C₂-C₆)-Alkenyloxy, Halogen-(C₂-C₆)-alkenyl, (C₂-C₆)-Alkinyl, (C₂-C₆)-Alkinyloxy, Halogen-(C₂-C₆)-alkinyl, Cyano, Nitro, Methylsulfenyl, Methylsulfinyl, Methylsulfonyl, (C₁-C₆)-Alkylcarbonylamino, (C₁-C₆)-Alkoxycarbonylamino, Benzoylamino, Methoxycarbonyl, Ethoxycarbonyl, Methoxycarbonylmethyl, Ethoxycarbonylmethyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl, oder jeweils durch s Reste aus der Gruppe bestehend aus Methyl, Ethyl, Methoxy, Trifluormethyl und Halogen substituiertes Heteroaryl, Heterocyclyl oder Phenyl;
R⁹ bedeutet Wasserstoff, (C₁-C₆)-Alkyl, R¹³O-(C₁-C₆)-Alkyl, CH₂R¹⁴, (C₃-C₇)-Cycloalkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, OR¹³, NHR¹³, Methoxycarbonyl, Ethoxycarbonyl, Methoxycarbonylmethyl, Ethoxycarbonylmethyl, Methylcarbonyl, Trifluormethylcarbonyl, Dimethylamino, Acetylamino, Methylsulfenyl, Methylsulfinyl, Methylsulfonyl oder jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiertes Heteroaryl, Heterocyclyl, Benzyl oder Phenyl,
R¹⁰ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl oder Phenyl,
R¹¹ bedeutet (C₁-C₆)-Alkyl, (C₂-C₆)-Alkenyl, (C₂-C₆)-Alkinyl oder Phenyl,
R¹² bedeutet (C₁-C₆)-Alkyl,
R¹³ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocycl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR¹⁵-Heteroaryl oder (C₁-C₆)-Alkyl-NR¹⁵-Heterocyclyl, wobei die 21 letztgenannten Reste durch s Reste aus der Gruppe bestehend aus Cyano, Halogen, Nitro, Rhodano, OR¹⁵, S(O)ₙR¹⁶, N(R¹⁵)₂, NR¹⁵OR¹⁵, COR¹⁵, OCOR¹⁵, SCOR¹⁶, NR¹⁵COR¹⁵, NR¹⁵SO₂R¹⁶, CO₂R¹⁵, COSR¹⁶, CON(R¹⁵)₂ und (C₁-C₄)-Alkoxy-(C₂-C₆)-alkoxycarbonyl substituiert sind, und wobei Heterocyclyl n Oxogruppen trägt,
R¹⁴ bedeutet Acetoxy, Acetamido, N-Methylacetamido, Benzoyloxy, Benzamido, N-Methylbenzamido, Methoxycarbonyl, Ethoxycarbonyl, Benzoyl, Methylcarbonyl, Piperidinylcarbonyl, Morpholinylcarbonyl, Trifluormethylcarbonyl, Aminocarbonyl, Methylaminocarbonyl, Dimethylaminocarbonyl, (C₃-C₆)-Alkoxy, (C₃-C₆)-Cycloalkyl oder jeweils durch s Reste aus der Gruppe bestehend aus Methyl, Ethyl, Methoxy, Trifluormethyl und Halogen substituiertes Heteroaryl, Heterocyclyl oder Phenyl;
R¹⁵ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R¹⁶ bedeutet (C₁-C₆)-Alkyl, (C₂-C₆)-Alkenyl oder (C₂-C₆)-Alkinyl,
R¹⁷ bedeutet (C₁-C₆)-Alkyl, Halogen-(C₁-C₆)-alkyl, (C₁-C₆)-Alkoxy und Halogen-(C₁-C₆)-alkoxy,
s bedeutet 0, 1, 2 oder 3,
n bedeutet 0, 1 oder 2,
D bedeutet O, S, oder NR¹¹,
wobei die Verbindungen 2-Methoxy-N-methyl-N-(1 H-tetrazol-5-yl)benzamid, 2-Hydroxy-N-methyl-N-(1 H-tetrazol-5-yl)benzamid, 2-Methoxy-N-methyl-N-(1-methyl-1 H-tetrazol-5-yl)benzamid, N-Methyl-N-(5-phenyl-1,3,4-oxadiazol-2-yl)benzamid und 3-Brom-4-methoxy-N-methyl-N-(5-phenyl-1,3,4-oxadiazol-2-yl)benzamid ausgenommen sind.

2. N-(1,3,4-Oxadiazol-2-yl)-, N-(Tetrazol-5-yl)- und N-(Triazol-5-yl)-arylcarbon-säureamide der Formel (I) oder deren Salze nach Anspruch 1, worin
Q bedeutet einen Rest Q1, Q2 oder Q4 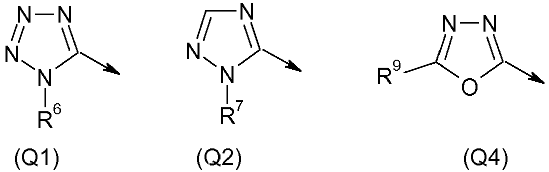 und alle anderen Reste und Indices jeweils wie in Anspruch 1 definiert sind.

3. N-(1,3,4-Oxadiazol-2-yl)- und N-(Tetrazol-5-yl)-arylcarbonsäureamide der Formel (I) oder deren Salze nach Anspruch 1 oder 2, worin
Q bedeutet einen Rest Q1 oder Q4 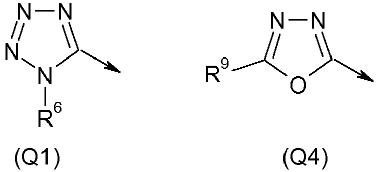 und alle anderen Reste und Indices jeweils wie in Anspruch 1 definiert sind.

4. N-(Tetrazol-5-yl)-arylcarbonsäureamide der Formel (I) nach einem der Ansprüche 1 bis 3, worin
Q bedeutet den Rest Q1 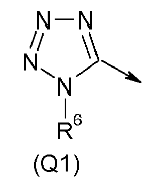 und alle anderen Reste und Indices jeweils wie in Anspruch 1 definiert sind.

5. Herbizide Mittel, **gekennzeichnet durch** einen herbizid wirksamen Gehalt an mindestens einer Verbindung der Formel (I) gemäß einem der Ansprüche 1 bis 4.

6. Herbizide Mittel nach Anspruch 5 in Mischung mit Formulierungshilfsmitteln.

7. Herbizide Mittel nach Anspruch 6 enthaltend einen Safener.

8. Herbizide Mittel nach Anspruch 7 enthaltend ein weiteres Herbizid.

9. Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 4 oder eines herbiziden Mittels nach einem der Ansprüche 5 bis 8 auf die Pflanzen oder auf den Ort des unerwünschten Pflanzenwachstums appliziert.

10. Verwendung von Verbindungen der Formel (I) gemäß einem der Ansprüche 1 bis 4 oder von herbiziden Mitteln nach einem der Ansprüche 5 bis 8 zur Bekämpfung unerwünschter Pflanzen.

11. Verwendung nach Anspruch 10, **dadurch gekennzeichnet, daß** die Verbindungen der Formel (I) zur Bekämpfung unerwünschter Pflanzen in Kulturen von Nutzpflanzen eingesetzt werden.

12. Verwendung nach Anspruch 11, **dadurch gekennzeichnet, daß** die Nutzpflanzen transgene Nutzpflanzen sind.

## Claims

1. N-(1,2,5-Oxadiazol-3-yl)-, N-(1,3,4-oxadiazol-2-yl)-, N-(tetrazol-5-yl)- or N-(triazol-5-yl)arylcarboxamides of the formula (I) or the salts thereof 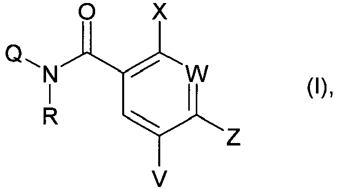 in which
R is (C₁-C₆) -alkyl, (C₁-C₆) -haloalkyl, (C₂-C₆)-alkenyl, (C₂-C₆)-haloalkenyl, (C₂-C₆)-alkynyl, (C₃-C₆)-haloalkynyl, (C₃-C₆)-cycloalkyl, (C₃-C₆)-halocycloalkyl, (C₃-C₆)-cycloalkyl- (C₁-C₆) -alkyl, (C₃-C₆)-halocycloalkyl-(C₁-C₆)-alkyl, (C₁-C₆)-alkyl-S(O)ₙR², (C₁-C₆)-alkyl-OR¹, (C₁-C₆) -alkyl-OCOR¹, (C₁-C₆) -alkyl-OSO₂R², (C₁-C₆) -alkyl-COOR¹, (C₁-C₆)-alkyl-C(O)R¹, (C₁-C₆)-alkyl-CN, (C₁-C₆)-alkyl-SO₂OR¹, (C₁-C₆)-alkyl-CON(R¹)₂, (C₁-C₆)-alkyl-SO₂N(R¹)₂, (C₁-C₆)-alkyl-NR¹COR¹, (C₁-C₆)-alkyl-NR¹SO₂R², OR¹, COOR¹, CON(R¹)₂, N(R¹)₂, NR¹COOR¹ NR¹CON(R¹) or benzyl in each case substituted by s radicals from the group of methyl, ethyl, methoxy, nitro, trifluoromethyl and halogen,
W is N or CY,
X and Z are each independently hydrogen, nitro, halogen, cyano, formyl, thiocyanato, (C₁-C₆)-alkyl, (C₁-C₆)-haloalkyl, (C₂-C₆)-alkenyl, (C₂-C₆)-haloalkenyl, (C₂-C₆)-alkynyl, (C₃-C₆)-haloalkynyl, (C₃-C₆)-cycloalkyl, (C₃-C₆)-halocycloalkyl, (C₃-C₆)-cycloalkyl- (C₁-C₆) -alkyl, (C₃-C₆)-halocycloalkyl-(C₁-C₆)-alkyl, COR¹, OR¹, OCOR¹, OSO₂R², S(O)ₙR², SO₂OR¹, SO₂N(R¹)₂, NR¹SO₂R², NR¹COR¹, (C₁-C₆)-alkyl-S(O)ₙR², (C₁-C₆)-alkyl-OR¹, (C₁-C₆)-alkyl-OCOR¹, (C₁-C₆) -alkyl-OSO₂R², (C₁-C₆) -alkyl-COOR¹, (C₁-C₆) -alkyl-SO₂OR¹, (C₁-C₆)-alkyl-CON(R¹)₂, (C₁-C₆)-alkyl-SO₂N(R¹)₂, (C₁-C₆)-alkyl-NR¹COR¹, (C₁-C₆)-alkyl-NR¹SO₂R², NR₁R₂, P(O)(OR⁵)₂, or
heteroaryl, heterocyclyl or phenyl each substituted by s radicals from the group of methyl, ethyl, methoxy, nitro, trifluoromethyl and halogen,
Y is hydrogen, nitro, halogen, cyano, thiocyanato, (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₂-C₆)-alkynyl, (C₃-C₆)-cycloalkyl, (C₃-C₆)-cycloalkenyl, halo-(C₃-C₆)-cycloalkyl, (C₃-C₆)-cycloalkyl-(C₁-C₆)-alkyl, halo-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, C(O)N(R¹)OR¹, NR¹SO₂R², NR¹COR¹, OR¹, OSO₂R², S(O)ₙR², SO₂OR¹, SO₂N(R¹)₂ (C₁-C₆)-alkyl-S(O)ₙR², (C₁-C₆)-alkyl-OR¹, (C₁-C₆) -alkyl-OCOR¹, (C₁-C₆) -alkyl-OSO₂R², (C₁-C₆) -alkyl-CO₂R¹, (C₁-C₆)-alkyl-CN, (C₁-C₆) -alkyl-SO₂OR¹, (C₁-C₆)-alkyl-CON(R¹)₂, (C₁-C₆)-alkyl-SO₂N(R¹)₂, (C₁-C₆)-alkyl-NR¹COR¹, (C₁-C₆)-alkyl-NR¹SO₂R², N(R¹)₂, P(O)(OR⁵)₂, CH₂P(O)(OR⁵)₂, CH=NOR¹, (C₁-C₆)-alkyl-CH=NOR¹, (C₁-C₆)-alkyl-O-N=C(C¹)₂, (C₁-C₆)-alkylphenyl, (C₁-C₆)-alkylheteroaryl, (C₁-C₆)-alkylheterocyclyl, phenyl, heteroaryl or heterocyclyl, where the 6 latter radicals are each substituted by s radicals from the group consisting of halogen, nitro, cyano, (C₁-C₆)-alkyl, halo-(C₁-C₆)-alkyl, (C₃-C₆)-cycloalkyl, S(O)ₙ-(C₁-C₆)-alkyl, (C₁-C₆)-alkoxy, halo-(C₁-C₆)-alkoxy, (C₁-C₆)-alkoxy-(C₁-C₄)-alkyl and cyanomethyl, and where heterocyclyl bears n oxo groups,
or
Y and Z together with the two atoms to which they are bonded form a 5-, 6- or 7-membered, unsaturated, partly saturated or saturated ring which, as well as carbon atoms, in each case has s nitrogen atoms, n oxygen atoms, n sulfur atoms and n S(O), S(O)₂, C=N-R¹⁷, C(OR¹⁷)₂, C[-O-(CH₂)₂-O-] or C(O) elements as ring members,
wherein the carbon atoms are substituted by s radicals from the group consisting of halogen, cyano, (C₁-C₆)-alkyl, (C₂-C₁₀)-alkenyl, (C₂-C₁₀)-alkynyl, (C₁-C₆)-haloalkyl, (C₁-C₆)-alkoxy, phenoxy, halo- (C₁-C₆)-alkoxy, (C₃-C₈)-cycloalkyl, (C₂-C₈)-alkoxyalkyl and phenyl, wherein the nitrogen atoms are substituted by n radicals from the group consisting of (C₁-C₆)-alkyl and phenyl,
and in which the aforementioned phenyl radicals are substituted by s radicals from the group consisting of cyano, nitro, halogen, (C₁-C₆)-alkyl, (C₁-C₆)-haloalkyl and (C₁-C₆)-alkoxy,
V is hydrogen, nitro, halogen, cyano, (C₁-C₄)-alkyl, (C₁-C₄)-haloalkyl, OR¹, S(O)ₙR²,
R¹ is hydrogen, (C₁-C₆)-alkyl, (C₁-C₆)-haloalkyl, (C₂-C₆)-alkenyl, (C₂-C₆)-haloalkenyl, (C₂-C₆)-alkynyl, (C₂-C₆)-haloalkynyl, (C₃-C₆)-cycloalkyl, (C₃-C₆)-cycloalkenyl, (C₃-C₆)-halocycloalkyl, (C₁-C₆)-alkyl-O-(C₁-C₆)-alkyl, (C₃-C₆)-cycloalkyl-(C₁-C₆)-alkyl, phenyl, phenyl-(C₁-C₆)-alkyl, heteroaryl, (C₁-C₆)-alkylheteroaryl, heterocycl, (C₁-C₆)-alkylheterocyclyl, (C₁-C₆) -alkyl-O-heteroaryl, (C₁-C₆) -alkyl-O-heterocyclyl, (C₁-C₆) -alkyl-NR³-heteroaryl, (C₁-C₆)-alkyl-NR³-heterocyclyl, where the 21 latter radicals are substituted by s radicals from the group consisting of cyano, halogen, nitro, thiocyanato, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ and (C₁-C₄)-alkoxy-(C₂-C₆)-alkoxycarbonyl, and where heterocyclyl bears n oxo groups,
R² is (C₁-C₆)-alkyl, (C₁-C₆) -haloalkyl, (C₂-C₆)-alkenyl, (C₂-C₆)-haloalkenyl, (C₂-C₆)-alkynyl, (C₂-C₆) haloalkynyl, (C₃-C₆)-cycloalkyl, (C₃-C₆)-cycloalkenyl, (C₃-C₆)-halocycloalkyl, (C₁-C₆) -alkyl-O-(C₁-C₆) -alkyl, (C₃-C₆)-cycloalkyl-(C₁-C₆)-alkyl, phenyl, phenyl-(C₁-C₆)-alkyl, heteroaryl, (C₁-C₆)-alkylheteroaryl, heterocyclyl, (C₁-C₆)-alkylheterocyclyl, (C₁-C₆) -alkyl-0-heteroaryl, (C₁-C₆)-alkyl-O-heterocyclyl, (C₁-C₆)-alkyl-NR³-heteroaryl, (C₁-C₆) -alkyl-NR³-heterocyclyl, where the 21 latter radicals are substituted by s radicals from the group consisting of cyano, halogen, nitro, thiocyanato, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ and (C₁-C₄)-alkoxy-(C₂-C₆)-alkoxycarbonyl, and where heterocyclyl bears n oxo groups,
R³ is hydrogen, (C₁-C₆)-alkyl, (C₂-C₆)-alkenyl, (C₂-C₆)-alkynyl, (C₃-C₆)-cycloalkyl or (C₃-C₆) -cycloalkyl-(C₁-C₆)-alkyl,
R⁴ is (C₁-C₆)-alkyl, (C₂-C₆)-alkenyl or (C₂-C₆)-alkynyl, (C₃-C₆)-cycloalkyl or (C₃-C₆)-cycloalkyl- (C₁-C₆)-alkyl,
R⁵ is (C₁-C₄)-alkyl,
n is 0, 1 or 2;
s is 0, 1, 2 or 3,
Q is a Q1, Q2, Q3 or Q4 radical, 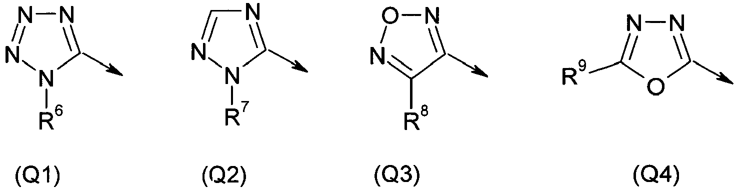
R⁶ is (C₁-C₆)-alkyl, halo- (C₁-C₆) -alkyl, (C₂-C₆)-alkenyl, halo-(C₂-C₆)-alkenyl, (C₂-C₆)-alkynyl, halo-(C₂-C₆)-alkynyl, where these 6 aforementioned radicals are each substituted by s radicals from the group consisting of nitro, cyano, SiR¹²₃, PO(OR¹²)₃, S(O)ₙ-(C₁-C₆)-alkyl, (C₁-C₆)-alkoxy, halo-(C₁-C₆)-alkoxy, N(R¹⁰)₂, COR¹⁰, COOR¹⁰, OCOR¹⁰, OCO₂R¹⁰, NR¹⁰COR¹⁰, NR¹⁰SO₂R¹¹, (C₃-C₆-cycloalkyl, heteroaryl, heterocyclyl, phenyl, D-heteroaryl, D-heterocyclyl, D-phenyl or D-benzyl, and where the 7 latter radicals are substituted by s radicals from the group of methyl, ethyl, methoxy, trifluoromethyl and halogen, and where heterocyclyl bears n oxo groups, or
R⁶ is (C₃-C₇)-cycloalkyl, heteroaryl, heterocyclyl or phenyl, each substituted by s radicals from the group consisting of halogen, nitro, cyano, (C₁-C₆)-alkyl, halo-(C₁-C₆) -alkyl, (C₃-C₆)-cycloalkyl, S(O)ₙ-(C₁-C₆)-alkyl, (C₁-C₆)-alkoxy, halo-(C₁-C₆) -alkoxy and (C₁-C₆)-alkoxy-(C₁-C₄)-alkyl,
R⁷ is (C₁-C₆)-alkyl, halo-(C₁-C₆) -alkyl, (C₂-C₆)-alkenyl, halo-(C₂-C₆)-alkenyl, (C₂-C₆)-alkynyl, halo-(C₂-C₆)-alkynyl,
where these 6 aforementioned radicals are each substituted by s radicals from the group consisting of nitro, cyano, SiR¹²₃, PO(OR¹²)₃, S(O)ₙ-(C₁-C₆) -alkyl, (C₁-C₆)-alkoxy, halo-(C₁-C₆)-alkoxy, N(R¹⁰)₂, COR¹⁰, COOR¹⁰, OCOR¹⁰, OCO₂R¹⁰, NR¹⁰COR¹⁰, NR¹⁰SO₂R¹¹, (C₃-C₆)-cycloalkyl, heteroaryl, heterocyclyl, phenyl, D-heteroaryl, D-heterocyclyl, D-phenyl and D-benzyl, and where the 7 latter radicals are substituted by s radicals from the group of methyl, ethyl, methoxy, trifluoromethyl and halogen, and where heterocyclyl bears n oxo groups,
R⁷ is (C₃-C₇)-cycloalkyl, heteroaryl, heterocyclyl or phenyl, each substituted by s radicals from the group consisting of halogen, nitro, cyano, (C₁-C₆)-alkyl, halo-(C₁-C₆)-alkyl, (C₃-C₆)-cycloalkyl, S(O)ₙ-(C₁-C₆)-alkyl, (C₁-C₆)-alkoxy, halo-(C₁-C₆) -alkoxy and (C₁-C₆)-alkoxy-(C₁-C₄)-alkyl,
R⁸ is hydrogen, (C₁-C₆)-alkyl, (C₃-C₇) -cycloalkyl, halo-(C₁-C₆)-alkyl, (C₁-C₆)-alkoxy, halo-(C₁-C₆) -alkoxy, (C₂-C₆)-alkenyl, (C₂-C₆)-alkenyloxy, halo-(C₂-C₆)-alkenyl, (C₂-C₆)-alkynyl, (C₂-C₆)-alkynyloxy, halo-(C₂-C₆)-alkynyl, cyano, nitro, methylsulfenyl, methylsulfinyl, methylsulfonyl, (C₁-C₆)-alkylcarbonylamino, (C₁-C₆)-alkoxycarbonylamino, benzoylamino, methoxycarbonyl, ethoxycarbonyl, methoxycarbonylmethyl, ethoxycarbonylmethyl, benzoyl, methylcarbonyl, piperidinylcarbonyl, trifluoromethylcarbonyl, halogen, amino, aminocarbonyl, methylaminocarbonyl, dimethylaminocarbonyl, methoxymethyl, or heteroaryl, heterocyclyl or phenyl, each substituted by s radicals from the group consisting of methyl, ethyl, methoxy, trifluoromethyl and halogen;
R⁹ is hydrogen, (C₁-C₆)-alkyl, R¹³O-(C₁-C₆) -alkyl, CH₂R¹⁴, (C₃-C₇)-cycloalkyl, halo-(C₁-C₆) -alkyl, (C₂-C₆)-alkenyl, halo-(C₂-C₆)-alkenyl, (C₂-C₆)-alkynyl, halo-(C₂-C₆)-alkynyl, OR¹³, NHR¹³, methoxycarbonyl, ethoxycarbonyl, methoxycarbonylmethyl, ethoxycarbonylmethyl, methylcarbonyl, trifluoromethylcarbonyl, dimethylamino, acetylamino, methylsulfenyl, methylsulfinyl, methylsulfonyl or heteroaryl, heterocyclyl, benzyl or phenyl, each substituted by s radicals from the group consisting of halogen, nitro, cyano, (C₁-C₆)-alkyl, halo-(C₁-C₆)-alkyl, (C₃-C₆)-cycloalkyl, S(O)ₙ-(C₁-C₆) -alkyl, (C₁-C₆) alkoxy, halo-(C₁-C₆)-alkoxy and (C₁-C₆)-alkoxy-(C₁-C₄)-alkyl,
R¹⁰ is hydrogen, (C₁-C₆)-alkyl, (C₂-C₆)-alkenyl, (C₂-C₆)-alkynyl, (C₃-C₆)-cycloalkyl or (C₃-C₆)-cycloalkyl-(C₁-C₆)-alkyl or phenyl,
R¹¹ is (C₁-C₆)-alkyl, (C₂-C₆)-alkenyl, (C₂-C₆)-alkynyl or phenyl,
R¹² is (C₁-C₆)-alkyl,
R¹³ is hydrogen, (C₁-C₆)-alkyl, (C₁-C₆) -haloalkyl, (C₂-C₆)-alkenyl, (C₂-C₆)-haloalkenyl, (C₂-C₆)-alkynyl, (C₂-C₆)-haloalkynyl, (C₃-C₆)-cycloalkyl, (C₃-C₆)-cycloalkenyl, (C₃-C₆)-halocycloalkyl, (C₁-C₆)-alkyl-O-(C₁-C₆)-alkyl, (C₃-C₆)-cycloalkyl-(C₁-C₆)-alkyl, phenyl, phenyl-(C₁-C₆)-alkyl, heteroaryl, (C₁-C₆)-alkylheteroaryl, heterocyclyl, (C₁-C₆)-alkylheterocyclyl, (C₁-C₆)-alkyl-O-heteroaryl, (C₁-C₆)-alkyl-O-heterocyclyl, (C₁-C₆)-alkyl-NR¹⁵-heteroaryl or (C₁-C₆)-alkyl-NR¹⁵-heterocyclyl, where the 21 latter radicals are substituted by s radicals from the group consisting of cyano, halogen, nitro, thiocyanato, OR¹⁵, S(O)ₙR¹⁶, N(R¹⁵)₂. NR¹⁵OR¹⁵, COR¹⁵, OCOR¹⁵, SCOR¹⁶, NR¹⁵COR¹⁵, NR¹⁵SO₂R¹⁶, CO₂R¹⁵, COSR¹⁶, CON(R¹⁵)₂ and (C₁-C₄)-alkoxy-(C₂-C₆)-alkoxycarbonyl, and where heterocyclyl bears n oxo groups,
R¹⁴ is acetoxy, acetamido, N-methylacetamido, benzoyloxy, benzamido, N-methylbenzamido, methoxycarbonyl, ethoxycarbonyl, benzoyl, methylcarbonyl, piperidinylcarbonyl, morpholinylcarbonyl, trifluoromethylcarbonyl, aminocarbonyl, methylaminocarbonyl, dimethylaminocarbonyl, (C₃-C₆) -alkoxy, (C₃-C₆)-cycloalkyl, or heteroaryl, heterocyclyl or phenyl, each substituted by s radicals from the group consisting of methyl, ethyl, methoxy, trifluoromethyl and halogen;
R¹⁵ is hydrogen, (C₁-C₆)-alkyl, (C₂-C₆)-alkenyl, (C₂-C₆-alkynyl, (C₃-C₆)-cycloalkyl or (C₃-C₆)-cycloalkyl-(C₁-C₆)-alkyl,
R¹⁶ is (C₁-C₆)-alkyl, (C₂-C₆)-alkenyl or (C₂-C₆)-alkynyl,
R¹⁷ is (C₁-C₆)-alkyl, halo-(C₁-C₆) -alkyl, (C₁-C₆)-alkoxy and halo-(C₁-C₆)-alkoxy,
s is 0, 1, 2 or 3,
n is 0, 1 or 2;
is 0, S, or NR¹¹,
excluding the compounds 2-methoxy-N-methyl-N-(1H-tetrazol-5-yl)benzamide, 2-hydroxy-N-methyl-N-(1H-tetrazol-5-yl)benzamide, 2-methoxy-N-methyl-N-(1-methyl-1H-tetrazol-5-yl)benzamide, N-methyl-N-(5-phenyl-1,3,4-oxadiazol-2-yl)benzamide and 3-bromo-4-methoxy-N-methyl-N-(5-phenyl-1,3,4-oxadiazol-2-yl)benzamide.

2. N-(1,3,4-Oxadiazol-3-yl)-, N-(tetrazol-5-yl)- or N-(triazol-5-yl)arylcarboxamides of the formula (I) or the salts thereof according to Claim 1, in which
Q is a Q1, Q2 or Q4 radical, 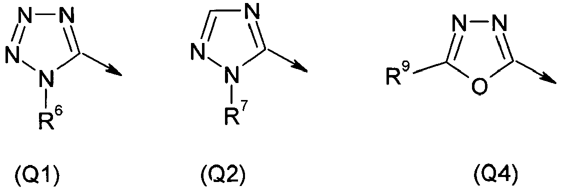 and all the other radicals and indices are each as defined in Claim 1.

3. N-(1,3,4-Oxadiazol-3-yl)- or N-(tetrazol-5-yl)arylcarboxamides of the formula (I) or the salts thereof according to Claim 1 or 2, in which
Q is a Q1 or Q4 radical, 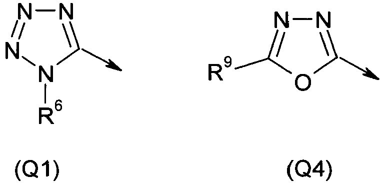 and all the other radicals and indices are each as defined in Claim 1.

4. N-(Tetrazol-5-yl)arylcarboxamides of the formula (I) according to any of Claims 1 to 3, in which
Q is the Q1 radical, 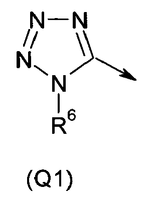 and all the other radicals and indices are each as defined in Claim 1.

5. Herbicidal composition, **characterized by** a herbicidally active content of at least one compound of the formula (I) according to any of Claims 1 to 4.

6. Herbicidal composition according to Claim 5 in a mixture with formulation auxiliaries.

7. Herbicidal composition according to Claim 6, comprising a safener.

8. Herbicidal composition according to Claim 7, comprising a further herbicide.

9. Method for controlling unwanted plants, which comprises applying an effective amount of at least one compound of the formula (I) according to any of Claims 1 to 4 or of a herbicidal composition according to any of Claims 5 to 8 to the plants or the site of the unwanted vegetation.

10. Use of compounds of the formula (I) according to any of Claims 1 to 4 or of herbicidal compositions according to any of Claims 5 to 8 for controlling unwanted plants.

11. Use according to Claim 10, **characterized in that** the compounds of the formula (I) are used for controlling unwanted plants in crops of useful plants.

12. Use according to Claim 11, **characterized in that** the useful plants are transgenic useful plants.

## Revendications

1. Amides de l'acide N-(1,2,5-oxadiazol-3-yl)-arylcarboxylique, de l'acide N-(1,3,4-oxadiazol-2-yl)-arylcarboxylique, de l'acide N-(tétrazol-5-yl)-arylcarboxylique et de l'acide N-(triazol-5-yl)-arylcarboxylique de formule (I) ou leurs sels 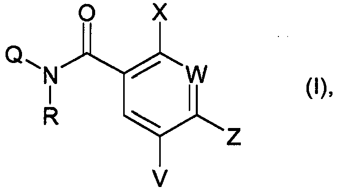 dans laquelle
R signifie (C₁-C₆)-alkyle, (C₁-C₆)-halogénoalkyle, (C₂-C₆)-alcényle, (C₂-C₆)-halogénoalcényle, (C₂-C₆)-alcynyle, (C₃-C₆)-halogénoalcynyle, (C₃-C₆)-cycloalkyle, (C₃-C₆)-halogénocycloalkyle, (C₃-C₆)-cycloalkyl- (C₁-C₆) -alkyle, (C₃-C₅)-halogénocycloalkyl-(C₁-C₆)-alkyle, (C₁-C₆)-alkyl-S(O)ₙR², (C₁-C₆) -alkyl-OR¹, (C₁-C₆)-alkyl-OCOR¹, (C₁-C₆) -alkyl-OSO₂R², (C₁-C₆) -alkyl-COOR¹, (C₁-C₆)-alkyl-C(O)R¹, (C₁-C₆)-alkyl-CN, (C₁-C₆)-alkyl-SO₂OR¹, (C₁-C₆)-alkyl-CON(R¹)₂, (C₁-C₆)-alkyl-SO₂N(R¹)2, (C₁-C₆)-alkyl-NR¹COR¹, (C₁-C₆) -alkyl-NR¹SO₂R², OR¹, COOR¹, CON(R¹)₂, N(R¹)₂, NR¹COOR¹, NR¹CON(R¹) ou benzyle à chaque fois substitué par s radicaux du groupe méthyle, éthyle, méthoxy, nitro, trifluorométhyle et halogène,
W signifie N ou CY,
X et Z signifient, indépendamment l'un de l'autre, à chaque fois hydrogène, nitro, halogéno, cyano, formyle, thiocyano, (C₁-C₆)-alkyle, (C₁-C₆)-halogénoalkyle, (C₂-C₆)-alcényle, (C₂-C₆)-halogénoalcényle, (C₂-C₆)-alcynyle, (C₃-C₆)-halogénoalcynyle, (C₃-C₆)-cycloalkyle, (C₃-C₆)-halogénocycloalkyle, (C₃-C₆)-cycloalkyl-(C₁-C₆)-alkyle, (C₃-C₆)-halogénocycloalkyl-(C₁-C₆) -alkyle, COR¹, OR¹, OCOR¹, OSO₂R², S(O)R², SO₂OR¹, SO₂N(R¹)₂, NR¹SO₂R², NR¹COR¹, (C₁-C₆)-alkyl-S(O)ₙR², (C₁-C₆)-alkyl-OR¹, (C₁-C₆) -alkyl-OCOR¹, (C₁-C₆) -alkyl-OSO₂R², (C₁-C₆)-alkyl-COOR¹, (C₁-C₆) -alkyl-SO₂OR¹, (C₁-C₆) alkyl-CON(R¹) ₂, (C₁-C₆)-alkyl-SO₂N(R¹)₂, (C₁-C₆)-alkyl-NR¹COR¹, (C₁-C₆)-alkyl-NR¹SO₂R², NR¹R², P(O)(OR⁵)₂ ou hétéroaryle, hétérocyclyle ou phényle à chaque fois substitué par s radicaux du groupe méthyle, éthyle, méthoxy, nitro, trifluorométhyle et halogène,
Y signifie hydrogène, nitro, halogéno, cyano, thiocyano, (C₁-C₆) -alkyle, halogéno-(C₁-C₆)-alkyle, (C₂-C₆)-alcényle, halogéno-(C₂-C₆)-alcényle, (C₂-C₆)-alcynyle, halogéno-(C₂-C₆)-alcynyle, (C₃-C₆)-cycloalkyle, (C₃-C₆)-cycloalcényle, halogéno-(C₃-C₆)-cycloalkyle, (C₃-C₆)-cycloalkyl- (C₁-C₆) -alkyle, halogéno-(C₃-C₆)-cycloalkyl- (C₁-C₆) -alkyle, COR¹, COOR¹, OCOOR¹, NR¹COOR¹, C(O)N(R¹)₂, NR¹C(O)N(R¹)₂, OC(O)N(R¹)₂, C(O)N(R¹)OR¹, NR¹SO₂R², NR¹COR¹, OR¹, OSO₂R², S(O)ₙR², SO₂OR¹, SO₂N(R¹)2 (C₁-C₆)-alkyl-S(O)ₙR², (C₁-C₆)-alkyl-OR¹, (C₁-C₆) -alkyl-OCOR¹, (C₁-C₆) -alkyl-OSO₂R², (C₁-C₆) -alkyl-CO₂R¹, (C₁-C₆) -alkyl-CN, (C₁-C₆)-alkyl-SO₂OR¹, (C₁-C₆)-alkyl-CON(R¹)₂, (C₁-C₆)-alkyl-SO₂N(R¹)₂, (C₁-C₆)-alkyl-NR¹COR¹, (C₁-C₆)-alkyl-NR¹SO₂R², N(R¹)₂, P(O)(OR⁵)², CH₂P(O)(OR⁵)₂, CH=NOR¹, (C₁-C₆)-alkyl-CH=NOR¹, (C₁-C₆)-alkyl-O-N=C(^{R}1)₂, (C₁-C₆)-alkyl-phényle, (C₁-C₆)-alkyl-hétéroaryle, (C₁-C₆)-alkyl-hétérocyclyle, phényle, hétéroaryle ou hétérocyclyle, les 6 derniers radicaux mentionnés étant à chaque fois substitués par s radicaux du groupe constitué par halogéno, nitro, cyano, (C₁-C₆)-alkyle, halogéno-(C₁-C₆)-alkyle, (C₃-C₆)-cycloalkyle, S(O)ₙ-(C₁-C₆) -alkyle, (C₁-C₆)-alcoxy, halogéno-(C₁-C₆)-alcoxy, (C₁-C₆)-alcoxy-(C₁-C₄)-alkyle et cyanométhyle et hétérocyclyle portant n groupes oxo ou
Y et Z forment ensemble avec les deux atomes, auxquels ils sont liés, un cycle insaturé, partiellement saturé ou saturé de 5, 6 ou 7 chaînons, qui comprend, outre les atomes de carbone, à chaque fois s atomes d'azote, n atomes d'oxygène, n atomes de soufre et n éléments S(O), S(O)₂, C=N-R¹⁷, C(OR¹⁷)₂, C[-O-(CH₂)₂-O-] ou C(O) comme chaînons de cycle, dont les atomes de carbone sont substitués par s radicaux du groupe constitué par halogéno, cyano, (C₁-C₆) -alkyle, (C₂-C₁₀)-alcényle, (C₂-C₁₀)-alcynyle, (C₁-C₆)-halogénoalkyle, (C₁-C₆)-alcoxy, phénoxy, halogéno-(C₁-C₆)-alcoxy, (C₃-C₈-cycloalkyle, (C₂-C₈)-alcoxyalkyle et phényle, dont les atomes d'azote sont substitués par n radicaux du groupe constitué par (C₁-C₆)-alkyle et phényle et dans lequel les radicaux phényle susmentionnés sont substitués par s radicaux du groupe constitué par cyano, nitro, halogéno, (C₁-C₆)-alkyle, (C₁-C₆)-halogénoalkyle et (C₁-C₆)-alcoxy,
V signifie hydrogène, nitro, halogéno, cyano, (C₁-C₄)-alkyle, (C₁-C₄)-halogénoalkyle, OR¹, S(O)ₙR²,
R¹ signifie hydrogène, (C₁-C₆)-alkyle, (C₁-C₆)-halogénoalkyle, (C₂-C₆)-alcényle, (C₂-C₆)-halogénoalcényle, (C₂-C₆)-alcynyle, (C₂-C₆)-halogénoalcynyle, (C₃-C₆)-cycloalkyle, (C₃-C₆)-cycloalcényle, (C₃-C₆)-halogénocycloalkyle, (C₁-C₆)-alkyl-O- (C₁-C₆) -alkyle, (C₃-C₆)-cycloalkyl- (C₁-C₆)-alkyle, phényle, phényl-(C₁-C₆)-alkyle, hétéroaryle, (C₁-C₆)-alkyl-hétéroaryle, hétérocyclyle, (C₁-C₆)-alkyl-hétérocyclyle, (C₁-C₆)-alkyl-O-hétéroaryle, (C₁-C₆)-alkyl-O-hétérocyclyle, (C₁-C₆)-alkyl-NR³-hétéroaryle, (C₁-C₆)-alkyl-NR³-hétérocyclyle, les 21 derniers groupes mentionnés étant substitués par s radicaux du groupe constitué par cyano, halogéno, nitro, thiocyano, OR³, S(O)ₙR⁴, N(R³)₂, NR³OR³, COR³, OCOR³, SCOR⁴, NR³COR³, NR³SO₂R4, CO₂R³, COSR⁴, CON(R³) et (C₁-C₄)-alcoxy-(C₂-C₆)-alcoxycarbonyle et hétérocyclyle portant n groupes oxo,
R² signifie (C₁-C₆)-alkyle, (C₁-C₆)-halogénoalkyle, (C₂-C₆)-alcényle, (C₂-C₆)-halogénoalcényle, (C₂-C₆)-alcynyle, (C₂-C₆)-halogénoalcynyle, (C₃-C₆)-cycloalkyle, (C₃-C₆)-cycloalcényle, (C₃-C₆)-halogénocycloalkyle, (C₁-C₆)-alkyl-O-(C₁-C₆)-alkyle, (C₃-C₆)-cycloalkyl-(C₁-C₆)-alkyle, phényle, phényl-(C₁-C₆)-alkyle, hétéroaryle, (C₁-C₆)-alkyl-hétéroaryle, hétérocyclyle, (C₁-C₆)-alkyl-hétérocyclyle, (C₁-C₆)-alkyl-O-hétéroaryle, (C₁-C₆) alkyl-O-hétérocyclyle, (C₁-C₆)-alkyl-NR³-hétéroaryle, (C₁-C₆) -alkyl-NR³-hétérocyclyle, les 21 derniers groupes mentionnés étant substitués par s radicaux du groupe constitué par cyano, halogéno, nitro, thiocyano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ et (C₁-C₄)-alcoxy-(C₂-C₆)-alcoxycarbonyle et hétérocyclyle portant n groupes oxo,
R³ signifie hydrogène, (C₁-C₆)-alkyle, (C₂-C₆)-alcényle, (C₂-C₆)-alcynyle, (C₃-C₆)-cycloalkyle ou (C₃-C₆)-cycloalkyl- (C₁-C₆)-alkyle,
R⁴ signifie (C₁-C₆)-alkyle, (C₂-C₆)-alcényle ou (C₂-C₆)-alcynyle, (C₃-C₆) -cycloalkyle ou (C₃-C₆)-cycloalkyl-(C₁-C₆)-alkyle,
R⁵ signifie (C₁-C₄) -alkyle,
n signifie 0, 1 ou 2,
s signifie 0, 1, 2 ou 3,
Q signifie un radical Q1, Q2, Q3 ou Q4 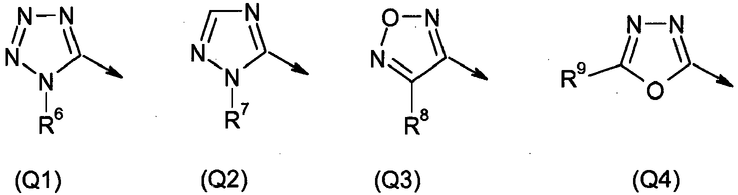
R⁶ signifie (C₁-C₆)-alkyle, halogéno-(C₁-C₆)-alkyle, (C₂-C₆)-alcényle, halogéno-(C₂-C₆)-alcényle, (C₂-C₆)-alcynyle, halogéno-(C₂-C₆)-alcynyle, ces 6 groupes susmentionnés étant à chaque fois substitués par s radicaux du groupe constitué par nitro, cyano, SiR¹²₃, PO(OR¹²)₃, S(O)ₙ-(C₁-C₆)-alkyle, (C₁-C₆)-alcoxy, halogéno-(C₁-C₆)-alcoxy, N(R¹⁰)₂, COR¹⁰, COOR¹⁰, OCOR¹⁰, OCO₂R¹⁰, NR¹⁰COR¹⁰, NR¹⁰SO₂R¹¹, (C₃-C₆-cycloalkyle, hétéroaryle, hétérocyclyle, phényle, D-hétéroaryle, D-hétérocyclyle, D-phényle ou D-benzyle et les 7 derniers radicaux mentionnés étant substitués par s radicaux du groupe méthyle, éthyle, méthoxy, trifluorométhyle et halogéno et hétérocyclyle portant n groupes oxo ou
R⁶ signifie (C₃-C₇)-cycloalkyle, hétéroaryle, hétérocyclyle ou phényle à chaque fois substitué par s radicaux du groupe constitué par halogéno, nitro, cyano, (C₁-C₆) -alkyle, halogéno-(C₁-C₆)-alkyle, (C₃-C₆)-cycloalkyle, S(O)ₙ-(C₁-C₆) -alkyle, (C₁-C₆)-alcoxy, halogéno-(C₁-C₆)-alcoxy et (C₁-C₆)-alcoxy-(C₁-C₄)-alkyle,
R⁷ signifie (C₁-C₆)-alkyle, halogéno- (C₁-C₆) -alkyle, (C₂-C₆)-alcényle, halogéno-(C₂-C₆)-alcényle, (C₂-C₆)-alcynyle, halogéno-(C₂-C₆)-alcynyle, ces 6 radicaux susmentionnés étant à chaque fois substitués par s radicaux du groupe constitué par nitro, cyano, SiR¹²₃, PO(OR¹²)₃, S(O)ₙ-(C₁-C₆)-alkyle, (C₁-C₆)-alcoxy, halogéno-(C₁-C₆)-alcoxy, N(R¹⁰)₂, COR¹⁰, COOR¹⁰, OCOR¹⁰, OCO₂R¹⁰, NR¹⁰COR¹⁰, NR¹⁰SO₂R¹¹, (C₃-C₆)-cycloalkyle, hétéroaryle, hétérocyclyle, phényle, D-hétéroaryle, D-hétérocyclyle, D-phényle et D-benzyle et les 7 derniers radicaux mentionnés étant substitués par s radicaux du groupe méthyle, éthyle, méthoxy, trifluorométhyle et halogéno et hétérocyclyle portant n groupes oxo ou
R⁷ signifie (C₃-C₇)-cycloalkyle, hétéroaryle, hétérocyclyle ou phényle à chaque fois substitué par s radicaux du groupe constitué par halogéno, nitro, cyano, (C₁-C₆)-alkyle, halogéno-(C₁-C₆)-alkyle, (C₃-C₆)-cycloalkyle, S(O)ₙ-(C₁-C₆) -alkyle, (C₁-C₆)-alcoxy, halogéno-(C₁-C₆)-alcoxy et (C₁-C₆)-alcoxy-(C₁-C₄)-alkyle,
R⁸ signifie hydrogène, (C₁-C₆)-alkyle, (C₃-C₇)-cycloalkyle, halogéno-(C₁-C₆)-alkyle, (C₁-C₆)-alcoxy, halogéno-(C₁-C₆)-alcoxy, (C₂-C₆)-alcényle, (C₂-C₆)-alcényloxy, halogéno-(C₂-C₆)-alcényle, (C₂-C₆)-alcynyle, (C₂-C₆)-alcynyloxy, halogéno-(C₂-C₆)-alcynyle, cyano, nitro, méthylsulfényle, méthylsulfinyle, méthylsulfonyle, (C₁-C₆)-alkylcarbonylamino, (C₁-C₆)-alcoxycarbonylamino, benzoylamino, méthoxycarbonyle, éthoxycarbonyle, méthoxycarbonylméthyle, éthoxycarbonylméthyle, benzoyle, méthylcarbonyle, pipéridinylcarbonyle, trifluorométhylcarbonyle, halogéno, amino, aminocarbonyle, méthylaminocarbonyle, diméthylaminocarbonyle, méthoxyméthyle ; ou hétéroaryle, hétérocyclyle ou phényle à chaque fois substitué par s radicaux du groupe constitué par méthyle, éthyle, méthoxy, trifluorométhyle et halogéno ;
R⁹ signifie hydrogène, (C₁-C₆)-alkyle, R¹³O-(C₁-C₆)-alkyle, CH₂R¹⁴, (C₃-C₇)-cycloalkyle, halogéno-(C₁-C₆)-alkyle, (C₂-C₆)-alcényle, halogéno-(C₂-C₆)-alcényle, (C₂-C₆)-alcynyle, halogéno-(C₂-C₆)-alcynyle, OR¹³, NHR¹³, méthoxycarbonyle, éthoxycarbonyle, méthoxycarbonylméthyle, éthoxycarbonylméthyle, méthylcarbonyle, trifluorométhylcarbonyle, diméthylamino, acétylamino, méthylsulfényle, méthylsulfinyle, méthylsulfonyle ; ou hétéroaryle, hétérocyclyle, benzyle ou phényle à chaque fois substitué par s radicaux du groupe constitué par halogéno, nitro, cyano, (C₁-C₆)-alkyle, halogéno-(C₁-C₆) -alkyle, (C₃-C₆)-cycloalkyle, S(O)ₙ-(C₁-C₆)-alkyle, (C₁-C₆)-alcoxy, halogéno-(C₁-C₆)-alcoxy et (C₁-C₆)-alcoxy-(C₁-C₄)-alkyle,
R¹⁰ signifie hydrogène, (C₁-C₆)-alkyle, (C₂-C₆)-alcényle, (C₂-C₆-alcynyle, (C₃-C₆)-cycloalkyle ou (C₃-C₆)-cycloalkyl-(C₁-C₆) -alkyle ou phényle,
R¹¹ signifie (C₁-C₆)-alkyle, (C₂-C₆)-alcényle, (C₂-C₆)-alcynyle ou phényle,
R¹² signifie (C₁-C₆)-alkyle,
R¹³ signifie hydrogène, (C₁-C₆)-alkyle, (C₁-C₆)-halogénoalkyle, (C₂-C₆)-alcényle, (C₂-C₆)-halogénoalcényle, (C₂-C₆)-alcynyle, (C₂-C₆)-halogénoalcynyle, (C₃-C₆)-cycloalkyle, (C₃-C₆)-cycloalcényle, (C₃-C₆)-halogénocycloalkyle, (C₁-C₆)-alkyl-O-(C₁-C₆)-alkyle, (C₃-C₆)-cycloalkyl- (C₁-C₆) alkyle, phényle, phényl-(C₁-C₆)-alkyle, hétéroaryle, (C₁-C₆)-alkyl-hétéroaryle, hétérocyclyle, (C₁-C₆)-alkyl-hétérocyclyle, (C₁-C₆)-alkyl-O-hétéroaryle, (C₁-C₆)-alkyl-O-hétérocyclyle, (C₁-C₆)-alkyl-NR¹⁵-hétéroaryle ou (C₁-C₆)-alkyl-NR¹⁵-hétérocyclyle, les 21 derniers groupes mentionnés étant substitués par s radicaux du groupe constitué par cyano, halogéno, nitro, thiocyano, OR¹⁵, S(O)ₙR¹⁶, N(R¹⁵)₂, NR¹⁵OR¹⁵, COR¹⁵, OCOR¹⁵, SCOR¹⁶, NR¹⁵COR¹⁵, NR¹⁵SO₂R¹⁶, CO₂R¹⁵, COSR¹⁶, CON(R¹⁵)₂ et (C₁-C₄)-alcoxy-(C₂-C₆)-alcoxycarbonyle et hétérocyclyle portant n groupes oxo,
R¹⁴ signifie acétoxy, acétamido, N-méthylacétamido, benzoyloxy, benzamido, N-méthylbenzamido, méthoxycarbonyle, éthoxycarbonyle, benzoyle, méthylcarbonyle, pipéridinylcarbonyle, morpholinylcarbonyle, trifluorométhylcarbonyle, aminocarbonyle, méthylaminocarbonyle, diméthylaminocarbonyle, (C₃-C₆)-alcoxy, (C₃-C₆)-cycloalkyle ; ou hétéroaryle, hétérocyclyle ou phényle à chaque fois substitué par s radicaux du groupe constitué par méthyle, éthyle, méthoxy, trifluorométhyle et halogéno ;
R¹⁵ signifie hydrogène, (C₁-C₆)-alkyle, (C₂-C₆)-alcényle, (C₂-C₆)-alcynyle, (C₃-C₆)-cycloalkyle ou (C₃-C₆)-cycloalkyl-(C₁-C₆)-alkyle,
R¹⁶ signifie (C₁-C₆)-alkyle, (C₂-C₆)-alcényle ou (C₂-C₆)-alcynyle,
R¹⁷ signifie (C₁-C₆)-alkyle, halogéno-(C₁-C₆)-alkyle, (C₁-C₆)-alcoxy et halogéno-(C₁-C₆) -alcoxy;
s signifie 0, 1, 2 ou 3,
n signifie 0, 1 ou 2,
D signifie 0, S ou NR¹¹,
les composés 2-méthoxy-N-méthyl-N-(1H-tétrazol-5-yl)benzamide, 2-hydroxy-N-méthyl-N-(1H-tétrazol-5-yl)benzamide, 2-méthoxy-N-méthyl-N-(1-méthyl-1H-tétrazol-5-yl)benzamide, N-méthyl-N-(5-phényl-1,3,4-oxadiazol-2-yl)benzamide et 3-bromo-4-méthoxy-N-méthyl-N-(5-phényl-1,3,4-oxadiazol-2-yl)benzamide étant exclus.

2. Amides de l'acide N-(1,3,4-oxadiazol-2-yl)-arylcarboxylique, de l'acide N-(tétrazol-5-yl)-arylcarboxylique et de l'acide N-(triazol-5-yl)-arylcarboxylique de formule (I) ou leurs sels selon la revendication 1, dans lesquels
Q signifie un radical Q1, Q2 ou Q4 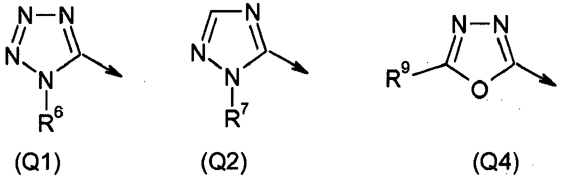 et tous les autres radicaux et indices sont à chaque fois définis comme dans la revendication 1.

3. Amides de l'acide N-(1,3,4-oxadiazol-2-yl)-arylcarboxylique et de l'acide N-(tétrazol-5-yl)-arylcarboxylique de formule (I) ou leurs sels selon la revendication 1 ou 2, dans lesquels
Q signifie un radical Q1 ou Q4 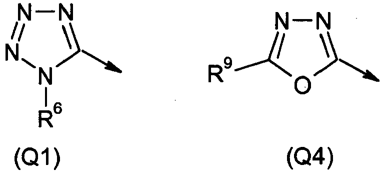 et tous les autres radicaux et indices sont à chaque fois définis comme dans la revendication 1.

4. Amides de l'acide N-(tétrazol-5-yl)-arylcarboxylique de formule (I) selon l'une quelconque des revendications 1 à 3, dans lesquels
Q signifie le radical Q1 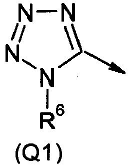 et tous les autres radicaux et indices sont à chaque fois définis comme dans la revendication 1.

5. Agents herbicides, **caractérisés par** une teneur active en tant qu'herbicide en au moins un composé de formule (I) selon l'une quelconque des revendications 1 à 4.

6. Agents herbicides selon la revendication 5 en mélange avec des adjuvants de formulation.

7. Agents herbicides selon la revendication 6 contenant un antidote.

8. Agents herbicides selon la revendication 7 contenant un autre herbicide.

9. Procédé pour lutter contre des plantes non souhaitées, **caractérisé en ce qu'**on applique une quantité active d'au moins un composé de formule (I) selon l'une quelconque des revendications 1 à 4 ou d'un agent herbicide selon l'une quelconque des revendications 5 à 8 sur les plantes ou sur le lieu de la croissance des plantes non souhaitées.

10. Utilisation de composés de formule (I) selon l'une quelconque des revendications 1 à 4 ou d'agents herbicides selon l'une quelconque des revendications 5 à 8 pour lutter contre des plantes non souhaitées.

11. Utilisation selon la revendication 10, **caractérisée en ce que** les composés de formule (I) sont utilisés pour lutter contre des plantes non souhaitées dans les cultures de plantes utiles.

12. Utilisation selon la revendication 11, **caractérisée en ce que** les plantes utiles sont des plantes utiles transgéniques.
